(19) Europäisches Patentamt
European Patent Office
Office européen des brevets

(11) EP 2 559 750 A1

(12) **EUROPÄISCHE PATENTANMELDUNG**

(43) Veröffentlichungstag:
20.02.2013 Patentblatt 2013/08

(51) Int Cl.:
*C12M 1/00* (2006.01)

(21) Anmeldenummer: 12180864.6

(22) Anmeldetag: 17.08.2012

(84) Benannte Vertragsstaaten:
AL AT BE BG CH CY CZ DE DK EE ES FI FR GB GR HR HU IE IS IT LI LT LU LV MC MK MT NL NO PL PT RO RS SE SI SK SM TR
Benannte Erstreckungsstaaten:
BA ME

(30) Priorität: 18.08.2011 DE 102011110638

(71) Anmelder: Kujawski, Olaf
30853 Langenhagen (DE)

(72) Erfinder: Kujawski, Olaf
30853 Langenhagen (DE)

(74) Vertreter: Günther, Constantin et al
Gramm, Lins & Partner GbR
Freundallee 13a
30173 Hannover (DE)

(54) **Anlagenweites Steuerungs- und Regelungsverfahren für Biogasanlagen**

(57) Die Erfindung betrifft ein Anlagenweites Steuerungs- und Regelungsverfahren für Biogasanlagen, z.B. ein Verfahren zur Vorgabe einer Sollleistung einer Gasverwertungsanlage (18), die mit einer Biogasanlage verbunden ist und mit Biogas und/oder Methan aus einem Gasspeicher (12) der Biogasanlage versorgt wird, dadurch gekennzeichnet, dass

a) die Sollleistung unter Berücksichtigung der zeitlichen Änderung des in einem Gasspeicher (12) der Biogasanlage gespeicherten Gasvolumens bestimmt wird und/oder

b) die Sollleistung um einen vorbestimmten ersten Wert gegenüber einer ansonsten vorgegebenen Standard-Sollleistung erhöht wird, wenn das in dem Gasspeicher (12) gespeicherte Gasvolumen einen vorbestimmten Höchstwert erreicht oder überschreitet.

Abbildung 0.13 Wirkungskette für die entwickelte Regelung der Substratzufuhr für Biogasanlagen

EP 2 559 750 A1

**Beschreibung**

1 Abkürzungen

Abkürzungsverzeichnis

| | |
|---|---|
| ADM1 - Anaerobic Digestion Model No.1 | MSR-Technik - Mess-, Steuerungs- und Regelungstechnik |
| BioAbfV - Bioabfallverordnung | NawaRo - nachwachsende Rohstoffe |
| BGA - Biogasanlage | NIRS - Nahinfrarotspektroskopie |
| CSB - chemischer Sauerstoffbedarf | oTM - organische Trockenmasse |
| CSTR - continuous stirred-tank reactor (kontinuierlicher, idealer Rührkessel) | oTR - organischer Trockenrückstand |
| | oTS - organische Trockensubstanz |
| EEG - Erneuerbare-Energien-Gesetz | TAC - totales anorganisches Carbonat |
| FLC - Fuzzylogiccontroler | TOC - gesamtes organisches Kohlenstoff (total organic carbon) |
| FOS - flüchtige organische Säure | |
| FOS/TAC - Quotient aus Säurekonzentration und Pufferkapazität im Gärsubstrat | TM - Trockenmasse |
| | TMR - Tauchmotorrührwerk |
| GPS - Ganzpflanzensilage | TR - Trockenrückstand |
| KWK - Kraft-Wärme-Kopplung | TS - Trockensubstanz |
| LCB - Lignocellulose-Biomasse | |

Formelnverzeichnis

| | |
|---|---|
| $CH_3COOH$ - Essigsäure | $NH_4\text{-}N$ - Ammoniumstickstoff |
| $CH_4$ - Methan | $NH_3$ - Ammoniak |
| $CO_2$ - Kohlenstoffdioxid | $NO_3^-$ - Nitrat |
| $H_2$ - Wasserstoff | $NO_2^-$ - Nitrit |
| $H_2S$ - Schwefelwasserstoff | $PO_4^{3-}$ - Orthophosphat |
| $NH_4^+$ - Ammonium | TKN - Kjeldahl-Stickstoff |

2 Einleitung

2.1 Problemstellung

Der Einsatz der MSR-Technik auf Biogasanlagen ist in der Praxis auf einem niedrigen Niveau. Die Ergebnisse von umfangreichen Studien in Deutschland belegen, dass zahlreiche Biogasanlagen mangelhaft mit der Messtechnik ausgestattet sind. Eine logische Konsequenz dieser Tatsache ist, dass keine oder nur einfache Steuerungs- und Regelungsstrategien eingesetzt werden. Nach einer umfangreichen Analyse konnten in der Fachliteratur keine Berichte über anlagenweite Steuerungs- und Regelungskonzepte in der Praxis gefunden werden.

Der Stand des Wissens ist demgegenüber zwar ein Schritt voraus, es wurden bis dato jedoch keine marktreifen Produkte im Bereich MSR-Technik entwickelt. Die wichtigen Parameter für die Steuerung und Regelung wie z. B. die Konzentration der organischen Säuren oder der Wasserstoffpartialdruck in der wässrigen Phase können bis dato nicht zuverlässig online mit einem vertretbaren Aufwand gemessen werden. Es bestehen jedoch sehr genaue Messtechniken (z. B. Gaschromatografie) oder es werden neue Entwickelt (z. B. Nahinfrarotspektroskopie). Sie sind jedoch oft zu teuer, wenig robust, erfordern aufwendige Probenaufbereitung oder größeren Kalibrierungsaufwand. Darüber hinaus werden andere Messtechniken (z. B. Titration für die Bestimmung der organischen Säuren und der Säurekapazität) für die Bestimmung der angestrebten Parametern angewendet, die kostengünstig sind und schnell Ergebnisse liefern können. Die Ergebnisse geben jedoch eher eine Tendenz des Parameterwertes und keine genauen Absolutwerte wieder. Die Steuerungs- und Regelungsstrategien werden im Rahmen der wissenschaftlichen Projekte überwiegend im Labormaßstab durchgeführt. Wenn sie für full-scale Anlagen entwickelt werden, dann für eine spezielle Verfahrenstechnik und bestimmte Substratmischungen oder/und es wird vorausgesetzt, dass eine aufwendige Messtechnik (z. B. Gaschromatograf) eingesetzt werden muss.

Auf der anderen Seite bietet die komplexe Verfahrenstechnik der Biogasanlagen viele regelungstechnische Eingriffsmöglichkeiten. Ohne eine EDV-technische Unterstützung des Betriebes kann jedoch dieser Vorteil schnell zum Nachteil werden. Demzufolge können die Betreiber zurecht mit vielen Aufgaben, wie z. B. das Substratmanagement, die Wahl der Zufuhrstrategie, die Wahl der Rührstrategie oder die Steuerung des BHKW, überfordert sein. Schlechte oder suboptimale Entscheidungen in den Bereichen können einen wirtschaftlichen Nachteil für die Betriebe bedeuten.

Ein durchdachtes Substratmanagement, das auf Eigenerfahrungen des Betriebes basiert (z. B. Erfahrungswerte Methan-, Biogasbildungsraten und Abbauraten der eingesetzten Substrate), sowie im Zuge dessen mehrere Szenarien, die analysiert und verglichen werden, ist ohne Einsatz der EDV-Technik schwierig. Die Voraussetzung für EDV-gestützte Unterstützung des Betreibers ist eine anlagenweite Kontrolle des Betriebes.

Der Einsatz der MSR-Technik kann unter anderem die Stabilität der anaeroben Abbauprozesse und dadurch die Betriebssicherheit stärken. Darüber hinaus kann eine vorausschauende Substratzufuhrstrategie einen sicheren Betrieb gewährleisten. Dazu soll die Energieproduktion auf einem hohen Niveau - ohne Gasverluste oder einem Teillastbetrieb der Biogasverwertungsanlage (z. B. BHKW) - gehalten werden. Dadurch kann eine höhere Auslastung der Biogasanlage erreicht werden. Im Schnitt beträgt die BHKW-Leistungsausnutzung der untersuchten deutschen Anlagen 85 %. Es gibt aber viele, die einen Wert über 95 % erreichen.

Bisherige ungenaue Messungen des Gasvolumens führen zur sprunghaften Änderungen der Sollleitung der BHKW, wodurch die Gasspeicher entleert oder zu voll sind. Dies ist die Folge einer ungenauen Messung des Gasvolumens in den Speichern. Genauere Messungen in Normkubikmeter könnte die notwendigen Informationen für die Steuerung der BHKW liefern.

Außer der Optimierung der Energieproduktion und Ausnutzung der Substrate sollte auch in der Zukunft der Energieverbrauch der Anlage intensiver betrachtet werden. Ein großes Potenzial liegt dabei bei der Rührtechnik. Eine gezielte Regelungsstrategie der Rührwerkstechnik, einer der größten Energieverbraucher der Biogasanlage, kann die gewinne maximieren.

Die MSR-Technik auf Biogasanlagen und deren anlagenweite Betrachtung haben somit ein signifikantes Potenzial für das Erreichen und für die Sicherung der Wirtschaftlichkeit auf einem hohen Niveau.

2.2 Biogastechnik

2.3 Verfahrenstechnik auf Biogasanlagen

Die Verfahrenstechnik der Biogasanlagen muss so geplant werden, damit sie wirtschaftlich arbeiten sowie sicher, optimal und flexibel betrieben werden können.

2.4 Einflussfaktoren und verfahrenstechnische Prozessparameter des anaeroben Abbaus, mit der Zielsetzung der maximalen Biogasproduktion

Die anaeroben Abbauprozesse im Fermenter der Biogasanlage sollen möglichst stabil und effizient verlaufen. Dies garantiert hohe Erträge und einen wirtschaftlichen Betrieb. Um diesen Zustand erreichen zu können, muss der Betreiber einer Biogasanlage die Produktionsprozesse überwachen und kontrollieren können. Dies kann nur dann erfolgen, wenn ausreichend viel Transparenz geschaffen wird. Hinsichtlich der Prozesskontrolle werden folgende Messgrößen unterschieden: Kontrollparameter zur Früherkennung von Prozessstörungen (Frühwarnparameter) zur Vermeidung von Prozessstörungen sowie Kontrollparameter zur Beurteilung des Prozesszustands (variable Einstellgrößen). Die Messparameter auf Biogasanlagen können folgend klassifiziert werden:

● Kontrollparameter zur Früherkennung von Prozessstörungen

    o Flüchtige organische Säuren

    o FOS/TAC-Verhältnis

    o Redox-Potenzial

    o Wasserstoff in der Gas- und Flüssigphase

● Kontrollparameter zur Beurteilung des Prozesszustands:

o Substratmenge und Zusammensetzung

o Gasproduktion und Gaszusammensetzung

o Toxizität (z. B. Ammoniumstickstoff, Schwefelwasserstoff)

o Gärtemperatur

o Trockensubstanzgehalt und Viskosität des Gärgemisches

o pH-Wert

2.4.1 Kontrollparameter zur Früherkennung von Prozessstörungen

2.4.1.1 Flüchtige organische Säuren

Die flüchtigen organischen Säuren (z. B. Essigsäure, Propionsäure, Buttersäure, Valeriansäure und Capronsäure) werden im Laufe der Aceto- und Acidogenese gebildet. Die Produkte der beiden Prozesse: Essigsäure, Kohlendioxid und Wasserstoff, werden für die Methanbildung verbraucht. Bei einer stabilen Fermentation ist der Gehalt an flüchtigen Fettsäuren im Gärsubstrat stabil, da sich die Säurebildung und die Säureverwertung im Gleichgewicht befinden. Übersteigt die Säurebildung die maximale Abbaukapazität durch Essigsäurebildung und Methanbildung, kommt es zu einem Anstieg der Konzentration an flüchtigen organischen Säuren. Der Anstieg kann durch eine zu starke Belastung des Fermenters oder durch eine Hemmung der Methanogenese verursacht werden.

2.4.1.2 FOS/TAC-Verhältnis

Das FOS/TAC-Verhältnis steht für den Quotient aus der Summe der flüchtigen organischen Säuren (FOS) und dem gesamten anorganischen Carbonat (TAC). Der FOS-Wert spiegelt näherungsweise den Gehalt an sämtlichen flüchtigen Fettsäuren wieder. Die Höhe des TAC-Wertes gibt an, wie stark der pH-Wert einer Zugabe von Säure widersteht. Die beide Parameter werden mit Hilfe der Titration gemessen. Der Säureverbrauch bis zum pH Wert von 5 entspricht der Carbonat- und Bicarbonatkonzentration. Die Pufferkapazität ist für die Stabilität der anaeroben Abbauprozesse von großer Bedeutung. Säurebindungsvermögen haben mehrere Ionen. Die wichtigste Rolle in dem sogenannten Faulwasser spielen folgende Ionen:

- Karbonationen $HCO_3^-$, $CO_3^{2-}$ (Kohlensäure-Bicarbonat-Puffer)

- Ammoniak $NH_3$ (Ammoniakpuffer)

- Phosphate $PO_4^{3-}$, $HPO_4^{2-}$ (Phosphatpuffer)

- Acetate $COOH^-$ (Essigsäure-Acetat-Puffer).

In der Literatur werden sehr unterschiedliche Grenzwerte des Parameters angegeben (Tabelle 2.1).

Tabelle 2.1 Grenzwerte für FOS/TAC Verhältnis in der Literatur

| Quelle | [20] | [9] | [34] | [30] |
|---|---|---|---|---|
| Normalbereich des Parameters | < 0,8 * <br> < 0,3 ** | < 0,8 | < 0,3 <br> < 0,6 *** | 0,15< und <0,45 |
| *- für Fermenter, ** - für Nachgärer, *** - NawaRo-Anlagen | | | | |

In der Fachliteratur wurden keine direkten Hinweise auf die Einflussmaßnahmen gefunden. Sollte das FOS/TAC-Verhältnis zu hoch sein, dann bieten sich die gleichen Maßnahmen wie bei der erhöhten Konzentration an organischen Säuren an.

2.4.1.3 Redox-Potenzial

Das Redox-Potenzial spiegelt Änderungen des Oxidations- und Reduktionszustands des Faulschlammes. Negative Redox-Potenzial-Werte sind angemessen für anaerobe Abbauprozesse und anaerobe Biozönose [35], da in dem Milieu die meisten Reaktionen Reduktionscharakter haben. Ein Anstieg des Wertes ist ungünstig für die anaerobe Biozönose, da er eine Zunahme an der Anzahl der Oxidationsprozesse widerspiegelt. Die Messung erfolgt relativ einfach durch den Einsatz von Redox-Elektroden, die zur Vermeidung von Störungen infolge von Fouling regelmäßig gereinigt werden müssen. Die Methanbakterien brauchen laut [3] für ihr Wachstum ein Redox-Potenzial kleiner als - 330 mV. Anhand [6] liegt der Optimalbereich für methanogene Bakterien zwischen -330 und -300 mV . [5] gibt einen typischen Wert für die anaerobe Biozönose von -300 mV an. Schließlich berichtet [33], dass ein optimaler Bereich für die Hydrolyse- und Versäuerungsphase zwischen -300 mV und +400 mV liegt und für die Acetogene- und Methanogenephase im Bereich kleiner -250 mV liegt.

Ein rascher Anstieg des Redox-Potenzials signalisiert den Anstieg der Konzentration an organischen Säuren im Faulschlamm [11].

Sollte der Redox-Potenzial-Wert ansteigen, dann bieten sich die gleichen Maßnahmen wie bei der erhöhten Konzentration der organischen Säuren oder erhöhten FOS/TAC-Werten an.

2.4.2 Kontrollparameter zur Beurteilung des Prozesszustands

2.4.2.1 Substratmenge und -Zusammensetzung

Die Menge des zudosierten Substrates spielt eine wichtige Rolle insbesondere hinsichtlich der hydraulischen Verweilzeit im Fermenter sowie der organischen Belastung der Biologie. Im Zusammenhang damit steht die Substratzusammensetzung. TM- und oTM-Gehalte sind relevant für die Einschätzung der erwarteten Biogasproduktion. Zur Prozessstabilität ist auch der Stickstoffgehalt der Substrate von Bedeutung, da im Laufe des anaeroben Abbaus eine Umwandlung in toxische Stickstoffverbindungen auftritt. Der Gehalt an Spurenelementen wie z. B. Fe, Ni, Co, Mo, Mn, Se und Na in den Substraten ist für die Stoffwechselprozesse und die Zusammensetzung der Biozönose bzw. für das Wachstum entscheidend. Von Anlagen, die eine sog. Monovergärung betreiben, ist bekannt, dass Spurenelementmangel zur signifikanten Reduktion der Biogasproduktion führt [21]. Um das zu vermeiden, werden häufig Ausgangssubstrate so gewählt, dass ausreichend viele Spurenelemente für die Bakterien zur Verfügung stehen. Häufig werden deswegen auch die Wirtschaftsdünger wie Schweinegülle oder Hühnerkot eingesetzt. Wenn keine entsprechende Mischung der Substrate hergestellt werden kann, muss eins von den mittlerweile zahlreichen auf dem Markt befindlichen Produkte der Spurenelementemischung eingesetzt werden.

2.4.2.2 Gasproduktion und Gaszusammensetzung

Die Gasproduktion und die Gaszusammensetzung hängen sehr stark von der Belastung des Fermenters sowie von der Zusammensetzung der eingesetzten Substrate ab. Bei konstanter Substratzufuhr deutet eine Minderung der Gasproduktion und/oder des Methangehalts auf die Störung oder die Hemmung des Prozesses hin [32]. Die Biogasmengenmessung und die Messung der Biogaszusammensetzung sind auf jeder Biogasanlage zwingend notwendig. Die beiden Parameter können sehr gut für die Prozesskontrolle verwendet werden.

2.4.2.3 Toxizität

Es gibt eine ganze Reihe von Verbindungen, die auf die Bakterien toxisch wirken können. Hier werden die am häufigsten vorkommenden Verbindungen Ammoniak (undissoziiertes Ammonium) und Schwefelwasserstoff vorgestellt.

Die Freisetzung von Ammonium ($NH_4^+$) und Ammoniak ($NH_3$) kommt durch Abbau von stickstoffhaltigen Verbindungen (Proteine) zustande. Die toxische Wirkung des Ammoniums bestimmt der undissoziierte Anteil - Ammoniak. "Da die Konzentrationen oberhalb von ca. 100 mg $NH_3$-N/l bereits den Prozess hemmen kann, ist bei Verarbeitung stickstoffreicher Substrate eine regelmäßige Kontrolle sinnvoll. Der Anteil an freiem Ammoniak ist dabei vom pH-Wert und der Temperatur abhängig und nimmt bei Erhöhung beider Parameter zu" [34].

Die Stärke der Hemmwirkung hängt von der Temperatur-, dem pH-Wert sowie der Adaptionsfähigkeit der Biozönose ab. In der Literatur liegen daher die Grenzwerte in einer großen Bandbreite von 150 bis 700 mg/l $NH_3$. Somit lassen sich auch hier keine eindeutig verlässliche, allgemeingültige Grenzwerte festlegen.

Die Messung des Ammonium- und des Ammoniakgehaltes erfolgt mittels Spektrometrie nach Zentrifugation und Filtration der Faulschlammprobe.

Es gibt folgende Maßnahmen gegen eine erhöhte Konzentration an Ammoniak im Faulschlamm: eine Senkung der Gesamtstickstoffkonzentration durch Reduktion der stickstoffhaltigen Substrate im Input oder eine Absenkung der Gärtemperatur [36].

### 2.4.2.4 Trockensubstanzgehalt, Trockenrückstand und Viskosität des Gärgemisches

Der Trockensubstanzgehalt (TS), die Trockenmasse (TM) (auch als Trockenrückstand bezeichnet) und die Viskosität des Faulschlammes spielen eine wichtige Rolle hinsichtlich der Durchmischung, des Gasaustrages des Faulschlammes sowie der Pumpfähigkeit [34]. Die Trockenmasse (TM) bildet gelöste und partikuläre Feststoffe. Die Bestimmung erfolgt ohne Filtration, sodass das Gewicht der gelösten Verbindungen mit erfasst wird. Im Gegensatz dazu bildet die Trockensubstanz nur abfiltrierbare Feststoffe. Die TS- und TM-Werte korrelieren zwar nicht direkt mit der Viskosität [34], auf vielen Anlagen werden aber für die Beurteilung der Durchmischung des Fermenters TS oder TM überwacht. Da die Viskosität des Faulschlammes sehr schwer zu messen ist, kann sie z. B. durch die Leistungsaufnahme der Rührwerke indirekt kontrolliert und überwacht werden. Eine solche Überwachung der Rührwerksarbeit kann die Prozessstörungen durch eine unvollständige Durchmischung des Fermenterinhalts vermeiden [34]. Da die Rührtechnik viel Energie verbraucht ist jede Einsparung diesbezüglich relevant. Auf den meisten Biogasanlagen wird die Rührtechnik entweder nach einem vorgegebenen Zeitintervall oder über eine händisch eingestellte Drehzahl gesteuert. Es bestehen jedoch keine Systeme zur automatischen Steuerung der Rührtechnik des anaeroben Reaktors.

### 2.4.2.5 pH Wert

Die verschiedenen Bakteriengruppen, die die vier Schritte des anaeroben Abbaus durchführen, haben unterschiedliche pH-Optima. "Hydrolysierende und versäuernde Bakterien haben optimale Milieubedingungen in einer leicht sauren Umgebung (um pH 6), während methanbildende Mikroorganismen den neutralen Bereich bevorzugen" [16]. Allgemein kann man sagen, dass die anaerobe Biozönose einen Toleranzbereich von pH 6,8 bis 7,5 hat [13]. Zur Erkennung von Prozessstörungen sowie zur Prozesssteuerung ist der pH-Wert jedoch völlig ungeeignet, da sich durch die starke Pufferwirkung von Bicarbonat und Ammonium der pH-Wert erst bei einer massiven Anreicherung von Stoffwechselprodukten (meist organischen Säuren) verändert. Für die Überwachung der Prozesse ist es jedoch wichtig, dass der pH regelmäßig kontrolliert wird.

### 2.5 Biogas- und -methanausbeute

Die Biogas- und Methanausbeute sind von großer Bedeutung für die Auslegung und das Betreiben der Biogasanlagen. Die beiden Größen bezeichnen die erwartete Biogas- und Methanproduktion pro Frischmasse oder organischer Trockenmasse für ein bestimmtes Substrat.

Die Laborbestimmung der Parameter kann mit Hilfe von Durchfluss- oder Batchverfahren (gem. DIN 3841) erfolgen. Die Messergebnisse der Laborversuche für die Bestimmung der Biogas- und der Methanausbeute wurde in zahlreichen Arbeiten für diverse Substrate untersucht und zusammengestellt. Für jedes Substrat gibt es eine definierte Bandbreite für die Biogasausbeute sowie den Methangehalt des Biogases. Unter zahlreichen Publikationen sind zwei besonders renommiert, die häufig verwendet werden: Publikationen der Fachagentur nachwachsende Rohstoffe (FNR) [7, 9] sowie Publikation des Kuratoriums für Technik und Bauwesen in der Landwirtschaft e.V. (KTBL).

### 2.6 Modelle in der Anaerobtechnik für CSTR

Mittlerweile gibt es mehrere Modelle, die den anaeroben Abbau beschreiben. In der Diplomarbeit von Prause [25] wurden etwa 70 Modelle, die bis 1997 anstanden, zusammengestellt und nach Reaktortypen klassifiziert. In der Arbeit wurden Modelle für kontinuierliche, ideale Rührkessel (CSTR), UASB-, Wirbelschicht-, Festbett- und Hybridreaktoren erwähnt. Insgesamt wurden dort 40 dynamische Modelle für die Modellierung der CSTR zusammengestellt. Alle vorgestellten Modelle basierten auf Modellierung der anaeroben Biomasse sowie Abbauvorgänge der drei Stoffgruppen Kohlenhydrate, Proteine und Fette. Die meisten Modellen wurden auf Basis von CSB-Bilanzen entwickelt.

### 2.7 MSR-Technik auf Biogasanlagen

### 2.7.1 Stand der Messtechnik für Biogasanlagen

### 2.7.1.1 Stand der Technik

Um die einzelnen Produktionsprozesse überwachen und Inputdaten für die Steuerung und Regelung liefern zu können, muss eine ganze Reihe von Messgeräten auf Biogasanlagen eingesetzt werden, die die notwendigen Parameter bestimmen.

In der Messtechnik werden oft die Begriffe online, inline und offline verwendet. Deren Bedeutung hängt jedoch sehr stark vom konkreten Bezug ab. In Bezug auf das untersuchte Material werden die Messtechniken folgend klassifizieren:

● inline - bezeichnet alle Messungen, die das Substrat direkt im Prozess (im Reaktor oder in der Rohrleitung) untersuchen,
● offline - bezeichnet alle Messungen für die eine Probe vom Prozess genommen wird und quasi extern untersucht wird.

In Bezug auf die EDV-technische Anbindung wird die Messtechnik folgend klassifiziert:

● online - bezeichnet alle Messungen, deren Ergebnis direkt vom Messgerät zum Prozessleitsystem gesendet werden kann,

● offline - bezeichnet alle Messungen, deren Ergebnisse nicht sofort für das Prozessleitsystem verfügbar sind.

Zur Vereinfachung und zum Verständnis werden in dieser Arbeit die Begriffe online und offline nur in Bezug auf die EDV-technische Anbindung verwendet.

2.7.1.2 Stand des Wissens

Die Entwicklung der neuen Messtechniken für Biogasanlagen hat diverse Richtungen, die sich aus Bedürfnissen dieses speziellen Einsatzes (z. B. Analyse des Faulschlammes mit einem hohen TM-Gehalt) ergeben. Die Hauptanliegen bei der Entwicklung von neuen Messtechniken sind:

- Die Vereinfachung oder die Vermeidung der Probenaufbereitung

- Die Senkung der Analysekosten (Investitions- und Betriebskosten)

- Die Automatisierung der Messungen (Offline- zu Inlinemessung)

- Erhöhung der Verfügbarkeit der Messwerte für die Steuerung und Regelung (von Offline- zu Onlinemessung)

- Die Messung von Frühwarnparameter (z. B. einzelne organische Säuren, Wasserstoff)

- Die Abschätzung des Messwertes mit Hilfe von sogenannten Softsensoren

- Die Senkung des Kalibrationsaufwands

Im Nachfolgenden wird der Stand des Wissens für einzelne Messtechniken im Einzelnen dargestellt.

Durch Titration lassen sich die organischen Säuren, $HCO_3^-$, $NH_3$ und $S^{2-}$ bestimmen [29]. Die Messung beruht darauf, dass die Menge an der titrierten Säure oder Base proportional zu dem bestimmenden Parameter ist. Über Umrechnung mit Hilfe von mathematischen Funktionen lassen sich die oben genannten Messparameter bestimmen. In der Vergangenheit wurden einige Methoden zur Bestimmung der diversen Parameter über Titration z. B. von Kapp [14] (Lösung über lineare Regression) oder Moosburger et al. [23] (Lösung über lineare algebraische Gleichungen) entwickelt. Die Vorteile der Messtechnik sind: niedrige Betriebskosten, einfache Bestimmung (möglich auch vor Ort auf Biogasanlagen) und einfache Technik (für die Bestimmung braucht man nur die pH-Elektrode und Säure oder Base).

Zaher et al. [38] haben zwei Titratoren für die Bestimmung von organischen Säuren und Bikarbonatpuffer für einen Laborfermenter entwickelt und getestet. Im Rahmen der Untersuchung wurden zwei Titrationsmethoden miteinander verglichen. Die erste Methode beruht auf einer Berechnung der Säurekonstanten bei komplexen Puffersystemen. Die zweite Methode basiert auf der Annahme, dass in dem untersuchten Puffersystem die organischen Säuren und die Bikarbonate die dominierenden Anteile der Alkalinität bilden. Die Berechnung der Konzentration von organischen Säuren und Bikarbonatpuffer basiert auf den Methoden von Kapp [14] und Moosburger et al. [23]. Während der Testphase wurde der Laborfermenter überlastet, sodass unterschiedliche Konzentrationen an organischen Säuren gemessen werden konnten. Den Autoren ist es gelungen auch die Konzentrationen von Milchsäure mit Hilfe von Titration zu messen.

Der Einsatz von Nahinfrarotspektroskopie (NIRS) als prozessanalytische Technologie ist in vielen Sektoren, wie beispielsweise in der chemischen, pharmazeutischen und Lebensmittel verarbeitenden Industrie, bereits Standard [15]. NIRS wird in der Anaerobtechnik und in letzter Zeit in der Biogastechnik verstärkt getestet. Da das Biogassubstrat sehr hohe TM-Gehalte hat, wird insbesondere das Absorptions-Reflexions-Messprinzip der Messtechnik eingesetzt. Dabei wird das Phänomen des Übergangs zwischen spezifischen Energieniveaus der Moleküle der organischen Verbindungen nach Nahinfraroteinstrahlung (von 800 bis 2500 nm) verwendet. Die Differenz aus eingestrahlter und in der Probe absorbierter Energie kann als diffuse Reflexion aufgezeichnet werden. Dabei entstehen für das jeweilige Substrat charakteristische Spektren. Die Berechnung von Spektren der gesuchten Parameter erfolgt mit Hilfe der multivariablen statistischen Methoden; auch als Schätzmodelle bezeichnet. Mit Hilfe der Messtechnik ist es möglich inline und berührungslos Messparameter wie TM, oTM, TS, oTS, CSB, Essigsäureäquivalent, Essigsäure, pH, Pufferkapazität, Rohnährstoffe, Methanpotenzial, Restgaspotenzial und Düngernährstoffe im Rohsubstrat, Fermentersubstrat (Faulschlamm) sowie Gärreste der Biogasanlage zu bestimmen.

Nacke et al. [24] berichten über die Entwicklung von einer Inline-Mikrowellen-Prozesssensorik zur Bestimmung des Trockenmasseanteils und dessen organischen Anteil im Biogasfermenter. Das Messverfahren nutzt die Tatsache, dass die Dielektrizitätskonstanten von Wasser (Wert von ca. 80) und suspendierten Feststoffen (Wert von ca. 2 bis 10) sich wesentlich unterscheiden. Von der Messeinrichtung wird durch eine Antenne eine elektromagnetische Welle ins Material eingestrahlt oder ein elektromagnetisches Feld einer Streufeldanordnung (z. B. offene Leitung) breitet sich in das Material aus. Gemessen wird sowohl die in das Material hineinlaufende elektromagnetische Welle als auch die vom Material reflektierte Welle. Für die Auswertung der so entstandenen Spektren werden so genante Data-Mining-Methoden verwendet. Die Messtechnik wurde mit Hilfe von mehreren Proben im Labor als auch auf laufenden Biogasanlagen ausprobiert. Die Validierung der Messtechnik auf einer landwirtschaftlichen Biogasanlage mit einer typischen Substratzusammensetzung ergab eine gute Korrelation (Korrelationskoeffizient von 0,972) für TS und oTS zwischen der online und im Labor gemessenen Werten. Insgesamt wurden 638 Tageswerte miteinander korreliert. Laut Autoren kann die Messtechnik einen weitergehenden Beitrag zur Erfassung und Bilanzierung der Stoffströme an Biogasanlagen leisten.

Kujawski und Steinmetz [18] berichten über Monitoring der Fermentation auf drei full-scale Biogasanlagen. Dabei wurden auf dem Markt verfügbare Messtechniken eingesetzt und getestet. Eine genaue Datenanalyse verdeutlicht Defizite und Vorteile einzelner Messeinrichtungen. Für die Überwachung der Konzentration an flüchtigen organischen Säuren hat sich die Titration bewährt. Es wird belegt, dass ein wichtiger Indikator des Anstiegs der Säurekonzentration das Redox-Potenzial ist. Dabei wird auch eine Korrelation zwischen den flüchtigen organischen Säuren und dem Redox-Potenzial entdeckt. Dabei wurde eine Hypothese über den Zusammenhang des Redox-Potenzials und des Oxidationspotenzials der Reaktion der Oxidation der organischen Säuren zu Essigsäure (Essigsäurebildung). Des Weiteren wurde eine Korrelation der Ergebnisse der Leitfähigkeit und deren Korrelation mit der Alkalinität vorgestellt. Die Zusammenhänge können für die Steuerung und die Regelung der Biogasanlagen von Bedeutung sein. Die Autoren weisen darauf Hin, dass die Wahl eines optimalen Messsystems von der Anlagenkonstellation und vor allem von der Substratzusammensetzung abhängt. Dabei soll für jeden einzelnen Messparameter ein Kompromiss zwischen der Verfügbarkeit und der Genauigkeit getroffen werden. Kujawski und Steinmetz belegen auch, dass ein Mangel der Messtechnik zu erheblichen Schwierigkeiten, insbesondere auf Anlagen die eine variable Inputzusammensetzung haben sowie Substrate mit hoher Abbaukinetik einsetzen, führen kann.

2.7.2 Stand von Steuerungs-, Regelungs- und Automatisierungskonzepten für Biogasanlagen

Das Steuern nach DIN 19226 "ist ein Vorgang in einem System, bei dem eine oder mehrere Eingangsgrößen eine oder mehrere Ausgangsgrößen auf eine bestimmte Art und Weise beeinflussen. Der Informationsfluss ist dabei offen. Ein Signal wird in das technische System als Eingangsgröße eingegeben und macht sich im System an einer anderen Stelle als Ausgangsgröße bemerkbar. Diese Ausgangsgröße beeinflusst die Eingangsgröße in keiner Weise."

Das Regeln nach DIN 19226 ist "ein Vorgang, bei dem fortlaufend eine Größe, die Regelgröße (zu regelnde Größe), erfasst, mit einer anderen Größe, der Führungsgröße, verglichen und im Sinne einer Angleichung an die Führungsgröße beeinflusst wird."

Der Stand der Steuerungs- und Regelungstechnik auf Biogasanlagen ist noch relativ unterentwickelt [19]. Der Automationsgrad der meisten Anlagen hat niedriges Niveau. Viele Anlagen werden immer noch per Hand gesteuert. Damit finden Entwicklungen die z. B. in den letzten 20 Jahren auf den Kläranlagen in dieser Beziehung stattgefunden haben nahezu keine Berücksichtigung auf Biogasanlagen [19].

In der Praxis werden überwiegend nur einfache Steuerungs- und Regelungsstrategien umgesetzt wie z. B. Substratzugabe nach Gewicht und Dosierung des Sauerstoffs zur Entschwefelung anhand der $O_2$- und der $H_2S$ Konzentration im Biogas. "Viele Anlagen besitzen an Steuer- und Regelungstechnik nur die Minimalausstattung. Dies sind einfache Zeitrelais zur Steuerung der Pump- und Rührintervalle."[2].

Im Vergleich zur Entwicklung der Messtechnik tut sich im Bereich der Steuerungs- und Regelungstechnik wesentlich weniger. Dies ist jedoch logisch, da erst die Entwicklung einer Messtechnik die Entwicklung der Steuerungs- und Regelungstechnik ermöglicht.

Im Rahmen von wissenschaftlichen Projekten werden neue Steuerungs- und Regelungsstrategien überwiegen im Labormaßstab anstatt in der Großtechnik entwickelt. Im Folgenden werden die bisher bekannten Ergebnisse von laufenden und abgeschlossenen Forschungsvorhaben dargestellt.

In den letzten Jahren wurde mit Hilfe komplexerer Kontrollansätze die Effizienz und Anwendbarkeit zur Steuerung von Anaerobanlagen erhöht. Insbesondere im Rahmen des EU-Projektes "Telemac" wurden verschiedene Regelungsstrategien auf die Anaerobprozesse angewendet sowie entsprechende Hard- und Softwarekomponenten entwickelt [27].

Heinzle et al. [12] veröffentlichte eine der ersten Untersuchungen zur Regelung und Steuerung von Anaerobanlagen, die in erster Linie jedoch mit einfachen Reglern ausgestattet waren.

Scherer et al. [28] berichtet über den Einsatz von Fuzzylogiccontrolern (FLC). Im Laufe eines wissenschaftlichen Projektes wurde die Steuerungsstrategie für Fermenter im Labormaßstab entwickelt und optimiert. Die Untersuchung wurde an einer Pilotanlage mit einem 1 m$^3$ Fermenter und Gerstenschrot sowie Rübensilage durchgeführt. Am Anfang wurde insbesondere die Gasbildungsrate als Regelgröße verwendet und anschließend der pH-Wert sowie die CH$_4$- und Gasbildungsrate. Anhand der Angaben der Autoren ermöglichten die FLC einen stabilen Betrieb des Versuchsreaktors auch bei einer organischen Belastung von über 14 kg oTM/(m$^3$ d) sowie einer kurzen hydraulischen Aufenthaltszeit von 6,5 Tagen.

Wolf et al. [37] berichtet über einen Einsatz der mathematischen Modellierung mit Hilfe von Anaerobic Digestion Model No 1 (ADM 1) für die Steuerung und Regelung von Biogasanlagen. Mit Hilfe der parallelen Simulation sollen auf einer großtechnischen Biogasanlage, die anaerobe Prozesse simuliert werden. Das Steuerungssystem vergleicht die angestrebten Ziele und den aktuellen Zustand des Prozesses. Die Regelung und die Steuerung erfolgten mit Hilfe von genetischen Algorithmen. Damit wurden die Entscheidungen über Inputmenge und -Zusammensetzung getroffen. Damit war es möglich die Produktivität der Biogasanlage um 25 % zu erhöhen [37]. Das Steuerungssystem sollte in 2007 installiert und erprobt werden. Bisher fehlen zu diesem Projekt weitere Angaben.

Cimatoribus hat im Rahmen ihrer Doktorarbeit [4] ein mathematisches Modell der Biogasanlage implementiert und kalibriert. Anschließen hat sie einen nichtlinearen adaptiven Regelungsalgorithmus mittels einer Feedback-Linearisierungsmethode entworfen. Die Regelung wurde mit Hilfe des Modells auf einer NawaRo Biogasanlage sowie einer Bioabfallvergärungsanlage simulatorisch getestet. Das getestete Regelungssystem hat insbesondere die Konzentration der organischen Säuren kontrolliert. Der Regelungsalgorithmus ist dank des Adaptationsmechanismus bei gleichzeitig geringem Messaufwand sehr flexibel gewesen. Die Regelungsstrategie wurde auf einer typischen landwirtschaftlichen sowie Co-Vergärungsanlage [17] erfolgreich getestet. Nach Cimatoribus [4] ist diese Regelungsstrategie nach Anpassung der Reglerparameter auch auf andere Anlagen und Substrate anwendbar.

Franke et al. [10] und Prechtl et al. [26] berichtet über die Entwicklung einer Regelungsstrategie für Fermenter, bei der die Substratmenge auf dem Regelwert H$_2$-Konzentration im Biogas basierte. Die Untersuchungen wurden im Labormaßstab mit verschiedenen Substraten durchgeführt: Grasschnitt, Hähnchenfestmist, Maissilage, Getreidekorn und Zuckerrübenschnitzel. Solange eine vorgegebene Konzentration an H$_2$ unterschritten wurde, wurde die Raumbelastung der Fermenter gesteigert. Bei Überschreitung des Grenzwertes wurde die Raumbelastung bis zur Unterschreitung der kritischen H$_2$-Konzentration ausgesetzt bzw. reduziert. Die Autoren weisen darauf hin, dass die Regelungsstrategie sich noch in der Entwicklungsphase befindet, da selbst während der Untersuchung einige Defizite, z. B. schwierige Indikation der Überlastung aufgrund der schwach ausgeprägten H$_2$-Peaks, festgestellt worden sind.

Warmenhoven et al. [31] berichtet über die Entwicklung einer Regelung von hochbelasteten (von 11,3 bis 14,0 kg oTM/(m$^3$ d) expanded-granular-sludge-bed-reactor (EGSB-Reaktor) für die Vergärung von Abwasser aus der Fruchtverpackungsindustrie. Die Regelgröße im Fall des Systems ist der gesamte organische Kohlenstoff (TOC), der inline im Zulauf des Reaktors gemessen wurde. Der Einsatz der Steuerungsstrategie führte zur Senkung der Fluktuationen in der organischen Belastung des Fermenters. Obwohl der Pufferbehälter im Zulauf der Anlage sehr klein war, war es mit Hilfe der neuen Regelung möglich, die Fermentation stabil zu fahren. Laut der Aussage der Autoren wurde nach der Implementierung der CSB-Abbau in der Anlage verbessert.

## 3 Materialien und Methoden

### 3.1 Beschreibung der untersuchten Anlagen

Für die Analyse wurden drei Biogasanlagen mit einem ähnlichen verfahrenstechnischen Konzept, einer unterschiedlichen Inputzusammensetzung und einer überdurchschnittlichen messtechnischen Ausstattung gewählt.

Auf den untersuchten Biogasanlagen wird die methanogene Fermentation als zweistufiges Speicher-Durchfluss-Vefahren im Fermenter und Nachgärer realisiert. Das heißt, dass der Fermenter mit dem Rohsubstrat am stärksten belastet wird. Anschließend gelingt das teilweise ausgegorene Material mit einem geringen Zusatz an Rohsubstrat oder ohne Rohsubstrat vom Fermenter zum Nachgärer. Beide Stufen arbeiten auf den untersuchten Biogasanlagen im mesophilen Temperaturbereich. Die TM-Gehalte in Fermenter liegen im Bereich von 5 bis 9 %, da auf den Anlagen eine sog. Nassfermentation realisiert wird. Die pH-Werte liegen in beiden Fermentationskammern im Bereich von 7,0 bis 7,8, sodass ein simultaner anaerober Abbau- und Biogasbildungsprozess mit dem Ziel maximaler Methanproduktion stattfinden kann. Die Art des Verfahrens wird zurzeit, aufgrund ihrer verfahrenstechnischen Zuverlässigkeit, Flexibilität, Sicherheit des Betreibens und Redundanz, sehr häufig eingesetzt [8]. Im Folgenden werden die gewählten Anlagen detailliert vorgestellt.

3.1.1 Beschreibung der Verfahrenstechnik der untersuchten Biogasanlagen

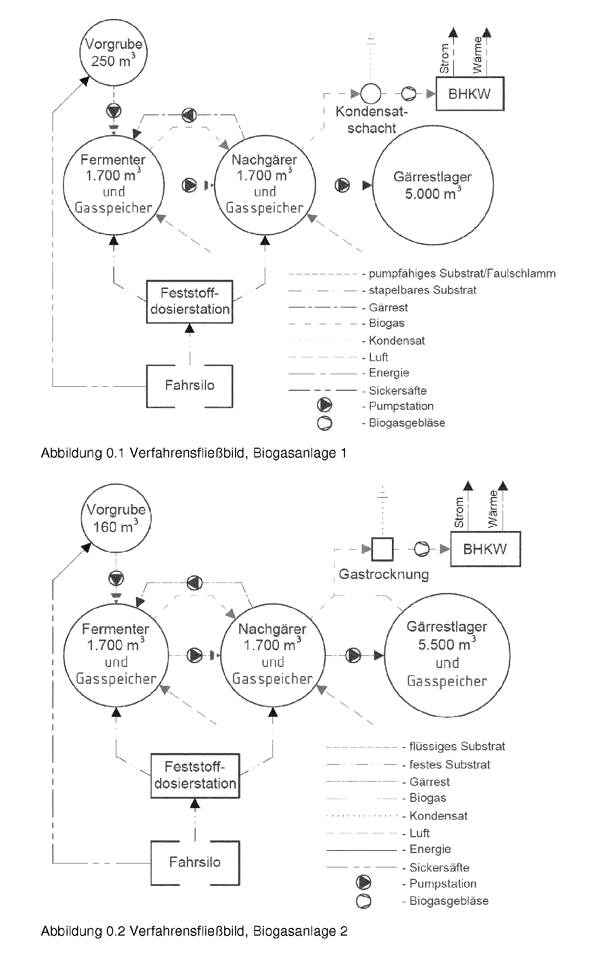

Die Biogasanlage 1 wurde in 2005 gebaut und um die Jahreswende 2005/2006 in Betrieb genommen. Die Biogasanlage verwertet überwiegend landwirtschaftliche Substrate wie Maissilage, Gülle, Grünroggen, Sudangras, Hühnerkot sowie Zucker- und Futterrüben und wird daher als NawaRo-Biogasanlage bezeichnet. Abbildung 0.1 ist das Verfahrensfließbild der Anlage und die Hauptwege der Stoffströme zu entnehmen. Die stapelbaren Substrate werden mit Hilfe von einer sogenannten Feststoffbeschickungsstation, die über eine Vorlage und ein Schneckensystem verfügt, direkt in den Fermenter und den Nachgärer eingebracht. Pumpfähige Substrate werden von der Vorgrube mit Hilfe der in der Pumpstation trockenaufgestellten Drehkolbenpumpe in den Fermenter oder in den Nachgärer zugegeben. Nach der Vergärung in der letzten Stufe (Nachgärer) gelangt der ausgegorene Faulschlamm in den Gärrestspeicher.

Das Biogas wird vor der Verwertung auf dem biologischen Weg (Sulfobakter oxydans) mit Hilfe der Einblasung geringer Luftmengen in die Gasspeicher des Fermenters und des Nachgärers entschwefelt. Des Weiteren wird das Biogas mit Hilfe des Gaskühlers entfeuchtet. Das produzierte Methan wird von einem Block-Heiz-Kraft-Werk (BHKW) in Strom und Wärme umgewandelt. Der elektrische Wirkungsgrad des BHKW beträgt ca. 36 % und die maximale Energieproduktion etwa 550 $kW_{el}$. Die maximale Wärmeproduktion vom BHKW beträgt ca. 600 $kW_{th}$. Die Wärme wird primär für die Beheizung des Fermenters und des Nachgärers genutzt. Auf der Biogasanlage wurde ein Wärmenutzungskonzept verwirklicht, sodass überschüssige Wärme für die Trocknung von Holzhackschnitzeln oder Getreide genutzt wird. Das Feuchtmaterial wird in speziell dafür entwickelten Containern transportiert und in ihnen mit der warmen Luft getrocknet.

Das besondere Augenmerk des verfahrenstechnischen Konzeptes der Anlage gilt der Konstruktion der Pumpstation der Anlage, die das Umpumpen von jedem Behälter zu jedem Behälter der Anlage mit Hilfe nur einer Drehkolbenpumpe ermöglicht. Dafür wurde jeder Behälter mit einer Saug- und einer Druckleitung an den Verteiler in der Pumpstation angeschlossen. Der Pumpweg wird beim jeweiligen Pumpvorgang durch das Öffnen der automatischen Schieber der entsprechenden Saug- und Druckleitung gewählt. Sämtliche Pumpvorgänge der Biogasanlage werden somit mit nur einer Pumpe bedient. Die Konzeption der Pumpstation bietet einige Vorteile beim Einsatz der Online-Messtechnik.

Die Biogasanlage 1 setzt sich aus folgenden Komponenten zusammen (Abbildung 0.1):

● Fahrsilo für Maissilage, Nutzvolumen ca. 12.000 $m^3$

● Vorgrube für Annahme von angelieferten Flüssigsubstraten (überwiegend Gülle), Nutzvolumen ca. 250 $m^3$

● Ein Fermenter und ein Nachgärer mit ca. je 1.700 $m^3$ Nutzvolumen und auch als Gasspeicher genutzt

● Gärrestlager, Nutzvolumen ca. 5.000 $m^3$

● Betriebshalle mit Steuerwarte, Schaltschrankraum, Pumpstation, Feststoffbeschickungsstation, BHKW-Raum

● Wärmenutzungskonzept für Trocknung von Holzhackschnitzeln, Getreide o. ä.

Die Biogasanlage 2 wurde in 2007 gebaut und um die Jahreswende 2007/2008 in Betrieb genommen. Die verfahrenstechnische Konzeption sowie Substratzusammensetzung der Anlage sind der Biogasanlage 1 sehr ähnlich. Die Anlage produziert Energie aus landwirtschaftlichen Substraten wie Maissilage, Schweinegülle sowie Grünroggen und wird daher genauso wie die BGA 1 als NawaRo-Anlage bezeichnet. Die Eintragstechnik der Anlage wurde identisch wie auf der BGA 1 ausgeführt. Im Gegensatz zur BGA 1 wurde der Gärrestbehälter der Biogasanlage mit einem gasdichten Dach abgedeckt. Der Abbildung 0.2 Verfahrensfließbild, Biogasanlage 2 ist das Verfahrensfließbild der Anlage mit den Hauptwegen der Stoffströme zu entnehmen.

Die Biogasanlage 2 setzt sich aus folgenden Komponenten zusammen:

● Fahrsilo für Maissilage mit Nutzvolumen von ca.12.000 $m^3$

● Vorgrube für Annahme von angelieferten Flüssigsubstraten (überwiegend Gülle) mit Nutzvolumen von ca. 160 $m^3$

● Ein Fermenter und ein Nachgärer mit ca. je 1.700 $m^3$ Nutzvolumen und Gasspeichern

● Ein gasdicht überdachte Gärrestlager mit ca. 5.000 m$^3$ Nutzvolumen (Flüssigkeit)

● Betriebshalle mit Steuerwarte, Schaltschrankraum, Pumpstation, Feststoffbeschickungsstation, BHKW-Raum

● Installation für Gastrocknung

Die Konzeption der Pumpstation der Anlage ist vergleichbar mit der der BGA 1.

Nach der biologischen Entschwefelung im Fermenter und Nachgärer wird das Biogas mit Hilfe eines Gastrocknungsmoduls aufbereitet. Das Modul kühlt das Biogas effektiver als der Gaskühler der BGA 1 ab, daher soll die Entfeuchtung hier besser funktionieren.

Das gesamte Biogas wird im BHKW mit einer maximalen Leistung von 534 kW$_{el}$, verstromt. Dabei entsteht auch die Wärme, die teilweise für die Beheizung der Fermentationskammer benötigt wird. Es wurden verschiedene Konzepte für die Verwertung der überschüssigen Wärme wie z. B. Holzhackschnitzeltrocknung oder Nahwärmenetz geplant. Bis das Messprogramm abgeschlossen wurde, wurde noch kein Wärmenutzungskonzept realisiert.

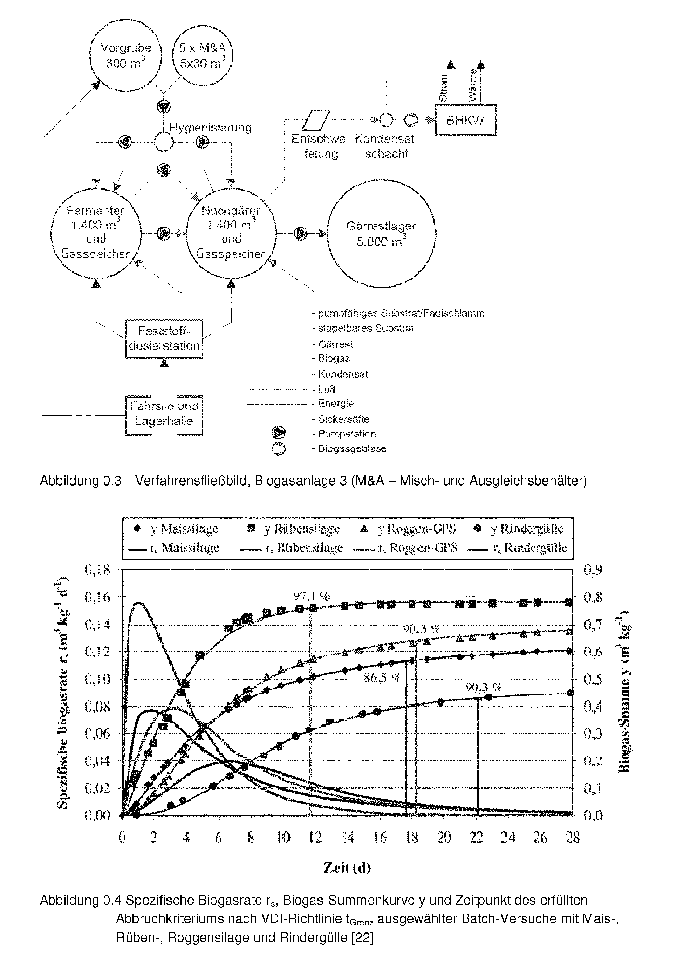

Biogasanlage 3 wurde in 2007 gebaut und in Betrieb genommen. Die verfahrenstechnische Konzeption der Anlage unterscheidet sich etwas von der BGA 2 und der BGA 1, da auf der Anlage auch andere Substrate wie hygienisierungspflichtige Abfälle aus der Lebensmittelindustrie oder energiereiches Glycerin verwertet werden. Außerdem werden auf der Anlage auch landwirtschaftliche Substrate wie Schweinegülle, Hühnermist oder Maissilage verwertet. Die Anlage kann also als Co-Vergärungsanlage bezeichnet werden. Aufgrund der Vielfältigkeit des Inputs musste die Anlage mit zusätzlichen verfahrenstechnischen Komponenten ausgerüstet werden. Abbildung 0.3 ist Verfahrensfließbild der Anlage mit Hauptwegen des Stoffströme zu entnehmen.

Für die Lagerung der flüssigen Substrate wurden auf der Anlage insgesamt fünf Behälter errichtet. Damit können Substrate mit unterschiedlicher Zusammensetzung getrennt gelagert und entsprechend miteinander vermischt zudosiert werden.

Die Anlage verfügt über eine Hygienisierungsstation, in der die hygienisierungspflichtigen Substrate mit einer Temperatur von 70 °C über eine Stunde, gemäß der EU Verordnung 1774/2002, behandelt werden. Aufgrund besonderer Anforderungen an die Hygiene des Betriebes wurde die Anlage in eine schwarze und weiße Zone unterteilt. Somit wurden die Leitungen der Pumpstation der Biogasanlage in zwei Zonen getrennt. Die schwarze Zone dient zur Förderung der rohen hygienisierungspflichtigen Substrate. Über die weiße Zone werden die nicht hygienisierungspflichtigen Substrate sowie der Faulschlamm gefördert. Anders als bei den beiden zuvor besprochenen Anlagen sind hier zwei Kolbenpumpen (Verdrängerpumpen) im Einsatz. Die Pumptechnik wurde vor allem deswegen eingesetzt, da die angelieferten Substrate oft Störstoffe wie z. B. Verpackungsmaterial oder Därme beinhalten können.

Da die Ausgangssubstrate der Anlage wie Schweinegülle oder Lebensmittelabfälle in der Regel erhöhte Schwefelkonzentrationen beinhalten können, verfügt die Anlage zusätzlich zur biologischen über eine externe nasschemische Entschwefelung.

Die Biogasanlage 3 setzt sich aus folgenden Komponenten zusammen:

● Fünf Misch- und Ausgleichsbehälter für Rohsubstrate

o Vorgrube für überwiegend Schweinegülle mit ca. 300 m$^3$

o Drei Misch- und Ausgleichsbehälter mit 30 m$^3$ je für z. B. pumpfähige Lebensmittelabfälle

o Ein beheizter Behälter für Glycerin von ca. 40 m$^3$

● Fahrsilo von ca. 400 m$^3$ für stapelbare Substrate wie z B. Maissilage oder Hühnerkot

● Ein Fermenter und ein Nachgärer mit ca. je 1.400 m$^3$ Nutzvolumen und Gasspeicher

● Ein Gärrestlager mit ca. 5.000 m$^3$ Nutzvolumen

● Externe Entschwefelung (mit Hilfe von Eisenoxid)

● Betriebshalle mit Steuerwarte, Schaltschrankraum, Pumpstation, Feststoffbeschickungsstation, BHKW-Raum

Genauso wie auf den zwei zuvor vorgestellten Biogasanlagen wird das gesamte Biogas mit Hilfe des BHKW mit einer maximalen Leistung von 714 kW$_{el}$, verstromt. Dabei entsteht überschüssige Wärme, von der ein Teil für die Beheizung des Fermenters und des Nachgärers sowie die Hygienisierung der Eingangssubstrate benötigt wird. Der Rest der Wärme wird für das Heizen von benachbarten Betrieben und Wohnhäusern verwendet.

3.1.2 Beschreibung der MSR-Technik der untersuchten Biogasanlagen

3.1.2.1 Online-Messtechnik

Die Biogasanlagen 3, 2 und 1 wurden überdurchschnittlich mit Online-Messtechnik im Vergleich zu anderen deutschen Anlagen ausgestattet (Tabelle 3.1).

Auf den Anlagen 1 und 2 wurden mehrere Online-Messungen in der Pumpstation installiert. Dank der besonderen Konstruktion der Pumpstation wird für die Messung der Parameter Durchfluss, TS, Leitfähigkeit, pH, Redox-Potenzial und Temperatur (bzw. andere Parametern die mit Hilfe von NIR-Spektrometer gemessen werden können) in dem Rohsubstratstrom bzw. in dem Faulschlamm gemessen. Dies ist möglich, weil über die Pumpstation von jedem Behälter zu jedem Behälter umgepumpt werden kann. Diese Lösung hat einige Vorteile, die später detailliert angesprochen werden.

Tabelle 3.1 Zusammenstellung der Online-Messtechnik auf untersuchten Biogasanlagen (BGA W-Biogasanlage 3, BGA L 0- Biogasanlage 1, BGA N - Biogasanlage 2)

| Einbaustelle | Messung und Messmethode | BGAW | BGAL | BGAN |
|---|---|---|---|---|
| Pumpstation | Magnetisch-Induktive-Durchflussmesser (MID) | | x | X |
| | Temperaturfühler (pt100) | x | | x |
| | pH-Sensor | | x | x |
| | Redox-Sensor (Salzbrücke) | | x | x |
| | Leitfähigkeit-Sensor | | | x |
| | TS-Sensor (optisch) | | x | x |
| | NIRS** | | x | |
| Biogasrohrleitung | Gasdurchfluss (Gas-Massen-Durchflussmesser), | x | x | x |
| | Gasanalysator: $CH_4$ (IR-Sensor), $CO_2$ (IR-Sensor), $O_2$ (elektrochemischer Sensor), $H_2S$ (elektrochemischer Sensor) | x | x | x |
| Feststoffdosier-station | Waage | x | x | x |
| | NIRS** | | x | |
| Fermenter und Nachgärer | Füllstandsmessungen: Gasphase, Flüssigphase | x | x | x |
| | Temperaturfühler (pt 100), | x | x | x*** |
| | Videocontroller | | | x |

\* - Redox-Potenzial, \*\* - NIRS - Nahinfrarotspektroskop - getestet auf der Biogasanlage während der Messkampagne.
\*\*\* - In dem Fermenter der Biogasanlage 2 wurden zusätzlich 32 Temperatursensoren installiert

Der Substratdurchfluss wurde mit Hilfe von einem Magnetisch-Induktiven-Durchflussmesser der Firma Siemens Typ Sitrans FM Magflo Mag 5000 W gemessen. Für die Temperaturmessung in der Pumpstation sowie im Fermenter wurden Sensoren der Firma Jumo verwendet. Die Messung von pH und Redox-Potenzial erfolgte mit Hilfe von der Differenzialelektroden pHD S sc vom Messgerätehersteller Hach-Lange. Das Besondere an den Elektroden ist die geschlossene Bauweise, die das Referenz-System vom Messmedium schützt, wodurch eine Elektrodenvergiftung ausgeschlossen ist. Demzufolge wird die Elektrode von dem im Substrat vorhandenen Schwefelwasserstoff geschützt. Die wesentlich schmutzunempfindlichere Salzbrücke reduziert den erforderlichen Reinigungsaufwand im Vergleich zu Systemen mit Diaphragmen. Auch Verdünnungen des Elektrolyts werden so verhindert. Die Leitfähigkeit wurde mit Hilfe des digitalen induktiven Leitfähigkeitssensors 3798 S sc von Hach-Lange gemessen. Der Trockensubstanzgehalt in dem flüssigen Substrat wurde mit Hilfe von Solitax sc von Hach-Lange mit einer entsprechenden Rohreinbauarmatur gemessen. Alle vier Sensoren wurden an den SC 1000 Controller der Firma Hach-Lange angeschlossen.

Während einer Messkampagne, die von Juni 2007 bis Dezember 2007 dauerte, wurde die Nahinfrarot-Messtechnik (NIR-Messtechnik) kalibriert und Schätzmodelle für Online-Messungen entwickelt. Auf der Biogasanlage 1 wurden insgesamt zwei NIR-Spektrometer installiert. Zuerst wurde ein Spektrometer auf der Rohrleitung in der Pumpstation eingebaut. Das zweite Spektrometer wurde an dem Vorlagebehälter der Feststoffdosierungsstation eingebaut.

Die Gasanalysatoren, die auf den untersuchten Anlagen verwendet wurden, sind in Tabelle 3.2 zusammengestellt. Die Infrarotmesszellen, die für die Methan- sowie Kohlenstoffdioxid-Bestimmung verwendet wurden, zeichnen sich, im Gegensatz zu den elektrochemischen Messesonden, durch einen robusten Betrieb und eine geringe Häufigkeit der Kalibrierung aus. Für Sauerstoff und Schwefelwasserstoff wurden elektrochemische Sensoren verwendet, die regelmäßig erneuert und kalibriert werden mussten.

Tabelle 3.2 Zusammenstellung der Gasanalysegeräte auf den untersuchten Biogasanlagen

|  | BGA 1 | BGA 2 | BGA 3 |
|---|---|---|---|
| Hersteller | ExTox | CHEMEC | PRONOVA |
| Typ | ET - 8D | BC 20 | SSM 6000 |
| Messung von $CH_4$ | IR | IR | IR |
| Messung von $CO_2$ | IR | IR | IR |
| Messung von $O_2$ | el.-chem. | el.-chem. | el.-chem. |
| Messung von $H_2S$ | el.-chem. | el.-chem. | el.-chem. |
| IR - Infrarot, el.-chem. - elektrochemisch | | | |

Auf der Biogasanlage 2 und auf der Biogasanlage 3 wurden zusätzlich Druck- sowie Temperatursensoren eingebaut, sodass der Durchfluss in Normkubikmeter gemessen wurde.

3.1.3 Begründung der Auswahl der Anlagen

Alle untersuchten Biogasanlagen wurden ähnlich nach dem Speicher-Durchfluss-Verfahren mit zwei Gärbehältern, d. h. einem für deutsche Verhältnisse typischen Verfahrenskonzept, konzipiert. Dadurch können Ergebnisse und Schlussfolgerungen der Untersuchung auf viele ähnliche Objekte übertragen werden. Zum anderen vereinfacht die ähnliche Verfahrenstechnik der Anlagen die Analyse des Einflusses der unterschiedlichen Inputzusammensetzungen auf das Verhalten und die Bedürfnisse hinsichtlich eines entwickelten anlagenweiten Steuerungs- und Regelungssystems für die Biogasanlagen.

Da ein Einsatz der Messtechnik und Automatisierungstechnik auf den drei Biogasanlagen im Vergleich zu anderen deutschen Biogasanlagen überdurchschnittlich ist, konnten verhältnismäßig viele Informationen über die Prozessabläufe in Form von Betriebstagebüchern und Datenbanken gesammelt werden. Des Weiteren konnten auch die vorgegebenen Betriebsstrategien (z. B. quasikontinuierliche Fermenterbeschickung, Rühren in festen Zeitabständen) gemäß der Planung ausgeführt werden. Mit Hilfe der Fernwirktechnik über Internetverbindung konnte der Betrieb der Anlagen genau verfolgt werden sowie die Absprache und der Erfahrungsaustausch mit den Betreibern einfacher durchgeführt werden.

3.2 Beschreibung der Datengrundlage der Entwicklungsarbeiten

3.2.1 Datenbanken und Betriebstagebücher

Für die Auswertungen standen Datenbanken der untersuchten Biogasanlagen zur Verfügung. In den Datenbanken befanden sich zahlreiche Informationen:

● Messwerte der Online-Messtechnik

● Zählerstände

● Laufzeiten der Antriebe

● Zustandsaufzeichnungen (z. B. Zeitpunkte des Einschaltens und des Ausschaltens der Antriebe)

● Alarm- und Meldelisten mit Statusmeldungen sowie Warn- und Alarmmeldungen

Für die Anfertigung dieser Arbeit wurden die Datenbanken und Betriebstagebücher mit Aufzeichnungen in den folgenden Zeiträumen analysiert:

● BGA 1: von Januar 2006 bis Dezember 2007

● BGA 3: von Oktober 2006 bis November 2008

● BGA 2: von Dezember 2007 bis Dezember 2008

3.2.2 Messkampagnen

Auf den untersuchten Biogasanlagen wurden umfangreiche Messkampagnen realisiert. In Tabelle 3.3 und Tabelle 3.4 wurden die auf den untersuchten Anlagen durchgeführten Offline-Messungen zusammengestellt. Dabei wurden auch die Messmethoden aufgelistet. Außer der Überwachung des Fermentationsprozesses wurden die Messkampagnen unter der Berücksichtigung der folgenden Ziele konzipiert und durchgeführt:

● Die Überprüfung der Messgenauigkeit der einzelnen Messtechniken

● Die Überprüfung der Störanfälligkeit der einzelnen Messtechniken im Einsatz auf Biogasanlagen mit unterschiedlichen Substraten

● Die Kalibrierung und das Ausprobieren von neuen Messtechniken (Daten wurden für die Kalibrierung vom NIR-Spektrometer genutzt sowie für das Testen von neuartiger Messtechnik zur Messung der Carbonatpufferkapazität mit Hilfe von BiogasPro)

● Die Überprüfung von Drift und von Shift der Online-Messtechnik der Anlagen

● Die Erklärung der Abweichungen bei Messungen von ähnlichen Prozessparametern mit unterschiedlichen Messtechniken (z. B. Messung von organischen Säuren mit Hilfe von Ionenchromatografie und Titration)

Auf den einzelnen Biogasanlagen wurden die Messkampagnen in folgenden Zeiträumen durchgeführt:

● BGA 1: vom Mai 2007 bis November 2007

● BGA 3: vom Mai 2007 bis November 2007 und vom Februar 2008 bis Mai 2008

● BGA 2 vom Dezember 2007 bis Juni 2008

Tabelle 3.3 Zusammenstellung von Offline-Messungen, die vor Ort auf den untersuchten Biogasanlagen im Einsatz waren.

| Messparameter | Messmethode, Messtechnik | Proben | BGA 1 | BGA 2 | BGA 3 |
|---|---|---|---|---|---|
| Organische Säuren (Essigsäureäquivalent) | Schnelltests Hach-Lange, Test Nr. LCK 365 | FS,G | x | x | |
| CSB | Schnelltests Hach-Lange, LCK 014 und LCK 114 | FS,S,G | x | x | |
| Säurekapazität | Schnelltests Hach-Lange, LCK 362 | FS, S,G | x | x | |

(fortgesetzt)

| Messparameter | Messmethode, Messtechnik | Proben | BGA 1 | BGA 2 | BGA 3 |
|---|---|---|---|---|---|
| Flüchtige organische Säuren (FOS) | Modifizierte Nordmann Titration, halb automatischer Titrator Tim Aqua, Radiometer | FS | x | x | x |
| Carbonat-Pufferkapazität (TAC) | Modifizierte Nordmann Titration, halb automatischer Titrator Tim Aqua, Radiometer | FS | x | x | x |
| Trockenrückstand | Gemessen mit Hilfe von Feuchtebestimmer Typ IR 35 von Denver Instruments | FS, S, G | x | x | x |
| Carbonat-Pufferkapazität | Gemessen mit Hilfe von Biogas Pro | FS | x | x | x |
| pH | Handmessgerät SENSION von Hach-Lange | FS, S, G | x | x | x |
| Redox-Potenzial | Handmessgerät SENSION von Hach-Lange | FS, S, G | x | x | x |
| Leitfähigkeit | Handmessgerät SENSION von Hach-Lange | FS, S, G | x | x | x |
| BGA L - Biogasanlage 1, BGA N - Biogasanlage 2, BGA W - Biogasanlage 3, FS - Faulschlamm, S - Substrat | | | | | |

Tabelle 3.4 Zusammenstellung von Offline-Messungen, im externen Labor

| Messparameter | Messmethode, Messtechnik | Proben | BGA 1 | BGA 2 | BGA 3 |
|---|---|---|---|---|---|
| Flüchtige organische Säuren (FOS) | Modifizierte Nordmann Titration, halb automatischer Titrator Tim Aqua, Radiometer | FS | x | | |
| Carbonat-Pufferkapazität (TAC) | Modifizierte Nordmann Titration, halb automatischer Titrator Tim Aqua, Radiometer | FS | x | | |
| Trockenrückstand | W: berechnet<br>L,N: DIN 38414, S3 | FS | x | | |
| Organischer Trockenrückstand | W: berechnet<br>L,N: DIN 38414, S3 | FS | x | x | x |
| pH | DIN 38404 C5 | FS | x | | |
| Leitfähigkeit | DIN EN 27888 | FS | x | | |
| Ammoniumstickstoff | DIN 38406 E5-1 photometrische Bestimmung des Ammonium-Stickstoffs mittels Natriumdichlorisocyanat und Natriumsalicylat | FS | x | | |

(fortgesetzt)

| Messparameter | Messmethode, Messtechnik | Proben | BGA 1 | BGA 2 | BGA 3 |
|---|---|---|---|---|---|
| Organische Säuren (Essigsäureäquivalent) | W: GC/UV-Det. - Hausmethode (Labor) L, N - BGK Methodenbuch | FS | x | x | x |
| Essigsäure | W: GC/UV-Det. - Hausmethode (Labor) L,N: LUFA Nord-West 1/1 B-906 | FS | x | x | x |
| Propionsäure | W: GC/UV-Det. - Hausmethode (Labor) L,N: LUFA Nord-West 1/1 B-906 | FS | x | x | x |
| n-Buttersäure | W: GC/UV-Det. - Hausmethode (Labor) L,N: LUFA Nord-West 1/1 B-906 | FS | x | x | x |
| Isobuttersäure | GC/UV-Det. - Hausmethode (Labor) | FS | x | | |
| Valeriansäure | GC/UV-Det. - Hausmethode (Labor) | FS | x | | |
| Capronsäure | GC/UV-Det. - Hausmethode (Labor) | FS | x | | |
| Rohasche | W - GC/UV-Det. - Hausmethode (Labor) L, N: VDLUFA Bd. 111, Kap. 8.1 | FS, S | x | x | x |
| Rohprotein (NIR Verfahren) | VDLUFA Bd. III, Kap. 4.1.1 | S | | x | x |
| Rohfett B | VDLUFA Bd. III, Kap. 5.1.1 | S | | x | x |
| Rohfaser (NIR Verfahren) | VDLUFA Bd. III, Kap. 6.1.1 | S | | x | x |
| NFE | Errechnet aus Weender Analyse | S | | x | x |
| Mineralische Substanz | DIN 38414, S 3 | FS | | x | x |
| Gesamtstickstoff | DIN 19684 | FS | | x | x |
| Phosphor | ISO 11885 Emissionsspektrometrie mit induktiv gekoppeltem Plasma | FS | | x | x |
| Kalium | ISO 11885 | FS | | x | x |
| Calcium | ISO 11885 | FS | | x | x |
| Magnesium | ISO 11885 | FS | | x | x |
| Schwefel | ISO 11885 | FS | | x | x |
| Kupfer | ISO 11885 | FS | | x | x |
| Zink | ISO 11885 | FS | | x | x |
| BGA L - Biogasanlage 1, BGA N - Biogasanlage 2, BGA W - Biogasanlage 3, FS - Faulschlamm, S - Substrat | | | | | |

3.3 Analyse der Durchmischung des Reaktorinhalts und der Homogenität der Substrate

3.3.1 Entgasung des Faulschlammes

Derzeit gibt es keine Messtechnik für die Untersuchung der Dichte des Faulschlammes in Fermentern. Daher wurde für die Untersuchung ein Ansatz entwickelt.

Die Messungen wurden direkt im Fermenter der Biogasanlage 2 durchgeführt. Im Laufe der Untersuchung wurde die Dichteänderung des Substrates bei ausgeschalteten Rührwerken im Fermenter in verschiedenen Tiefen gemessen. Die Messung war mit Hilfe eines höhenverstellbaren Drucksensors möglich. Somit konnte der hydrostatische Druck in verschiedenen Tiefen des Fermenters gemessen werden. Der hydrostatische Druck wird mit Hilfe der bekannten Gleichung berechnet:

$$p = \rho \cdot g \cdot h \qquad\qquad \text{[Gleichung 3.1]}$$

$p$ - hydrostatischer Druck in Pa
$p$ - Dichte des Mediums in kg/m$^3$
$g$ - Erdbeschleunigung ca. 9,81 m/s$^2$
$h$ - Tiefe der Drucksonde unter dem Spiegel in m

Im Laufe der Untersuchung wurde festgestellt, dass der hydrostatische Druck nicht proportional der Tiefe ist, da die Dichte des Mediums aufgrund der Gasblasenkompression in verschiedenen Tiefen unterschiedlich ist. Daher wurde die Dichte des Mediums getrennt für jede Schicht des Behälters anhand Gleichung 5.2 berechnet.

$$\rho = \frac{\Delta p}{g \cdot \Delta h} \qquad\qquad \text{[Gleichung 3.2]}$$

$\Delta p$ - Erhöhung des hydrostatischen Druckes über Höhe der Schicht [Pa]
$\Delta h$ - Dicke der Schicht in [m]

3.3.2 Repräsentativität der Probe

Die Repräsentativität der Probe ist entscheidend für die Aussagekraft der Messung. Die Probenahme verläuft demzufolge relativ einfach, wenn das Material, das im Silo gelagert ist, homogen ist oder wenn ein Behälter gut durchmischt ist. Die Durchmischung der Biogasreaktoren, der Vorlagebehälter und der Gärrestspeicher ist wichtig hinsichtlich der Probenahme. Im Rahmen dieser Arbeit wurden mehrere Untersuchungen zur Repräsentativität der Probenahme der stapelbaren und pumpfähigen Substrate durchgeführt.

Auf den Biogasanlagen 1 und 2 wurden mehrere Untersuchungen zur Bestimmung der Eigenschaften der Maissilage durchgeführt. Es wurden unter anderem die Maisproben von verschiedenen Stellen des Fahrsilos genommen und deren TM-Gehalt analysiert. Die Ergebnisse haben gezeigt, dass Maissilage im Fahrsilo große Ungleichmäßigkeiten hinsichtlich der Trockenmasse aufweißt. Da die Untersuchungen besonders intensiv auf der Biogasanlage 1 durchgeführt worden sind, werden hauptsächlich diese Ergebnisse vorgestellt.

Im Rahmen der Arbeit wurden auf der Biogasanlage 3 die Stichproben der angelieferten Substratchargen untersucht. Die Biogasanlage verarbeitete während der Versuchsphase überwiegend pumpfähige Ausgangssubstrate. Das Volumen einer Charge des flüssigen Inputsubstrates betrug zwischen 19 und 26 m$^3$. Die Anlage verfügte über drei Substratbehälter mit einem Speichervolumen von je 30 m$^3$. Die einzelnen Chargen wurden praktisch unvermischt in die Speicherbehälter gegeben. Es wurden insgesamt acht Chargen untersucht. Dabei wurde der TM-Gehalt und der CSB gemessen.

Die Durchmischung in den Gärbehältern wurde mit Hilfe von Online-Prozesssonden für Messungen des Trockensubstanzgehaltes, pH, Redox-Potenzials und Leitfähigkeit untersucht, die in der zentralen Pumpstation der Biogasanlagen 1 und 3 installiert wurden. Damit wurde die Verteilung der Feststoffe sowie der gelösten Produkte des anaeroben Abbaus überprüft. Die Messsignale wurden während längerer Pumpvorgänge aufgenommen. Dabei wurden mehrere Kubikmeter des Faulschlammes (bis 40 m$^3$) über die Messstrecke umgepumpt.

3.4 Analyse des Einsatzes der Messtechnik auf den untersuchten Biogasanlagen

3.4.1 pH, Redox-Potenzial und Leitfähigkeit

Die Biogasanlagen 1 und 2 wurden mit der Prozessmesstechnik für Online-Messung von pH, Redox-Potenzial und Leitfähigkeit (nur BGA 2) ausgestattet. Die Messtechniken wurden auf den beiden Anlagen in der Pumpstation auf sogenannten Messstrecken eingebaut, sodass die Parameter in jedem Substratstrom gemessen werden konnten. Auf der Biogasanlage 3 wurde keine Online-Prozess-Messtechnik installiert.

Für die Online-Messsonden der BGA 1 und der BGA 2 wurden innerhalb der Messkampagne Untersuchungen zur Feststellung der optimalen Kalibrierungs- und Serviceintervallen durchgeführt. Dabei wurden die Messwerte der Online-Sonden mit den Messwerten des Handmessgerätes "Sension" der Firma Hach-Lange verglichen. Da das Handmessgerät vor jeder Messung kalibriert und validiert wurde, konnte anschließend die Abweichung des Online-Messwertes festgestellt werden.

Die Online-Redox-Potenzial-Sonden auf der Biogasanlage 1 und auf der Biogasanlage 2 wurden regelmäßig mit Hilfe des Standards (200 mV und 400 mV) überprüft und kalibriert. Eine Überprüfung des Messwertes mit Hilfe des Handmessgerätes wurde auch durchgeführt. Die Ergebnisse waren jedoch nicht zufrieden stellend, da die Redox-Potenzialmessung sehr träge ist und auch beim Handmessgerät sich sehr langsam an die im Substrat oder Faulschlamm herrschenden Bedienungen anpasst. In der Praxis betrug die Zeit der Anpassung einige dutzende Minuten. Gleichzeitig liefen in der teilweise belüfteten Substratprobe die Oxidations- und Reduktionsreaktionen ab, die das Potenzial veränderten. Die Leitfähigkeit wurde auf allen untersuchten Biogasanlagen mit Hilfe eines Handmessgerätes (Sension von Hach-Lange) gemessen. Nur auf der Biogasanlage 2 wurde zusätzlich eine Online-Messung (Typ 3798 S sc von Hach-Lange) installiert. Da die Messung auf dem berührungslosen, induktiven Messverfahren basiert, ist die Technik sehr robust und benötigt in der Regel keine Kalibrierung. Im Rahmen der Messkampagne wurde die Genauigkeit der Online-Messung mit einem Handmessgerät überprüft. Im Rahmen der Untersuchung wurde die Leitfähigkeits-Sonde einmal pro Quartal kalibriert. Dabei wurde mit Hilfe eines Standards (Salzlösungen mit bekannter Leitfähigkeit) die Sonde kontrolliert. Während der Messkampagne wurde beobachtet, dass die Öffnung (Innenring) der Sonde mit Substratpartikeln oder kleinen Steinen ab und zu verstopft wurde. Nach mehreren Versuchen wurde die Sonde entsprechend in der Rohrleitung angeordnet, sodass die größeren Partikel die Sondenöffnung nicht verstopften.

### 3.4.2 Organische Säuren

Die organischen Säuren wurden vor Ort auf den untersuchten Biogasanlagen mit Hilfe des halb automatischen Titrators (TIMAQUA von Radiometer) sowie mit Hilfe des Küvetten-Tests (photometrische Bestimmung mit Hilfe von Schnelltests von Hach-Lange) als auch extern in einem zertifizierten Labor (Gaschromatografie, Ionenchromatograpie) bestimmt. Da die gleichen Proben mit mehreren Messverfahren untersucht worden sind, konnten die Messmethoden miteinander verglichen werden.

Die Schnelltests wurden mit Hilfe des photometrischen Messverfahrens durchgeführt. Für die Untersuchung wurden die Faulschlammproben aufwendig vorbereitet. Sie wurden zuerst mit dem destillierten Wasser verdünnt, anschließend zentrifugiert und gegebenenfalls wieder verdünnt.

Die Bestimmung der flüchtigen organischen Säuren wurde nach einer modifizierten Nordmann-Titration durchgeführt. Für die Titration wurde ein halb automatischer Titrator Typ TIMAQUA von Radiometer genutzt.

### 3.4.3 Ammonium und Ammoniak

Ammonium wurde auf den untersuchten Anlagen in einem externen Labor (nach DIN 38406 E5-1) sowie mittels Küvetten-Tests von Hach-Lange bestimmt. Somit konnten die Ergebnisse der beiden Messverfahren miteinander verglichen werden.

### 3.4.4 Alkalinität und Säurepuffer

Die Alkalinität und der Säurepuffer wurden auf den untersuchten Anlagen mit Hilfe von drei Messverfahren untersucht: Schnelltests von Hach-Lange (LCK 362), Titration mit Hilfe des halb automatischen Titrators (TIMAQUA von Radiometer) und mit Hilfe von BiogasPro. Mit Hilfe von BiogasPro wird im Wesentlichen der Kohlensäure-Bicarbonat-Puffer bestimmt. Durch die Zugabe von Salzsäure zur Probe wird der pH-Wert soweit herabgesetzt, dass Kohlendioxid, das zuvor in Form von Bicarbonat und Kohlensäure in der wässrigen Phase vorlag, von der Probe als Gas entweicht. Die Menge an Gas, die proportional zum Säurepuffer ist, wird mit Hilfe von BiogasPro erfasst.

### 3.4.5 Biogasanalyse und Biogasmengenmessung

Die Biogasanalyse wurde auf den untersuchten Biogasanlagen mit Hilfe der installierten Online-Gasanalysatoren durchgeführt. Drift und Shift der Messwerte wurde untersucht, sodass Methangehaltsmessung korrigiert werden konnte.

Der Gasdurchfluss wurde auf allen untersuchten Biogasanlagen online mit Hilfe des Massen-Durchflussmessers erfasst. Zusätzlich auf der Biogasanlage 2 und auf der Biogasanlage 3 wurden Temperatur- sowie Druckfühler installiert, um die Normkubikmeter des Biogases erfassen zu können. Über die Korrelation der Messwerte für den korrigierten Methangehalt des Biogases, die Biogasmengenmessung und die erzeugte elektrische Energie ließ sich die Plausibilitätsprüfung der Messwerte durchführen sowie der wahre oder wahrscheinliche Wirkungsgrad des BHKW berechnen.

### 3.4.6 Erfassung der Massenströme - Durchflussmessungen

Die Massenströme der Substrate auf den Biogasanlagen 1 und 2 wurden mit Hilfe des IDM Durchflussmessers erfasst. Die Messwerte wurden im Rahmen der Arbeit auf deren Plausibilität bilanztechnisch überprüft. Dafür wurden einige größere Umpumpvorgänge auf jeder Anlage mit signifikanten Änderung des Füllendes ausgesucht und analysiert. Bei der Analyse wurde das über die Durchflussmessung erhobene Volumen mit dem Volumen, das über die Füllstandsmessung erfasst wurde, verglichen. Die Korrekturfaktoren wurden bei der Massenbilanzierung der Anlagen berücksichtigt.

3.4.7 Füllstands- und Volumenmessungen

Auf allen Anlagen wurden Füllstandsmessungen über Drucksensoren gemessen. Der tatsächliche Füllstandswert des Faulschlammes und des flüssigen Substrates in den Behältern hängt signifikant von der Dichte des Materials ab. Die Dichtemessungen wurden auf den Biogasanlagen 3 und 1 mit Hilfe von Gewichtsmessung der Proben mit einem bekannten Volumen durchgeführt. Die Dichtemessung auf der Biogasanlagen 2 wurde mit Hilfe eines höhenverstellbaren Drucksensors gemessen.

Auf den meisten deutschen Biogasanlagen werden die Gasbehälter mit den Fermentern, den Nachgärern sowie den Gärrestspeichern durch den Einsatz des Membrandachs integriert. Die Füllstandsmessung in den meisten Behältern erfolgt mit Hilfe einer Seilsonde. Die Messung gibt keinen faktischen Füllstand des Gasspeichers, sondern als Messwert wird nur die relative Seillänge angegeben (der Füllstand des Gasspeichers wird mit Hilfe des Messverfahrens als Verhältnis zwischen der genutzten Seillänge und der maximalen Seillänge ermittelt).

Im Rahmen der Arbeit wurde für die Gasspeicher der Biogasanlage 2 die Berechnung des Volumens durchgeführt. Die Berechnung des Volumens des Gasspeichers wurde mit Hilfe der CAD 3D Zeichnung durchgeführt. Die 3D Zeichnungen wurden für fünf Fälle mit gewählten unterschiedlichen relativen Seillängen angefertigt. Damit konnten die Volumina im einzelnen Fall EDV-technisch vermessen werden.

3.4.8 NIRS

Im Laufe der Messkampagne in 2007 auf der Biogasanlage 1 wurde die Kalibrierung der NIRS-Messtechnik zur Untersuchung von flüchtigen organischen Säuren (Essigsäureäquivalent), oTS und $NH_4$-N durchgeführt. Nach Ab-schluss der Kalibrierung wurde die Messtechnik abgebaut. Die Ergebnisse der Kalibrierung werden im Rahmen der Arbeit präsentiert

3.5 Entwicklung eines Modells zur dynamischen Erstellung von Massenbilanzen

3.5.1 Auswahl des Bilanzierungsmodells

Der Sinn und das Ziel der Modellierung ist eine möglichst genaue und zielgerichtete Beschreibung eines Systems. Ein Modell soll daher mit ausreichender Genauigkeit für eine bestimmte Aufgabe, bei vorgegebenen Randbedingungen und Inputdaten, Ergebnisse liefern. Durch Änderung der Randbedingungen sowie des Inputs eines Modells lassen sich diverse Arbeitsbedingungen für ein System testen, ohne dass es in der Realität gemacht werden muss.

Die Bestimmung von vielen Parametern des anaeroben Abbaus wie z. B. die Abbaurate der Substrate, die spezifische Methan- und Biogasausbeute, das Restgaspotenzial sowie von wichtigen Kontrollparametern wie z. B. die Konzentration der organischen Säuren, der Ammoniumgehalt und die Pufferkapazität sind mit Hilfe der zurzeit verfügbaren Messtechnik nur mit hohem Aufwand möglich. Die Online-Modellierung (das Mitlaufen des Modells im Betrieb sowie die Online-Kalibrierung anhand der Betriebsdaten) kann einige von diesen Parametern zumindest schätzen, indem die gesuchten Werte berechnet werden und somit Lücken zwischen z. B. genaueren Ergebnissen der Offline-Messungen (Labormessungen) gefüllt werden können. Damit können dynamische Daten generiert werden. Aus diversen Gründen ist der Einsatz der zurzeit verfügbaren Modelle der anaeroben Abbauprozesse (wie z. B. von ADM 1) für die Online-Simulation und für die Steuerung und Regelung der Biogasanlagen schwierig.

Auf landwirtschaftlichen und industriellen Biogasanlagen werden meistens Substrate mit einem relativ hohen TM- und oTM-Gehalt eingesetzt, da sie eine höhere Biogasausbeute pro Frischmasse (Gesamtmasse des Substrates) aufweisen und somit eine bessere Wirtschaftlichkeit garantieren. Die TM- und oTM-Konzentrationen des Inputs und Outputs sind deutlich höher als die, die z. B. in der Abwassertechnik bekannt sind. In der Abwassertechnik liegen die TM-Konzentrationen im Zulauf der Kläranlagen oft im Milligramm Bereich und im Input von anaeroben Schlammstabilisierungsreaktoren selten über 50 g/l TM. Auf den meisten Biogasanlagen liegt die TM-Konzentration im Input zwischen 100 und 300 g/l TS (z. B. Mais von 290 bis 330 g/l TM, Lebensmittelabfälle von 100 bis 250 g/l, Gülle von 40 bis 70 g/l und Molke von 40 bis 70 g/l). Aufgrund der hohen Konzentrationen von TM und oTM lassen sich die Massenströme der Trockenmasse und der organischen Trockenmasse auf Biogasanlagen relativ einfach erfassen. Die CSB-basierte Bilanzierung verliert somit im Verglich zur Abwassertechnik ihre Vorteile. Zusätzlich ist die Messung der CSB-Gehalte der vielen Substrate erschwert, da die vollständige Oxidation des organischen Materials oft technisch nicht möglich ist.

Dadurch spielen die Massenbilanzen an Biogasanlagen eine signifikant wichtigere Rolle als die CSB-Bilanzen im Gegensatz zur Abwassertechnik.

Bisher entwickelte Modelle basieren auf der Modellierung der Bakterienpopulation. Die Gesamtkinetik und -stöchiometrie des anaeroben Abbaus hängen sehr stark von der Anzahl sowie den Eigenschaften der Mikroorganismen ab. Auf der anderen Seite ist die analytische Quantifizierung der Bakterienarten zurzeit noch nicht wirklich möglich. Des Weiteren sind auch die meisten Bakterienspezies sowie deren Verhalten und Adaptionsvermögen weitgehend nicht erforscht. Es stellt sich daher die Frage, ob die aufwendigen Modelle bezüglich der Biomassepopulation so verifiziert werden können. Des Weiteren stellt sich die Frage, ob es zurzeit nicht sinnvoller wäre, Modelle zu entwickeln, die eine vereinfachte Gesamtkinetik des Prozesses beschreibt. So eine Vereinfachung ist durchaus in vielen Fällen (z. B. für die Online-Simulation, die Grobbilanzierung für die Planung der Anlagen) berechtigt und denkbar.

3.5.2 Grundlagen des entwickelten dynamischen Massenbilanzmodells

Im Folgenden werden die Grundlagen und Annahmen des Modells, welches im Rahmen der Arbeit entwickelt wurde, vorgestellt. Das Modell basiert auf den Massenbilanzierungen der organischen Trockenmasse und des Stickstoffs. Zudem wird eine vereinfachte Beschreibung der Kinetik des anaeroben Abbaus und der Ammoniumbildung zu Grunde gelegt.

Der abbaubare Teil der organischen Masse des Substrates wird in das Biogas umgewandelt. Dabei werden ein Teil der Biomasse in $CH_4$ und ein anderer Teil in $CO_2$ umgewandelt. Mit dem Biogas entweicht aus dem Faulschlamm relativ viel Wasserdampf (etwa 40 g/m$^3$ des Biogases bei einer Temperatur von ca. 30°C), der aber zum großen Teil in der Regel vor der energetischen Nutzung kondensiert wird und als Kondensat zurück in die Gärbehälter gepumpt wird. Wasserdampf spielt somit in der Massenbilanz nur eine untergeordnete Rolle. Stickstoff und Sauerstoff gelangen zusammen mit der Luft für die biologische Entschwefelung oder mit dem Substrat in die Gasspeicher, daher müssen diese in der Massenbilanz berücksichtigt werden. Die Gesamtmenge der restlichen Gase, wie Schwefelwasserstoff und Spuren von anderen Gasen (Formaldehyd, Ammoniak), ist sehr gering und kann bei der Bilanzierung vernachlässigt werden.

Die Masse des Biogases hängt stark von der Biogaszusammensetzung ab. Dies wird am Beispiel der Biogasbildung von Essigsäure vereinfacht dargestellt (Gleichung 4.3)

$$CH_3COOH \rightarrow CH_4 + CO_2 \qquad \text{[Gleichung 3.3]}$$

Die molare Masse von $CH_4$ beträgt 16 g/mol, die von $CO_2$ 44 g/mol. Nach dem Massenerhaltungsgesetz entstehen 16 g $CH_4$ und 44 g $CO_2$ aus 60 g $CH_3COOH$.

Die gleiche Bilanz lässt sich für jede andere Substanz durchführen. Wenn die Zusammensetzung des Biogases bekannt ist, kann die Massenbilanz exakt berechnet werden. Dies wird anhand eines einfachen Beispiels dargestellt. Das Gewicht von 100 Nm$^3$ Biogas mit 55 %Vol. Methan und 45%Vol. $CO_2$ kann mit Hilfe der folgenden Gleichung vereinfacht berechnet werden:

$$M_G = \frac{1}{22{,}413\,\frac{m^3}{kmol}} x \left( 45m^3 x44\,\frac{kg}{kmol} + 55m^3 x16\,\frac{kg}{kmol} \right) = 127{,}6kg \qquad \text{[Gleichung 3.4]}$$

Das bedeutet, dass 100 m$^3$ Biogas von ca. 127,6 kg des Subrates produziert werden. Mit Hilfe einer vereinfachten Massenbilanzierung (Input = Output) lässt sich also die abgebaute Biomasse sowie die Biogasmenge ausrechnen.

Die Berechnung der Massenbilanzen für einen kontinuierlich beschickten Gärbehälter ist jedoch nicht einfach, da sie eine Nachbildung der Strömungsverhältnisse im Behälter erfordert. Zur Vereinfachung wird angenommen, dass die Gärbehälter ideal durchmischt (CSTR) sind. Dies ist aufgrund der sehr langen hydraulischen Aufenthaltszeit der Substrate vertretbar. Allerdings könnten Totzonen durch Gasblasen oder mangelnde Durchmischung auftreten. wodurch das Nutzvolumen unvollständig ausgenutzt wird. Daher wird in der Bilanzierung ein aktives Volumen des Gärbehälters berücksichtigt. Somit gilt für den Gärbehälter, eine Substratart und eine Substratzugabe die Bilanzgleichung:

$$V \times \frac{dc_{oTM,abb}}{dt} = q_Z \times c_{oTM,abb,Z} - q_A \times c_{oTM,abb} - c_{oTM,abb} \times V \times f_a(t) \qquad \text{[Gleichung 3.5]}$$

$C_{oTM,abb}$ - Konzentration von abbaubaren oTM im Gärbehälter [kg/m$^3$]

$c_{oTM,abb,Z}$ - Konzentration von abbaubaren oTM im Zulauf des Gärbehälters [kg/m$^3$]

$q_Z$ - Zulaufstrom des Gärbehälters [m$^3$/h]

$q_A$ - Ablaufstrom des Gärbehälters [m$^3$/h]

$f_a(t)$ - Gesamtkinetik des anaeroben Abbaus [1/d]

$t$ - Zeit [h]

$V$ - aktives Volumen des Gärbehälters

Die Umwandlung der organischen Trockenmasse in Biogas wird mit Hilfe der Energiebilanzbasierten Modelle durch mehrere Gleichungen beschrieben. Im Gegensatz dazu wird im vereinfachten Modell angenommen, dass für jedes Substrat separat die Gesamtkinetik des Abbaus der organischen Trockenmasse $f_a(t)$ direkt von der empirisch ermittelten Gassummenkurve abgeleitet werden kann. Dazu wird folgende Gleichung benötigt.

$$f_a(t_i) = \frac{df_g(t_i)}{dt} \times \frac{1}{f_g(t_\infty) - f_g(t_i)}$$ [Gleichung 3.6]

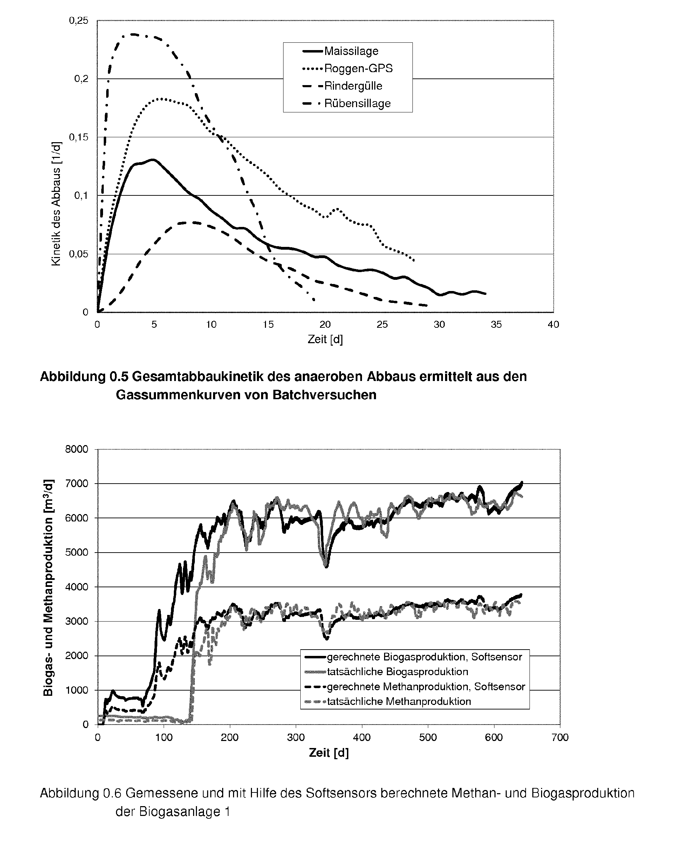

Das Ergebnis der Gleichung ist der Wert der Funktion $f_a(t)$ zu einem bestimmten Zeitpunkt $t_i$. $f_g(t_\infty)$ gibt die maximale Biogas-Summe für das Substrat an. Die Funktion $f_a(t)$ stellt eine zeitliche Veränderung der Biogasbildung dar und kann daher von der Biogasbildungsrate, die mit Hilfe von Batch-Biogasausbeuteversuchen bestimmt wird, abgeleitet werden. Im Rahmen der Arbeit wurden keine Biogasertragsmessungen der vorhandenen Substrate durchgeführt, daher wurden die Kurvender Literatur entnommen und digitalisiert. Die Beispiele der Gassummenkurven, die für die Ableitung genutzt wurden, können Abbildung 0.4 entnommen werden. Da die Beschreibung der Biogasbildungsfunktion sehr kompliziert ist, wurde sie für das Modell in einer diskreten Form übernommen. Die Ergebnisse der Ermittlung der Gesamtabbaukinetik für einzelne Substrate wurden in vorgestellt. Es ist ersichtlich, dass eine Annahme der Gesamtkinetik als Funktion erster Ordnung nicht zutreffend ist. Abbildung 0.5belegt, dass die Gesamtkinetik des Abbaus zeitabhängig ist.

Die Biogasproduktion wird mit Hilfe der Gleichung berechnet.

$$q_B = \gamma \times \frac{dc_{oTM,abb}}{dt} \times V \times f_a(t_i)$$ [Gleichung 3.7]

$\gamma$ - steht für die Biogasbildung pro Masse der organischen, abbaubaren Trockensubstanz, die mit Hilfe der Gleichung 4.8 berechnet werden kann:

$$\gamma \approx \frac{22{,}413 \frac{m^3}{kmol}}{v_{CH_4} \times 16 \frac{kg}{kmol} + v_{CO_2} \times 44 \frac{kg}{kmol}}$$ [Gleichung 3.8]

$v_{CH_4}$ - Volumenanteil des Methans im Biogas, das aus einem Substrat gebildet wird [%] $v_{CO_2}$ - Volumenanteil des Kohlenstoffdioxids im Biogas, das aus einem Substrat gebildet wird Für die Berechnung der Methanproduktion $q_M$ wurde angenommen, dass das gebildete Biogas aus einem Substrat einen konstanten Methangehalt hat. Somit wird die Methanproduktion mit Hilfe der Gleichung berechnet.

$$q_M = q_B \cdot v_{CH_4}$$ [Gleichung 3.9]

Die Berechnung der Konzentration von Ammonium-Stickstoff und Ammoniak erfordert eine genaue Bilanzierung aller Stickstofffraktionen sowie des Stickstoffkreislaufs. Selbst für sehr umfangreiche Modelle des anaeroben Abbaus wie z. B. ADM 1 [1 ist die Aufgabe, aufgrund der Komplexität der anaeroben Vorgänge, relativ schwierig. Im Rahmen der Arbeit wurde ein vereinfachtes Modell der Stickstoffbilanzierung implementiert und erprobt.

Die Freisetzung des Ammonium-Stickstoffs wurde mit Hilfe der Annahme berechnet, dass Stickstoff homogen in der abbaubaren organischen Trockenmasse verteilt ist. Die Konzentration des Ammoniumstickstoffs im Reaktor $c_{NH_4-N}{}^{\prime*}$ wurde mit Hilfe der nachfolgenden Gleichung gerechnet.

$$V \times \frac{dc_{NH_4-N}}{dt} = q_Z \times (c_{N,abb,Z} + c_{NH_4-N,Z}) - q_A \times (c_{N,abb} + c_{NH_4-N}) + V \times f_{NH_4-N}(t) \times c_{N,abb}$$

[Gleichung 3.10]

Konzentration an partikulären abbaubaren Stickstoff im zugeführten Substrat [kg/m$^3$]

$c_{NH_4-N,Z}$ - Konzentration an Ammoniumstickstoff im zugeführten Substrat [kg/m$^3$]

$c_{N,abb}$ - Konzentration an partikulären abbaubaren Stickstoff [kg/m$^3$]

$f_{NH_4-N}(t)$ · - Gesamtkinetik der Freisetzung von Ammonium [1/d]

In der hier vorgestellten Methode wurde angenommen, dass nahezu der gesamte Stickstoff in der organischen Trockensubstanz vorhanden ist. Da keine Untersuchungen über die Gesamtkinetik der Freisetzung von Ammonium vorlagen, wurde sie mit der der - Gesamtkinetik des anaeroben Abbaus $f_a(t)$ gleichgesetzt.

Ein Teil des im Substrat enthaltenen Stickstoffs findet sich in der Bakterienbiomasse wieder. Mit Hilfe des Online-Bilanzierung-Modells (Kapitel 3.7) kann sowohl die durch Umwandlung von organischen N in NH$_4$-N freigesetzte Stickstoffmenge, als auch die Menge, die von der Biomasse absorbiert wird, abgeschätzt werden. Der Stickstoffgehalt der Trockenmasse der anaeroben Bakterien kann zwischen 7% und 11 % betragen [3]. Für die vereinfachte Berechnung wurde angenommen, dass die Bakterienbiomasse etwa 9 % vom Stickstoff beinhaltet.

Im Rahmen dieser Arbeit wurde mit Hilfe der hier dargestellten Abhängigkeiten ein Modell aufgestellt, anhand dessen eine Simulationssoftware erstellt worden ist. Damit konnte die dynamische Massenbilanzierung der Gärbehälter der untersuchten Biogasanlagen durchgeführt werden. Auf Grundlage des Modells wurden Entwürfe der Softsensoren entwickelt. Somit können unter anderem Online-Werte sowie die Vorhersage folgender Parameter der einzelnen Gärbehälter berechnet und abgeschätzt werden:

- maximal zu erwartende Biogasproduktion

- tatsächliche Biogas- und Methanausbeute der einzelnen Substrate

- TM des Faulschlammes sowie nicht abgebaute oTM-Menge (Biogaspotenzial des Faulschlammes),

- Konzentrationen von Ammoniak und Ammoniumstickstoff (in Verbindung mit Offline-Messwerten)

Darüber hinaus konnte anhand des Vergleiches der Simulationsergebnisse und der tatsächlichen Biogasproduktion der Verlauf der Konzentrationen an flüchtigen organischen Säuren abgeschätzt werden.

3.6 Systemanalyse der untersuchten Biogasanlagen

3.7 Ergebnisse der Simulation mit Hilfe des entwickelten Modells.

3.7.1 Berechnung der Soll-Methan und Soll-Biogasproduktion anhand einer Online-Massenbilanzierung

Für die Steuerung und Regelung der Substratzufuhr ist die genaue Ermittlung der zu erwartenden Biogas- und Methanproduktion von Bedeutung. Mit Hilfe einer statischen Bilanzierung der Betriebsdaten lässt sich mit einer ausreichenden Genauigkeit für längere Zeiträume (z. B. 6 Monate) des Betriebes der Durchschnittswert berechnen. Diese Ergebnisse sind jedoch zur Steuerung und Regelung oder für ein Monitoring der Anlagen nicht zu verwenden. Laut der Ergebnisse der Analyse der Betriebsdaten ist es daher zwingend notwendig, die zu erwartenden Methan- und Biogasproduktionen über dynamische Simulation zu berechnen.

Das Modell, das im Kapitel 3.5 vorgestellt wurde, wurde unter anderem für die dynamische Berechnung der Soll-Biogasproduktion sowie der Soll-Methanproduktion für die Biogasanlagen 1 und 3 genutzt. Die Online-Berechnung wurde unter folgenden Annahmen durchgeführt:

● Die Biogasausbeute der Substrate ist konstant im gesamten Zeitraum

● Die anaeroben Reaktoren (Fermenter und Nachgärer) sind ideal durchmischt

● Ein bestimmter Anteil des Nutzvolumens des Reaktors ist durchgängig vom Biogas belegt

Die Ergebnisse der gemessenen und berechneten Biogasproduktion für die Biogasanlage 1 können Abbildung 0.6 entnommen werden.

In den ersten Monaten des Betriebes wurde die Biogasmenge rechnerisch höher als die gemessene Biogasproduktion eingeschätzt. Das kann folgende Ursachen haben:

● Die Bakterienpopulation hat sich noch nicht entwickelt, wodurch die Substrate nicht vollständig in Biogas umgewandelt wurden (Inbetriebnahmephase)

● In den Gärbehältern lagen erhöhte Konzentrationen an organischen Säuren vor, wodurch eine erhebliche Fracht aus Säuren mit den Gärresten in den Gärrestspeicher gelangte

Ab Tag 180 zeigen die Ergebnisse der berechneten Biogasproduktion eine Übereinstimmung mit der gemessenen Biogasproduktion. Es ist deutlich zu erkennen, dass die Dynamik der Prozesse gut abgebildet wurde.

Das Beispiel zeigt, dass eine relativ einfache Modellierung der anaeroben Biogasprozesse und eine einfache Technik zur Charakterisierung des Anlageninputs (offline TM Messung), eine hinreichende Genauigkeit liefern kann.

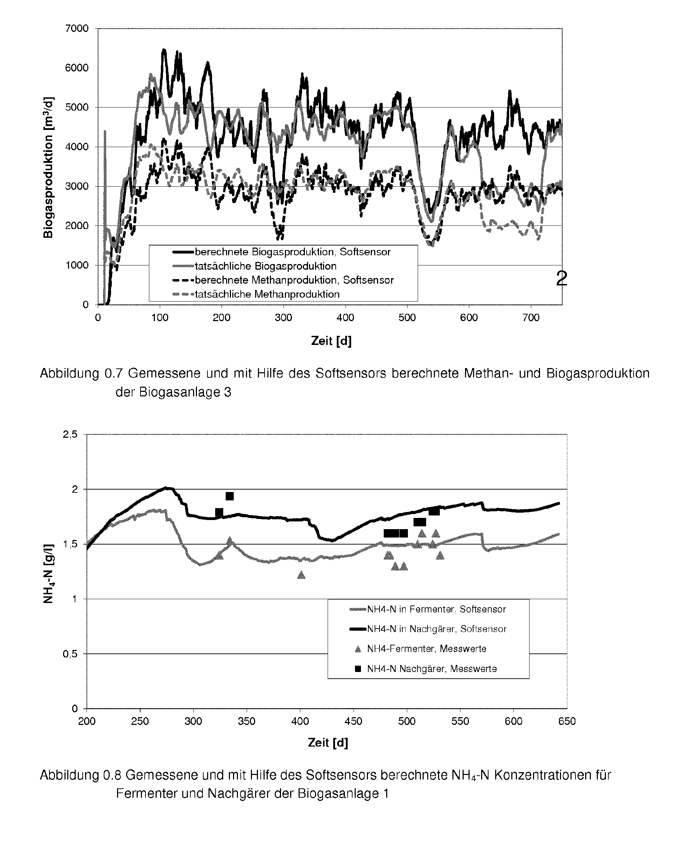

Abbildung 0.7 können Ergebnisse der Simulation für die Biogasanlage 3 entnommen werden. Mit Nummer 1 wurde der Zeitraum markiert in der die Substratzufuhr deutlich reduziert wurde. Es ist deutlich zu sehen, dass in dem Zeitraum sich die berechnete und tatsächliche Biogasproduktion überlappen. Zu der Zeit wurden auch die einzigen Substratuntersuchungen bezüglich des TM- und CSB-Gehaltes auf der Anlage durchgeführt. Mit Nummer 2 wurde der Zeitraum markiert in dem die berechnete Biogasproduktion deutlich höher als die tatsächliche Biogasproduktion war. In dem Zeitraum entwich aufgrund einer signifikanten Undichtigkeit in der Membran des Gasspeichers eine größere Menge an Biogas.

Es ist deutlich zu erkennen, dass die berechnete Methan- und Biogasproduktion nicht eng mit den tatsächlichen Werten übereinstimmen. Dafür gibt es folgende Gründe. Zum einen wurden die TM- und oTM-Messungen für wenige Substrate und nur für Stichproben (im Zeitraum der mit Nr. 1 markiert wurde) ausgeführt. Zum anderen wurden für die Berechnung Annahmen zur Abbaukinetik getroffen, da die Untersuchungen dazu nicht durchgeführt wurden.

3.7.2 Berechnung der $NH_4$-N und $NH_3$ Konzentrationen

Abbildung 0.8 stellt die Ergebnisse der Berechnung der $NH_4$-N Konzentration für den Fermenter und den Nachgärer der Biogasanlage 1 dar. Es ist zu erkennen, dass die $NH_4$-N-Konzentration im Nachgärer höher als im Fermenter ist. Dies liegt daran, dass ein Teil der Substrate direkt dem Fermenter zugeführt wird und im Anschluss erst den Nachgärer erreicht. Somit wurde während des Abbaus des organischen Materials Stickstoff als Ammoniumstickstoff freigesetzt. Abbildung 0.8 zeigt, dass die $NH_4$-N Konzentration im Betrieb sich relativ langsam verändern kann.

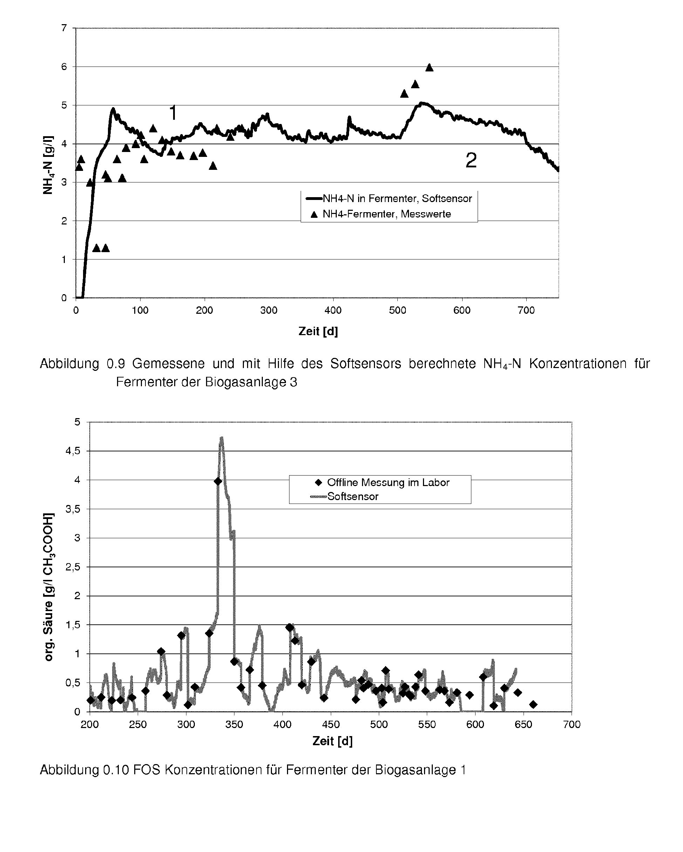

Die Ergebnisse der Simulation für die BGA 3 können Abbildung 0.9 entnommen werden. Leider lagen im Zeitraum der Untersuchung nur wenige Messwerte der $NH_4$-N Konzentration im Faulschlamm und der $N_{ges}$ Messwerte der Substrate vor. Die wenigen Untersuchungen wurden lediglich für den Faulschlamm des Fermenters und wenige Stichproben ausgewählter Substrate durchgeführt. Demzufolge wurden für die Simulation überwiegend Literaturwerte angenommen. Trotzdem es ist deutlich zu sehen, dass die Ergebnisse der Simulation mit dem Softwaresensor am Anfang des Betriebes in dem Bereich der realen Messwerte liegen (Abbildung 0.9, Bereich Nr. 1, links im Diagramm).

Im Laufe des weiteren Betriebes sind die tatsächlichen Ammonium-Stickstoff-Konzentrationen angestiegen (Abbildung 0.9, Bereich Nr. 2, rechts im Diagramm).. Die mit Hilfe des Softsensors ermittelten Konzentrationen sind niedriger als die tatsächlich auf der Anlage gemessenen. Dies kann eine fehlende Untersuchung des Stickstoffgehaltes der Substrate zur Folge haben. Trotzdem ist die steigende Tendenz sowohl bei den Messwerten als auch bei der rechnerisch ermittelten Kurve zu erkennen.

3.7.3 Berechnung der Konzentrationen der organischen Säuren

Abbildung 0.10 zeigt das Ergebnis der Berechnung der Konzentration der organischen Säuren für Fermenter der Biogasanlage 1. Der Berechnungsalgorithmus berücksichtigt die Labormesswerte. Die Messwerte zwischen den Labormessungen wurden durch Berechnung mit Hilfe der Massenbilanzierung interpoliert. Die Ergebnisse geben die Dynamik der Säurebildung sehr gut wieder.

Die Ergebnisse zeigen, dass die FOS-Konzentration im Gärbehälter mit Hilfe der Offline-Messungen sowie der Online-Massenbilanzierung kontrolliert werden kann. Mit Hilfe des entwickelten Modells kann vorausschauend berechnet werden wie sich die Konzentrationen an organischen Säuren aktuell entwickelt. Damit können jedoch keine langfristigen Vorhersagen erstellt werden.

3.7.4 Berechnung des TM-Gehaltes

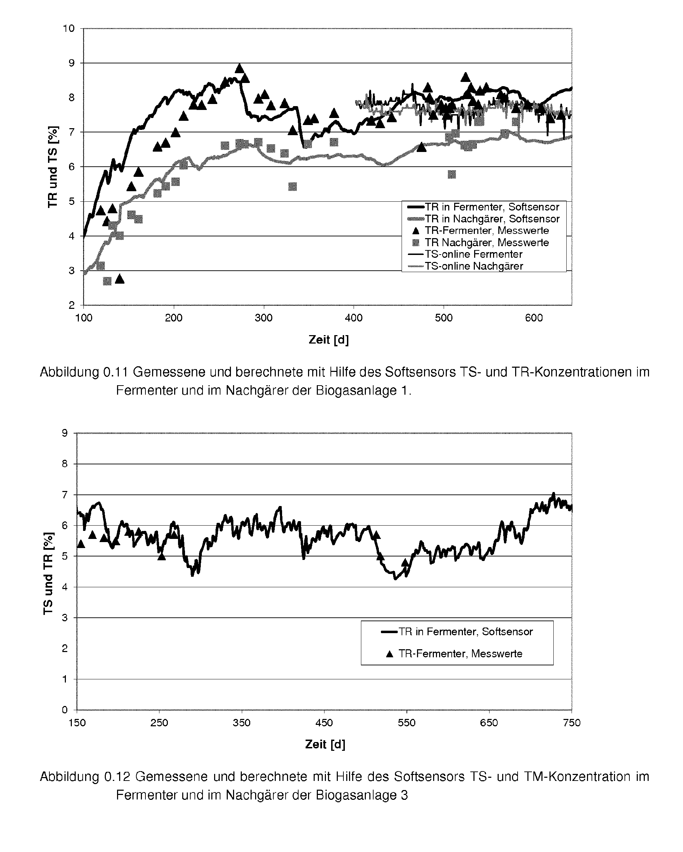

Die Ergebnisse der TM Berechnung mit Hilfe des Softsensors und TM-Messwerten für die Biogasanlage 1 sind in Abbildung 0.11 dargestellt. Es ist deutlich zu erkennen, dass sowohl die Tendenzen als auch die Absolutwerte sehr genau mit Hilfe der Simulation ermittelt werden konnten. Der Anstieg der TM-Konzentration bis zum Wert 8,5% verläuft dynamisch. Nachdem dieser Wert auf der Anlage erreicht wurde, sind Probleme beim Pumpen und Rühren im Fermenter aufgetreten, da die Viskosität des Faulschlammes sehr hoch war. Der Wert könnte als Grenzwert bei der Ermittlung der optimalen Substratzufuhrstrategie gewählt werden. Insgesamt war die Konzentration der Trockenmasse im Nachgärer der Anlage deutlich geringer. Dies hängt damit zusammen, dass im Nachgärer die Substrate weitgehend abgebaut wurden.

Die Abbildung 0.12 stellt Ergebnisse der TM Berechnung mit Hilfe des Softsensors und TM-Messwerten für den Fermenter der Biogasanlage 3 dar. Es ist zu erkennen, dass der TM-Gehalt viel geringer als im Fermenter der Biogasanlage 1 ist. Dies ist nachvollziehbar, da in der Anlage 3 Substrate verwendet wurden, die einen höheren Wassergehalt haben. Leider lagen nur wenige Labormesswerte für die Überprüfung der Genauigkeit der Simulation vor. Die gute Übereinstimmung der Berechnung mit den tatsächlichen Ergebnissen ist sehr gut.

4 Entwurf eines anlagenweiten Steuerungs- und Regelungsverfahrens für Biogasanlagen

4.1.1 Abgrenzung und Einleitung

Die anlagenweite Steuerung und Regelung der Biogasanlagen kann in drei miteinander gekoppelte Teilbereiche unterteilt werden:

● Messen, Steuern und Regeln der Produktionskapazitäten (z. B. der Gasverwertung, der Energieproduktion der Biogasanlage, der Speicherkapazität des Gärrestes)

● Messen, Steuern und Regeln der anaeroben Abbauvorgänge (z. B. die Regelung der Substratzufuhr hinsichtlich der Stabilität des Prozesses oder maximaler Ausnutzung der Substrate)

● Messen und Überwachen von Maschinen und Anlagenteilen (z B. des BHKW, der Rührwerkstechnik, der Pumptechnik)

Das MSR der Produktionskapazitäten beinhaltet alle Kontrollmechanismen zur Messung der vorhandenen Anlagenkapazitäten, wie Energieproduktion, Durchsatzleistung sowie Biogasproduktion, und definiert die übergeordneten Ziele für die Anlage zur Auslastung der vorhandenen Kapazitäten. Da die Entscheidungen die Wirtschaftlichkeit der Anlage direkt beeinflussen, werden sie oft direkt vom Betreiber getroffen. Im Rahmen der Arbeit wird ein voll automatisiertes MSR-Konzept zur Steuerung und Regelung der Biogasverwertungsanlagen (z. B. BHKW) sowie ein MSR-Konzept zur wirtschaftlich optimalen Substratzufuhr des Gärbehälters entwickelt und dargestellt.

Die MSR-Technik der anaeroben Abbauvorgänge beschäftigt sich überwiegend mit der Kontrolle sowie der Steuerung und Regelung der Gärbehälter. Darunter versteht man die entsprechende Prozessführung der Fermentation. Durch die Prozessführung werden Produktionsziele erreicht und ein stabiler Betrieb gewährleistet. Die Überwachung und Kontrolle der Stabilität der Fermentation spielt eine sehr wichtige Rolle, weil sie direkt mit der Wirtschaftlichkeit durch optimale Gasproduktion und Ausnutzung der Methanausbeuten steht. Im Rahmen der Arbeit wird ein MSR-Konzept für die Fermentation vorgeschlagen.

Die erreichbare Auslastung der Biogasanlage und die mittlere Energieproduktion hängen sehr stark vom zuverlässigen Betrieb der Anlagenteile und der Maschinen ab. Ein Ausfall des BHKW oder des Rührwerks kann schwerwiegende wirtschaftliche Folgen haben. Daher müssen die wichtigen Anlagenteile messtechnisch überwacht werden, damit deren Ausfallrisiko minimiert wird. Dies wird jedoch in der Praxis oftmals nicht umgesetzt. Zudem finden sich in der Fachliteratur nur wenige Informationen dazu.

Die verfahrenstechnische Ausführung der Biogasanlage wie z. B. die Anzahl der Fermentationsstufen, das aktive Nutzvolumen des Gärraums und der Temperaturbereich der Fermentation (mesophil oder thermophil) haben einen sehr großen Einfluss auf die Möglichkeiten der Steuerung und Regelung der Biogasanlage.

Das Inputmanagement sollte auf Biogasanlagen eine zentrale Rolle spielen, da Informationen wie die Substratverfügbarkeit, die Substratkosten oder die Ergebnisse der Substratrecherche (Untersuchungsergebnisse der Biogasausbeute von neuen potenziellen Substraten) sowohl auf die Zufuhrstrategie des Betriebes als auch auf dessen Steuerung und Regelung einen sehr großen Einfluss haben.

Das Informationsmanagement spielt auf Biogasanlagen eine immer wichtigere Rolle. Die Informationen müssen gesammelt, ausgewertet und an die Betreiber weitergegeben werden. Für eine effiziente Nutzung, der auf der Anlage gesammelten Informationen, muss ein Datenmanagement geschaffen werden, mit dem Informationen geprüft, gesammelt und verwaltet werden können. Ähnlich wie bei Kläranlagen werden automatisierte Betriebstagebücher, Substratannahme- und Gärrestabgabestationen entwickelt, die mit dem Prozessleitsystem verbunden sind. Die Steuerung und Regelung von Anlagen ist noch weit davon entfernt voll automatisiert zu erfolgen. Deswegen werden in naher Zukunft verstärkt halb automatische Systeme erforderlich sein, die durch Visualisierung der Daten oder über andere Kommunikationswege (z. B. über das Telefon, Alarm- und Lichthupe) dem Betreiber Informationen übermitteln. Die Art und Weise der Informationsübermittlung spielt für Betreiber von Biogasanlagen eine zentrale Rolle (sie muss oft an projektspezifische Gegebenheiten angepasst werden), da diese Elemente zur Steigerung des Prozessverständnisses führen und die Entscheidung über Betriebsablaufe vereinfachen. Dieser Themenbereich wurde jedoch im Rahmen dieser Arbeit nicht näher betrachtet.

Die Untersuchungen zum Einsatz der Messtechnik sowie die statische und die dynamische Bilanzierung der Anlagen bilden die Grundlage für die Entwicklung eines anlagenweiten Konzeptes. Anhand der gesammelten Informationen sowie den entwickelten Werkzeugen wurde ein Entwurf des anlagenweiten Steuerungs- und Regelungssystems erstellt, der im Folgenden vorgestellt wird.

4.1.2 Überwachung der Prozessstabilität

Um die Biogasanlagen überwachen, steuern und regeln zu können, muss die messtechnische Ausrüstung viele Aufgaben gleichzeitig erfüllen:

● Erhebung von Informationen über Zustand und Kapazität des Prozesses des anaeroben Abbaus und Biogasproduktion

● Erhebung von Informationen über Zustand von Maschinen und Aggregaten der Biogasanlage (z. B. Pumpen, BHKW, Rührwerke, Feststoffeintrag)

● Erhebung von Informationen über Nutzung von vorhandenen Kapazitäten (z. B. Füllstände von Gas und Flüssigkeit in Gärbehältern, Füllstand im Gärrestbehälter)

● Erhebung von Informationen über sicherheitstechnische Aspekte des Betriebes (z. B. Konzentrationen von gefährlichen Gasen wie $CH_4$ und $H_2S$, Schutz vor Überfüllung des Behälters)

Der erforderliche Umfang der messtechnischen Ausrüstung bzw. des Messsystems und Messprogramms bei einer konkreten Anlage hängt von den spezifischen Randbedingungen und der Aufgabestellung ab. Bei der Wahl der Messtechnik spielen verschiedene Faktoren eine wichtige Rolle, da messtechnische Einrichtungen unterschiedliche Eigenschaften aufweisen (z. B.: Häufigkeit der Messung, Genauigkeit, Repräsentativität der Probe, Aufwand für die Kalibrierung, Art der Probenaufbereitung, Kosten der Messeinrichtung und der Messung).

Der Zustand und die Produktivität der Fermenterbiologie hängen von den eingesetzten Substraten, der verfahrenstechnischen Auslegung der Anlage und der Stabilität der Prozesse ab. Die Stabilität der anaeroben Prozesse ist abhängig von:

● Größe und Art (z. B. kontinuierliche oder diskontinuierliche, regelmäßige oder unregelmäßige) der Belastung des Fermenters

● Eigenschaften der eingesetzten Substrate:

o Zusammensetzung (Gehalt an Proteinen, Fette, Kohlenhydrate) sowie Dynamik des Substratabbaus

o Gehalt der potenziellen Hemm- und/oder Giftstoffe

o Gehalt der Spurenelemente

o Eignung zur Bildung der Hemm- und Giftstoffe (z. B. Schwefelwasserstoff, Ammoniak, organischen Säuren) im Laufe des anaeroben Abbaus

o Eignung zur Bildung von Ausgangssubstraten der Verbindungen, die für Erhöhung der Pufferkapazität sorgen

In dieser Arbeit wurde besonderes Augenmerk auf die Erhebung und die Aufbereitung der plausiblen Messdaten für das Steuern und Regeln der Biogasanlage gelegt. Ein zuverlässiges Messsystem ist die Grundlage für das Monitoring sowie für die Steuerung und Regelung der Biogasanlage.

Die Schlussfolgerung der umfangreichen Analyse der Messetechnik für Biogasanlagen ist, dass derzeit auf dem Markt keine zuverlässige Messtechnik erhältlich ist, welche die Schlüsselparameter (Konzentration der organischen Säuren, Konzentration des Ammoniumstickstoffes und Säurekapazität) online bestimmen kann, sodass die für Steuerungs- und Regelungsstrategien erforderlichen Werte anderweitig gewonnen werden müssen. Über eine Kombination von Offline- und Online-Messtechnik können relativ viele zuverlässige Informationen gesammelt werden, die für ein einfaches Monitoring ausreichen. Für die Steuerung und Regelung muss jedoch die Qualität und die Zuverlässigkeit der Daten verbessert werden.

Im Rahmen der Arbeit wurden Softsensoren für Biogasanlagen entwickelt und in ein Simulationen erprobt. Die Sensoren basieren im Wesentlichen auf einem Online-Massenbilanzierungsmodell, welches in Rahmen der Arbeit entwickelt wurde.

Die organischen Säuren (FOS - flüchtige organische Säuren), die TM-Konzentration im Faulschlamm, sowie die $NH_4$-N Konzentration können mit Hilfe der Softsensoren, die auf dem Modell basieren, ermittelt werden. Dadurch kann der Verlauf der Messwerte zwischen den Laboranalysemesswerten ermittelt und zum Zweck der Steuerung und Regelung genutzt werden. Die Häufigkeit der Offline-Messungen wird anhand der Trendentwicklung sowie anderer Online-Kontrollparameter (pH, Redox Leitfähigkeit) festgelegt.

Die Prozesskontrolle beruht auf der Auswertung des Unterschiedes der gemessenen Methan/Biogas-Produktion und der berechneten Methan/Biogas-Produktion. Weichen die beiden Werte zu stark voneinander ab, kann dies zwei Ursachen haben:

● Abweichung durch Entweichung des Biogases durch Undichtigkeiten im System

● Anaeroben Abbauprozesse verlaufen instabil.

Ein weiterer Einsatz des entwickelten Modells ist für die Analyse der Betriebsdaten der Anlage möglich. Durch Einsatz in der Simulation einer einfachen iterativen Näherung oder eines Optimierungsalgorithmuses (z. B. genetischer Algorithmus oder Simplexverfahren) können folgende Parameter ermittelt werden:

● maximale Biogas- und Methanausbeute für jedes eingesetzte Substrat

● auf der Anlage erreichte Biogas- und Methanausbeute für jedes eingesetzte Substrat

● Restgaspotenzial der Gärreste

Auf diese Art und Weise können sehr wichtige Informationen für den Betrieb der Anlage gewonnen werden.

Die Berechnung des TAC-Wertes war anhand der Bilanzierung nicht möglich, daher wird der Wert mit Hilfe einer Offline-Messung ermittelt. Im Rahmen der Untersuchung wurde jedoch beobachtet, dass der Parameter mit der Leitfähigkeit des Faulschlammes gut korreliert. Zudem ist die Bestimmung der Pufferkapazität mit Hilfe von BiogasPro eine robuste und einfache Methode. Die Automatisierung der Messung könnte einen großen Vorteil der MSR-Technik für Biogasanlagen bringen.

Die Online-Messungen von Kontrollgrößen wie pH, das Redox-Potenzial und die Leitfähigkeit sind zwar sehr robust und geeignet für den Einsatz auf Biogasanlagen, liefern jedoch Informationen, die für die Steuerung und die Regelung der Biogasanlagen nur begrenzt zu verwerten sind. Der pH-Wert ist abhängig von vielen unterschiedlichen Parametern wie beispielsweise Alkalinität und Säurekonzentration. Eine signifikante Verminderung des Parameters bedeutet, dass die biologische Stufe sich bereits in einem extrem instabilen Zustand befindet. In der Regel ist es dann zu spät, um reagieren zu können und um erhebliche Leistungseinschränkung zu verhindern. Die Parameter können jedoch in der sogenannten Notbremse- und Alarmfunktion eine entscheidende Rolle spielen. Die sensibleren Parameter wie Säurekonzentration (insbesondere Propionsäurekonzentration) und Wasserstoffkonzentration in der gelösten Phase informieren wesentlich schneller über Probleme im Prozessablauf.

4.1.3 Regelung der Substratzufuhr der Biogasanlage

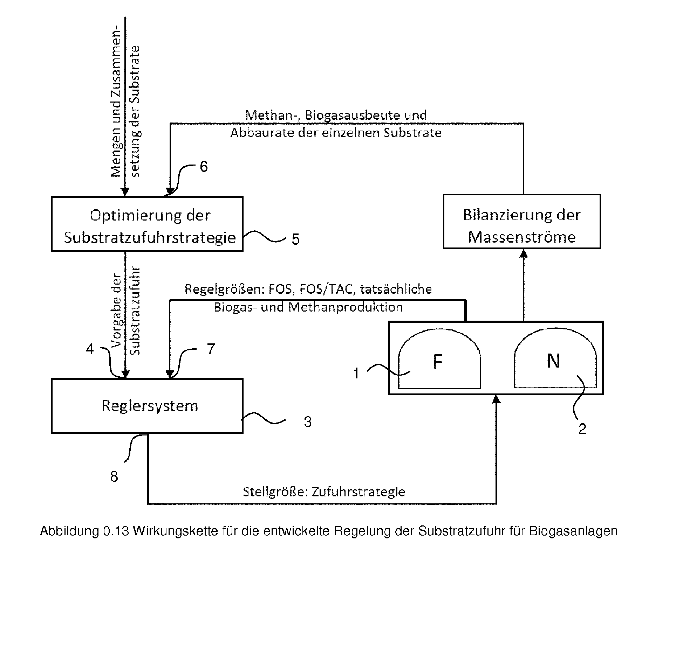

Abbildung 0.13 stellt Wirkungskette der entwickelten Regelung der Substratzufuhr der Biogasanlagen dar. Dabei sind drei wichtige Prozessschritte aufgezeichnet:

1. Dynamische Bilanzierung der Massenströme. Mit Hilfe des entwickelten Modells werden die maximale Methan- und Biogasausbeute sowie die maximale Abbaurate der einzelnen Substrate berechnet. Die Berechnung wird anhand der Simulation der letzten Wochen des Betriebes erstellt.

2. Optimierung der Substratzufuhrstrategie. Anhand der zur Verfügung stehenden Substratmischung der einzelnen Substraten, deren Methanausbeuten und der Abbaurate sowie der Zusammensetzung (TM, oTM, TS, oTS, Gesamtstickstoff und $NH_4$-N), werden verschiedene zukünftige Substratzufuhrszenarien mit Hilfe des entwickelten Modells simuliert. Dabei wird das Szenario gewählt bei der die angestrebte Methanausbeute (Energieausbeute) bei maximalen Abbaurate der organischen Trockenmasse der Substrate erzielt wird. Gleichzeitig werden folgende Bedingungen erfüllt:

a. $NH_4$-N-Konzentrationen im Fermenter und Nachgärer sind kleiner als der vorgegebene Sollwert

b. TM-Konzentration im Fermenter und Nachgärer sind kleiner als der vorgegebene Sollwert.

Gemäß einer vorteilhaften Ausführungsform der Erfindung wird daher ein Verfahren zur Regelung einer Biogasanlage vorgeschlagen, bei dem die Zufuhr von Substraten zu einem Fermenter und einem Nachgärer der Biogasanlage durch ein Regelungssystem 3 im laufenden Betrieb der Biogasanlage geregelt wird, dem als Eingangsgröße eine durch Computersimulation bestimmte Substratzufuhr-Strategie zugeführt wird, die nach Maßgabe der verfügbaren Substrate bestimmt ist.

Auf dem Computer kann z. B. die dynamische Bilanzierung der Massenströme gemäß dem in Kapitel 4.6 beschriebenen Modell ausgeführt werden. Die Bestimmung der Substratzufuhr-Strategie kann z.B. anhand der Simulation der letzten Wochen des Betriebes der Biogasanlage durchgeführt werden.

Gemäß einer vorteilhaften Ausführungsform der Erfindung werden anhand der Vorratsmengen und Arten der verfügbaren Substrate sowie deren charakteristischer Methanausbeutewerte und ihrer Abbaurate verschiedene zukünftige Substratzufuhrstrategien zunächst mittels eines Computers simuliert, die Substratzufuhrstrategie mit einer angestrebten Methanausbeute bzw. Energieausbeute bei einer maximalen Abbaurate der organischen Trockenmassen ausgewählt und sodann die Regelung der Biogasanlage gemäß der ausgewählten Substratzufuhrstrategie durchgeführt.

Gemäß einer vorteilhaften Ausführungsform der Erfindung wird aus den simulierten Substratzufuhrstrategien diejenige ausgewählt, bei der die $NH_4$-N Konzentrationen im Fermenter und im Nachgärer der Biogasanlage jeweils kleiner als ein vorgegebener Sollwert sind.

Gemäß einer vorteilhaften Ausführungsform der Erfindung wird diejenige Substratzufuhrstrategie ausgewählt, bei der die Trockenmasse Konzentration im Fermenter und im Nachgärer jeweils kleiner ist als ein vorgegebener Sollwert. Gemäß einer vorteilhaften Ausführungsform der Erfindung wird ein Verfahren zur Regelung einer Biogasanlage vorgeschlagen, bei dem im laufenden Betrieb der Biogasanlage eine oder mehrere der Größen: Methanausbeute, Biogasausbeute und Abbaurate der der Biogasanlage zugeführten einzelnen Substrate, unter Heranziehung im laufenden Betrieb gemessener Zulauf- und Ablaufströme des Fermenters und/oder des Nachgärers der Biogasanlage nach Art einer Massenbilanz bestimmt werden und als Eingangsgröße für die Computersimulation zur Bestimmung der Substratzufuhrstrategie verwendet werden.

Durch die zuvor erläuterten Ausführungsformen wird der Regelungssystem 3 mit einer optimierten Substratzufuhrstrategie als Eingangsgröße 4 versorgt, wie in Abbildung 0.13 dargestellt. Die Erfindung betrifft ferner die Optimierung der Regelung der weiteren Parameter des Regelkreises, insbesondere der Reglereingangsgröße 7 und der Stellgröße 8 des Regelungssystems 3. Diese Optimierungen können kombiniert mit oder auch unabhängig von den zuvor erläuterten Ausführungsformen realisiert werden.

3. Regelungssystem. Das System erstellt eine optimale Substratzufuhrstrategie separat für Fermenter und Nachgärer.

a. Stellgrößen: Menge der Substratmischung pro Fermenter und Nachgärer

b. Führungsgrößen: maximale Konzentration von flüchtigen organischen Säuren, maximale FOS/TAC-Verhältnis, maximale Tendenz der Bildung von flüchtigen organischen Säuren, maximale Tendenz der Bildung des FOS/TAC-Verhältnisses, vorgegebene Methanproduktion

c. Regelgrößen: Konzentration von flüchtigen organischen Säuren, FOS/TAC-Verhältnis, Tendenz der Bildung der flüchtigen organischen Säuren, Tendenz der Bildung des FOS/TAC- Verhältnisses, aktuelle Methanproduktion

Gemäß einer vorteilhaften Ausführungsform der Erfindung wird ein Verfahren zur Regelung einer Biogasanlage vorgeschlagen, bei dem wenigstens eine der Größen FOS, FOS/TAC, tatsächliche Biogas- und Methanproduktion anhand im laufenden Betrieb der Biogasanlage bestimmter physikalischer Größen gemessen oder berechnet werden und dem Regelungssystem als Eingangsgröße zugeführt werden. Diese Eingangsgrößen stellen damit Führungsgrößen für das Regelungssystem dar.

Gemäß einer vorteilhaften Ausführungsform der Erfindung stellt das Regelungssystem als Stellgröße die Menge der Substratmischung jeweils separat für den Fermenter und für den Nachgärer ein.

Somit erhält das Regelungssystem der Anlage wichtige Informationen, die notwendig sind um die Substratzufuhr der Biogasanlage sowie die Massenströme optimal zu regeln. Der Regelkreis wird geschlossen, indem die Informationen direkt (über Sonden und Labormesswerte) und/oder indirekt (über Softsensoren) von den anaeroben Reaktoren und deren Ablauf- und Zulaufströmen erhoben werden. Bei der gesamten Regelung werden folgende Vorteile erreicht:

● Stabiler Betrieb: Langfristige Sicherung des TM-Gehaltes (Pumpfähigkeit des Substrates) und der $NH_4$-N Konzentration im Fermenter und Nachgärer unter vorgegebenen Grenzwerten; kurzfristige Sicherung des Gehaltes an flüchtigen organischen Säuren, FOS/TAC unter vorgegebenen Grenzwerten

● Gute Wirtschaftlichkeit im Betrieb: Optimale Ausnutzung der Methan- und Biogasbildungspotenziale der eingesetzten Substrate sowie stabile Methanproduktion auf einem hohen angestrebten Level

● Feedback vom System: Über die Bilanzierung der Massenströme und des Stickstoffes werden die tatsächlichen Methan- und Biogasausbeuten sowie maximale und tatsächliche Abbauraten der eingesetzten Substrate ermittelt. Die Informationen werden primär zur Erstellung der Zufuhrstrategie genutzt, können aber auch für die Planung des Betriebes genutzt werden

In mehreren Forschungsvorhaben wurden die Parameter und deren Grenzwerte, deren Einhaltung für die Stabilität des Gärprozesses und damit für die Maximierung der Biogasproduktion notwendig ist, ermittelt und festgelegt. Diese Literaturwerte können als Grundlage für den Betrieb des Steuerungssystems genutzt werden.

Es ist bekannt, dass die anaerobe Biozönose sich an unterschiedliche Bedingungen anpassen kann. Aufgrund der Adaption der Biozönose müssen die tolerierbaren Grenzen der Stabilitätsparameter ständig untersucht und angepasst werden. Der Betreiber hat beim Einsatz der Regelung die Möglichkeit die Sollwerte ständig anzupassen. Da für die Regelung ständig die tatsächliche, aktuelle Abbaurate der Substrate sowie die tatsächlichen, aktuellen Biogas- und Methanausbeuten der einzelnen Substrate ermittelt werden, passt sich die Regelung an die Betriebsbedingungen an. Somit besitzt das entwickelte System eine Lernfunktion.

Die Beibehaltung der Fließfähigkeit des Faulschlammes ist ein wichtiges Thema in Bezug auf den störungsfreien Betrieb der Anlagen. Der Faulschlamm sowie die frischen Substrate werden mit Hilfe der Pumptechnik von Behälter zu Behälter umgepumpt. Je nach Pumptechnik und den hydraulischen Eigenschaften der Rohrleitungen (hydraulischer Widerstand) kann das Fördern des Substrates bei einer hohen Viskosität des Mediums erschwert werden. Je höher der TM-Gehalt des Faulschlammes desto höher ist auch dessen Viskosität. Die TM-Grenze für eine bestimmte Biogasanlage kann für die Regelung individuell eingestellt werden. Für die im Rahmen dieser Arbeit untersuchten Biogasanlagen lag die Grenze bei ca. 9% TM. Die Regelung kann eine optimale Kombination der Substrate bestimmen (Funktion der Prädikation des TM Gehaltes sowie des $NH_4$-N Gehaltes), sodass die Fließfähigkeitsgrenze in den Gärbehältern nicht überschritten wird.

Eine optimale Substratzufuhrstrategie muss den wirtschaftlichen Betrieb der Anlage garantieren. Daher muss die Regelung sowohl die Substratpreise als auch deren tatsächliche Biogasausbeute berücksichtigen.

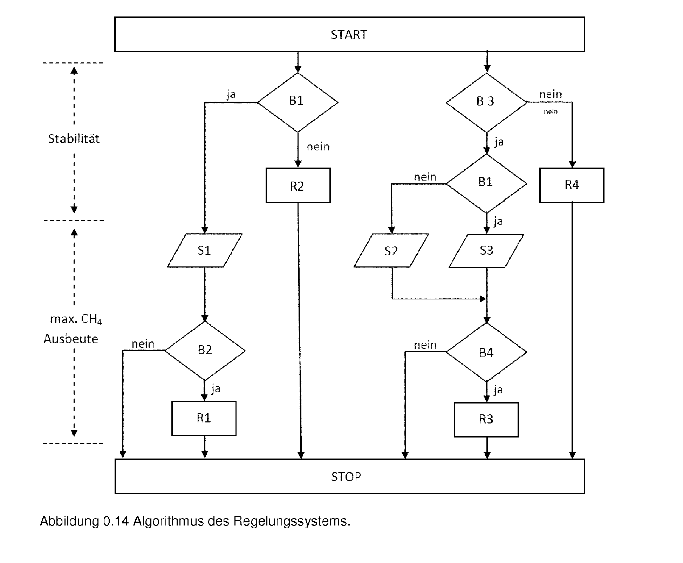

Der Algorithmus des Regelungssystems wird in Abbildung 0.14 dargestellt. Das Regelungssystem wurde so entworfen, dass in erster Linie die Stabilität der Fermentation in den jeweiligen Gärbehälter gesichert wird. Erst wenn, dieses Kriterium erfüllt ist, kann die Substratmenge so eingestellt werden, dass eine maximale und optimale Methanproduktion bei einer maximalen Abbaurate erreicht wird.

Im Folgenden werden die einzelne Bedingungen "B", Regler "R" und Schritte "S" erklärt:

B1: Die Bedingung ist erst dann erfüllt, wenn die Konzentration der flüchtigen organischen Säuren, das FOS/TAC-Verhältnis, die Tendenz der Bildung der organischen Säuren und die Tendenz der Bildung der FOS/TAC Verhältnisses im Fermenter kleiner als die jeweiligen Grenzwerte sind.

B2: Die Bedingung ist erst dann erfüllt, wenn die aktuelle Methanproduktion höher als der angestrebte Wert für die Biogasanlage liegt und kein frisches Substrat in den Nachgärer gegeben wird.

B3: Die Bedingung ist erst dann erfüllt, wenn die Konzentration der flüchtigen organischen Säuren, das FOS/TAC-Verhältnis, die Tendenz der Bildung der organischen Säuren und die Tendenz der Bildung der FOS/TAC-Verhältnisses im Nachgärer kleiner als die jeweiligen Grenzwerte sind.

B4: Die Bedingung ist erst dann erfüllt, wenn die aktuelle Methanproduktion für die Gesamtanlage geringer oder höher als der angestrebte Wert ist und dem Nachgärer frisches Substrat zugeführt wird.

R1: Wenn die aktuelle Methanproduktion höher als der angestrebte Wert ist und kein frisches Substrat dem Nachgärer zugegeben wird, wird die gesamte Substratzufuhrmenge des Fermenters über einen Regler (z. B. PID) korrigiert (reduziert).

R2: Wenn zumindest einer von den genannten Parametern im Fermenter (siehe B1) höher als dessen Grenzwert liegt, wird die gesamte Substratzufuhrmenge des Fermenters über einen Regler (z. B. PID) korrigiert (reduziert).

R3: Wenn die aktuelle Methanproduktion geringer oder höher als der angestrebte Wert ist und dem Nachgärer frisches Substrat zugeführt wird, wird die gesamte Substratzufuhrmenge des Nachgärers über einen Regler (z. B. PID) korrigiert (entsprechend erhöht oder reduziert).

R4: Wenn zumindest einer von den genannten Parametern im Nachgärer (siehe B3) höher als dessen Grenzwert liegt, wird die gesamte Substratzufuhrmenge des Nachgärers über einen Regler (z. B. PID) korrigiert (reduziert).

S1: Der Schritt wird erst realisiert, wenn die Stabilitätskriterien der Vergärung im Fermenter (B1) erfüllt sind. In diesem Schritt werden die Vorgaben hinsichtlich der Substratzufuhr, die über "Optimierung der Substratzufuhrstrategie" berechnet wurden, übernommen.

S2: Wenn die Fermentation im Nachgärer stabil verläuft (B3 ist erfüllt) und zumindest einer von den genannten Parametern in Fermenter höher als dessen Grenzwert liegt (B1 ist erfüllt) wird die gesamte Substratzufuhrmenge des Nachgärers entsprechend erhöht. Die Erhöhung wird mit Hilfe des entwickelten Modells berechnet, sodass die angestrebte Methanproduktion der Gesamtanlage beibehalten wird.

S3: Der Schritt wird erst realisiert, wenn die Stabilitätskriterien im Fermenter und im Nachgärer gemäß B1 und B3 erfüllt sind. In diesem Schritt werden die Vorgaben hinsichtlich der Substratzufuhr, die über "Optimierung der Substratzufuhrstrategie" berechnet wurden, übernommen.

Die Messdaten (Regelgrößen) für die Regelung der Substratzufuhr können auf folgende Art und Weise mit Hilfe der verfügbaren Messtechnik erhoben werden:

● Konzentration flüchtiger organischer Säuren (FOS): Ermittlung im Labor über Gaschromatografie alle 2 bis 4 Wochen. Im Zeitraum zwischen den Labormessungen werden die Messwerte mithilfe des entwickelten Softsensors abgeschätzt. Dabei soll auch das Redox-Potenzial des Faulschlammes z.B. mithilfe einer Online-Prozesssonde überwacht werden. Sollte das Redox-Potenzial stark ansteigen, müssen Labormessungen sofort ausgeführt werden.

● FOS/TAC Verhältnis: Die Ermittlung findet auf der Anlage mit Hilfe eines halb automatischen Titrators statt. Die Häufigkeit richtet sich nach dem Wert der letzten Messung (z. B jeden zweiten Tag oder einmal pro Woche).

● TM-Gehalt des stapelbaren Substrates: Jeden zweiten Tag sollen zumindest 2 - 4 Proben des Ausgangssubstrates gemessen werden (wenn der Anteil des Substrates gering ist, kann die Häufigkeit der Probenahme verringert werden). Dies kann mit Hilfe eines halbautomatischen Feuchtebestimmers erfolgen.

● oTM-Gehalt des stapelbaren Substrates: Jede Woche sollen zumindest 2 - 4 Proben des Ausgangssubstrates gemessen werden (wenn der Anteil des Substrates gering ist, kann die Häufigkeit der Probenahme verringert werden). Dies kann im Labor erfolgen.

● TS-Gehalt des pumpfähigen Substrates: Der Parameter soll mithilfe einer TS-Onlinesonde gemessen werden.

● oTS-Gehalt des pumpfähigen Substrates: Jede Woche sollen zumindest 2 - 4 Proben des Ausgangssubstrates gemessen werden (wenn der Anteil des Substrates gering ist, kann die Häufigkeit der Probenahme verringert werden). Dies kann im Labor erfolgen.

● $NH_4$-N-Gehalt: Bestimmung im Labor und mit Hilfe des entwickelten Softsensors.

● N-Gehalt der Substrate: Bestimmung im Labor.

● TM-Gehalt des Faulschlammes: Berechnung mit Hilfe des entwickelten Softsensors. Zur Kontrolle alle 2- 4 Wochen Bestimmung einer repräsentativen Probe mit Hilfe eines halbautomatischen Feuchtebestimmers.

● Tatsächliche Biogas- und Methanproduktion: Bestimmung anhand einer Normkubikmetermessung des Gasdurchflusses und anhand der im Rahmen der Arbeit entwickelten Normkubikmetermessung des Volumens des Gasspeichers.

Die nachfolgend erläuterten Ausführungsformen der Erfindung können kombiniert mit oder auch unabhängig von den zuvor erläuterten Ausführungsformen realisiert werden. Gemäß einer vorteilhaften Ausführungsform der Erfindung wird ein Verfahren zur Regelung einer Biogasanlage vorgeschlagen, mit dem die tatsächliche Biogas- und/oder Methanproduktion der Biogasanlage mit erhöhter Genauigkeit ermittelt werden kann. Vorgeschlagen wird ein Verfahren, bei dem die tatsächliche Biogas- und/oder Methanproduktion der Biogasanlage wenigstens unter Verwendung der Größen Gasdurchflussmenge an einer Gasabgabeleitung der Biogasanlage und Volumen bzw. Volumenänderung in einem Gasspeicher bestimmt wird. Das Volumen bzw. die Volumenänderung des Gasspeichers kann z. B. mit einer Seilsonde bestimmt werden. Für die Bestimmung der Gasdurchflussmenge im Normkubikmeter können handelsübliche Durchflussmengenmesser verwendet werden, wie z. B. MID-Durchflussmesser in der Kombination mit der Temperatur- und mit der Druckmessung verwendet werden

Gemäß einer vorteilhaften Ausführungsform der Erfindung wird das Volumen bzw. die Volumenänderung im Gasspeicher anhand eines Füllstandssensors, eines Temperatursensors und eines Drucksensors bestimmt. Hierdurch kann die Genauigkeit der Bestimmung der produzierten Gasmenge weiter erhöht werden.

Falls Online-Messtechnik für die Messung der oben genannten Parameter in Zukunft verfügbar ist, kann die oben vorgeschlagene Messmethode durch die Onlinemessung ersetzt werden. Ein Beispiel wäre die Weiterentwicklung der NIRS-Messtechnik wodurch z. B. die Konzentration organischen Säuren, der Säurepuffer des Faulschlammes, die TS, oTS, TM-und oTM-Konzentration der Substrate oder der Anteil an Ammoniumstickstoff im Faulschlamm bestimmt werden könnten.

4.1.4 Regelung der Rührtechnik

Die Rührtechnik im Gärbehälter muss folgende Aufgaben erfüllen:

● Die Verteilung vom frischen bzw. unvergorenen Substrat im gesamten Volumen des Gärbehälters

● Unterstützen der biologischen Prozesse durch Vermeidung der Aufkonzentrierung der Stoffwechselprodukte (z. B. lokale Erhöhung der Säure- oder Ammoniakkonzentration) oder durch das Heranführen der Nährstoffe an die Bakterien.

● Die Unterstützung der Wärmeübertragung

● Die Vermeidung von Schwimm- und Sinkschichten sowie Ablagerungen von Sedimenten

● Die Unterstützung der Entgasung des Faulschlammes

Ein weiterer Aspekt der Erfindung betrifft daher die Regelung der Rührtechnik. Die nachfolgend erläuterten Ausführungsformen der Erfindung können kombiniert mit oder auch unabhängig von den zuvor erläuterten Ausführungsformen realisiert werden.

Gemäß einer vorteilhaften Ausführungsform der Erfindung wird ein Verfahren zum Betrieb einer Biogasanlage vorgeschlagen, bei dem eine Messung der elektrischen Leitfähigkeit des Faulschlamms im Fermenter und/oder im Nachgärer durchgeführt wird und anhand eines oder mehrerer gemessener Leitfähigkeitswerte das Einschalten des Rührwerks im Fermenter und/oder im Nachgärer gesteuert wird.

Gemäß einer vorteilhaften Ausführungsform der Erfindung werden mehrere Leitfähigkeitswerte bestimmt. Hieraus wird die Standardabweichung der Leitfähigkeitswerte bestimmt. Das Rührwerk wird eingeschaltet, wenn die Standardabweichung größer als ein bestimmter Grenzwert ist.

Gemäß der im Rahmen der Arbeit durchgeführten Untersuchungen der Durchmischung ist die Leitfähigkeit ein gut geeigneter Parameter für die Beurteilung der Verteilung von gelösten Produkten und Zwischenprodukten des anaeroben Abbaus. Ein weiterer geeigneter Parameter für die Regelung der Rührtechnik ist die Abweichung der Messwerte der Leitfähigkeit vom Mittelwert (z. B. als Standardabweichung). Der vorgeschlagene Regler soll über die Laufzeit der Rührwerke dazu führen, dass die Standardabweichung der Leitfähigkeit unter einem vom Betreiber vorgegebenen Grenzwert bleibt. Die Bestimmung des optimalen Grenzwerts ist je nach Biogasanlage durch Versuche zu ermitteln.

Im Laufe der Arbeit wurden Untersuchungen über die Wärmeverteilung im Gärbehälter durchgeführt. Die Ergebnisse verdeutlichen, dass bei ausgeschalteten Rührwerken und niedrigen Außentemperaturen die Temperatur an unterschiedlichen Stellen des Gärbehälters über einen relativ langen Zeitraum (mehrere Minuten) konstant bleibt. Das Rühren scheint somit einen geringeren Einfluss auf die Wärmeverteilung zu haben als gemutmaßt wurde.

Ein weiterer Aspekt der Erfindung betrifft die Berücksichtigung der Dichte des Faulschlamms in einem Gärbehälter der Biogasanlage. Der Gärbehälter kann z. B. der Fermenter oder der Nachgärer sein. Es kann auch in dem Fermenter und in dem Nachgärer die Dichte des Faulschlamms berücksichtigt werden. Die nachfolgend erläuterten Ausführungsformen der Erfindung können kombiniert mit oder auch unabhängig von den zuvor erläuterten Ausführungsformen realisiert werden.

Gemäß einer vorteilhaften Ausführungsform der Erfindung wird ein Verfahren zum Betrieb einer Biogasanlage vorgeschlagen, bei dem zur Dichtemessung des Faulschlamms in einem Gärbehälter der Biogasanlage wenigstens ein erster Druckwert in einer ersten Höhe des Gärbehälters und wenigstens ein zweiter Druckwert in einer zweiten, anderen Höhe des Gärbehälters gemessen wird und aus dem ersten und dem zweiten Druckwert die Dichte bestimmt wird. Zur Bestimmung des ersten und des zweiten Druckwerts können in unterschiedlichen Höhen des Gärbehälters angeordnete Drucksonden, z. B. in Form von Drucksensoren, eingesetzt werden. Es sind wenigstens zwei Drucksonden in unterschiedlichen Höhen des Gärbehälters erforderlich. Es können auch mehr als zwei Drucksonden verwendet werden, wodurch die Genauigkeit der Dichtemessung noch erhöht werden kann. Zudem ist damit eine genauere Aussage über die Dichte des Faulschlamms in unterschiedlichen Höhenschichten möglich.

Gemäß einer vorteilhaften Ausführungsform der Erfindung wird anhand der ermittelten Werte der Dichte des Faulschlamms das Rührwerk eingeschaltet.

Im Laufe der Untersuchung des Faulschlammes im Fermenter der Biogasanlage 2 wurde festgestellt, dass die Dichte des Faulschlammes durch das Ansteigen des Biogasgehaltes nach Abschalten der Rührwerke mit der Zeit sinkt. Der Biogasgehalt erreichte sogar den Wert von 15 % des Gesamtnutzvolumens des Reaktors. Somit wurde ein erheblicher Teil des Volumens des Reaktors durch Biogasblasen "gefüllt". Das Einhalten einer bestimmten Dichte ist wichtig, da sonst eine Reduktion des Nutzvolumens des Reaktors erfolgt und die Gefahr der Überfüllung des Reaktors durch das Ansteigen des Faulschlammkuchens besteht. Für die Untersuchung der Dichte des Faulschlammes sind bisher keine Messtechniken bekannt und auf dem Markt erhältlich.

Ein Messsystem für die Untersuchung der Dichte des Faulschlammes, das im Rahmen der Arbeit entwickelt wurde, setzt sich aus mindestens zwei Drucksonden zusammen (die Anzahl der Sonden kann beliebig hoch sein), die untereinander auf verschiedenen Tiefen unter der Faulschlammoberfläche angeordnet werden. Da der Höhenunterschied zwischen den Sonden bekannt ist, kann die Dichte mit ausreichender Genauigkeit mit Hilfe der Gleichung 6.1 gerechnet werden.

$$\rho = \frac{p_2 - p_1}{g \cdot \Delta h} \qquad\qquad \text{[Gleichung 4.1]}$$

$\rho$ - Dichte des Faulschlammes [kg/m$^3$]
$p_1$ - Messwert des oberen Drucksensors [Pa]
$p_2$ - Messwert des unteren Drucksensors [Pa]
$g$ - Erdbeschleunigung [m/s$^2$]
$\Delta h$ - Höhenunterschied zwischen der Drucksensoren [m]

In der vorgeschlagenen Regelung würden die Rührwerke mit Hilfe des Reglers anhand der Dichte geregelt. Der Einschaltpunkt wäre bei der Unterschreitung eines bestimmten Wertes (z. B. 900 kg/m$^3$). Die Rührwerke könnten erst dann ausgeschaltet werden, wenn die Dichte des Faulschlammes höher als der obere Wert (z. B. 980 kg/m$^3$) wäre.

Die vorgeschlagene Regelung wäre die Ergänzung zur herkömmlichen Zeit-Pause Regelung. In der klassischen Rührwerksregelung werden die Rührwerke direkt nach der Substratzufuhr eingeschaltet und laufen eine festgelegte Zeit lang.

4.1.5 Steuerung und Regelung der Gasverwertungsanlagen und der Gasspeicher

Ein weiterer Aspekt der Erfindung betrifft die Optimierung des Betriebs einer Biogasanlage durch Einbeziehung von damit verbundenen Gasverwertungsanlagen und Gasspeichern. Die nachfolgend erläuterten Ausführungsformen der Erfindung können kombiniert mit oder auch unabhängig von den zuvor erläuterten Ausführungsformen realisiert werden.

Gemäß einer vorteilhaften Ausführungsform der Erfindung wird ein Verfahren zum Betrieb einer Biogasanlage vorgeschlagen, bei dem eine Sollleistung einer Gasverwertungsanlage vorgegeben wird, die mit der Biogasanlage verbunden ist und mit Biogas und/oder Methan aus einem Gasspeicher der Biogasanlage versorgt wird, wobei die Sollleistung unter Berücksichtigung der zeitlichen Änderung des in einem Gasspeicher der Biogasanlage gespeicherten Gasvolumens bestimmt wird. Dies erlaubt eine Vorausschau auf die zukünftige Entwicklung des im Gasspeicher der Biogasanlage gespeicherten Gasvolumens und damit eine vorausschauende und verbesserte Steuerung der Biogasanlage.

In der Regel soll die Gasverwertungsanlage 100 % des Biogases verwerten. Jede Anlage hat eine bestimmte Dauerdurchsatzleistung und kann zusätzlich kurzzeitig mit höherer Leistung arbeiten.

Die Vorgabe der Sollleistung wurde auf den im Rahmen der Arbeit untersuchten Biogasanlagen vom Betreiber oder halb automatisch vom Prozessleitsystem erteilt. Vorgaben vom Betreiber beruhen überwiegend auf der Einschätzung der Werte anhand des Vortages. Dies führte zur Überschätzung oder Unterschätzung der Biogasproduktion. Dadurch kann es oft zum Entleeren oder zur Überfüllung der Gasspeicher kommen.

Die Vorgabe des Prozessleitsystems auf den untersuchten Biogasanlagen basierte entweder auf der Füllstandsmessung des Gasbehälters oder auf der Druckmessung im Gasbehälter. Dies führte zu einem sehr ungleichmäßigen Betrieb des BHKW (die produzierte Leistung schwankte um den Mittelwert) durch sprunghafte Änderung der Sollleistung. Diese suboptimale Arbeitsweise kann zusätzlich zur Verkürzung der Lebensdauer der Gasverwertungsanlage führen.

Hiermit wird eine Lösung für die Berechnung der Soll-Leistung-Vorgabe für Gasverwertungsanalgen vorgeschlagen, die auf der Bilanzierung zur Berechnung der tatsächlichen Methan- und Biogasproduktion basiert. Um das Ziel zu erreichen, müssen in dem Gasspeicher der Druck und die Temperatur gemessen werden. Zusätzlich dazu müssen die Füllstände im Behälter sehr genau bestimmt werden. Die Berechnung der tatsächlichen Methan- und Biogasproduktion spielt für die Vorgabe der Soll-Leistung die bedeutendste Rolle. Die Berechnung basiert im Wesentlichen auf der Massenbilanzgleichung des Gasspeichers:

$$\frac{dV_0}{dt} = q_{gb} - q_{gv} \qquad\qquad \text{[Gleichung 4.2]}$$

$V_0$ - Normvolumen des Biogasbehälters [m$^3$]
$t$ - Zeit [h]
$q_{gb}$- Biogasbildung [Nm$^3$/h]
$q_{gv}$ - Biogasverbrauch durch Gasverwertungsanlage [Nm$^3$/h]

Die Geschwindigkeit der Volumenzunahme des Gasbehälters wird mit Hilfe folgender Gleichung gerechnet:

$$\frac{dV_0}{dt} \approx \frac{\Delta V_0}{\Delta t} = \frac{T_0}{p_0 \cdot t}\left( \frac{p_2 \cdot V_2}{T_2} - \frac{p_1 \cdot V_1}{T_1} \right) \qquad\qquad \text{[Gleichung 4.3]}$$

$T_0$ - Temperatur in Normbedingungen, 273,15 K
$p_0$ - Druck in Normbedingungen 1,01325 bar
$p_1$ - Gasdruck im Gasbehälter am Anfang der Zeitperiode $\Delta$t [Pa]
$p_2$- Gasdruck im Gasbehälter am Ende der Zeitperiode At [Pa]
$V_1$ - Gasspeichervolumen am Anfang der Zeitperiode $\Delta$t [m$^3$]
$V_2$ - Gasspeichervolumen am Ende der Zeitperiode $\Delta t$ [m$^3$]
$T_1$ - Temperatur im Gasspeicher am Anfang der Zeitperiode $\Delta$t [K]
$T_2$ - Temperatur im Gasspeicher am Ende der Zeitperiode $\Delta$t [K]

Je nach Messverfahren zur Gasspeichervolumenerfassung muss das jeweilige Gasspeichervolumen anhand individueller Abhängigkeiten berechnet werden. Am häufigsten wird die Messung mit Hilfe der Seilsonde verwendet. Somit besteht die Abhängigkeit:

$$V = f(L)$$ [Gleichung 4.4]

L - Anzeige der Seilsonde [%]

Die elektrische Leistung des gebildeten Gases für die Verwertung vom BHKW kann mit Hilfe der folgender Gleichung errechnet werden:

$$W = \eta \cdot c_{CH4} \cdot \phi \cdot q_{gb}$$ [Gleichung 4.5]

W - elektrische Leistung [kW] $\eta$ - elektrischer Wirkungsgrad des BHKW [%]
$c_{CH4}$ Volumenanteil von Methan in Biogas [%]

$$9{,}986 \frac{kWh}{Nm^3}$$

$\phi$ - Heizwert des Methans, ca.

Die Vorgabe der Soll-Leistung anhand der gemessenen Biogasproduktion ist eine grundlegende Größe für die Steuerung und Regelung der Gasverwertungsanlage.

Auf der anderen Seite muss die Biogasanlage über eine ausreichende Kapazität des freien Gasspeichers verfügen. Idealerweise sollten die Gasspeicher der Biogasanlage immer leer sein, damit das gesamte Volumen im Fall des Ausfalls der Gasverwertung zur Verfügung stehen könnte.

Im Rahmen der Arbeit wurde gezeigt, dass die Messung des Gasspeichervolumens mit Hilfe der Seilsonde sehr ungenau ist, da z. B. für den Gasspeicher der Biogasanlage 2 die "0" Wert Anzeige der Sonde etwa von 0 %vol. bis 68 %vol. des Gesamtvolumens des Gasspeichers entspricht. Die Messung des Gasfüllstands muss deswegen verbessert werden, da das Bereitstellen eines möglichst großen Volumens in einer Minderung der Gasverluste und Erhöhung des Gewinns resultiert.

Da es zurzeit keine bessere Messtechnik auf dem Markt in diesem Bereich gibt, wird auf deren Grundlage die Regelung zur optimalen Kapazitätsnutzung des Gasspeichers durch einen einfachen Regler vorgeschlagen. Für die Biogasanlage 2 würden die beiden Einschaltpunkte des Reglers z. B. als 75 % und 85 % des Volumens (die Werte sind hoch aufgrund der vorhin angesprochenen Defizite der Seilsonde) festgelegt. Ist der Gasspeicher stärker als die obere Grenze gefüllt, wird die Verstärkung der Leistungsvorgabe der vorgestellten Steuerung der Gasverwertungsanlage eingesetzt (z. B. 1,3-mal höhere Vorgabe als berechnet). Wird der Gasspeicher unter der unteren Grenze gefüllt, wird die Leistungsvorgabe der Biogasverwertung reduziert (z. B. 0,7-mal die Vorgabe des ersten Reglers). Die Regelung kann auch in dem Fall mit Hilfe eines einfachen PID-Reglers realisiert werden.

Gemäß einer vorteilhaften Ausführungsform der Erfindung wird daher ein Verfahren zum Betrieb einer Biogasanlage vorgeschlagen, bei dem eine Sollleistung einer Gasverwertungsanlage vorgegeben wird, die mit der Biogasanlage verbunden ist und mit Biogas und/oder Methan aus einem Gasspeicher der Biogasanlage versorgt wird, wobei die Sollleistung um einen vorbestimmten ersten Wert, z. B. 30%, gegenüber einer ansonsten vorgegebenen Standard-Sollleistung erhöht wird, wenn das in dem Gasspeicher (12) gespeicherte Gasvolumen einen vorbestimmten Höchstwert erreicht oder überschreitet, z. B. 75% oder 85% des zur Verfügung stehenden Gasspeichervolumens. Das Verfahren kann auch mit dem zuvor genannten Verfahren, bei dem die Sollleistung unter Berücksichtigung der zeitlichen Änderung des in einem Gasspeicher der Biogasanlage gespeicherten Gasvolumens bestimmt wird, kombiniert werden.

Gemäß einer vorteilhaften Ausführungsform der Erfindung wird die Sollleistung um einen vorbestimmten zweiten Wert, z. B. 30%, gegenüber einer ansonsten vorgegebenen Standard-Sollleistung reduziert wird, wenn das in dem Gasspeicher (12) gespeicherte Gasvolumen einen vorbestimmtem Mindestwert erreicht oder unterschreitet, z. B. 15% oder 25% des zur Verfügung stehenden Gasspeichervolumens.

4.1.6 Weitere vorteilhafte Realisierungen

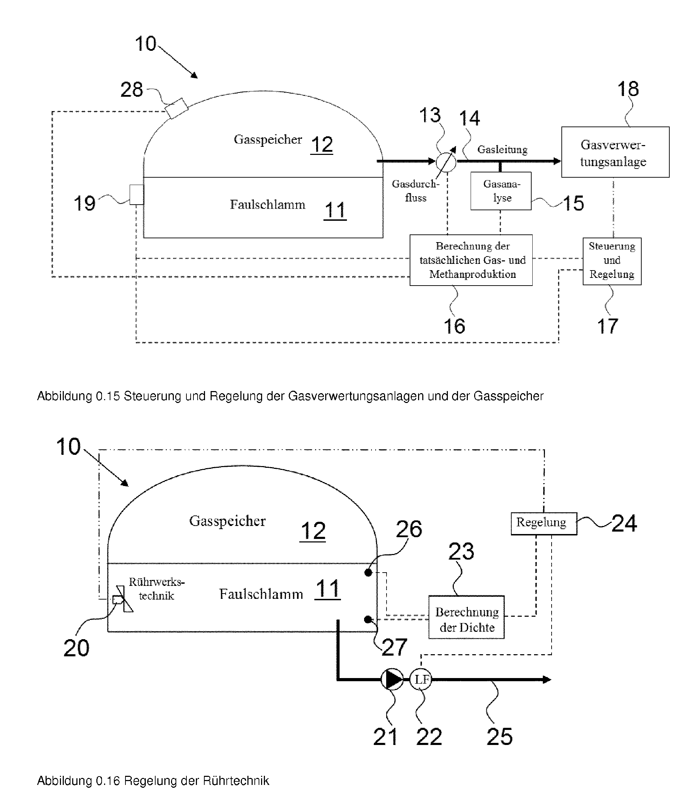

Abbildung 0.15 und Abbildung 0.16 zeigen Ausführungsformen der Erfindung. In beiden Abbildungen ist ein Gärbehälter 10 dargestellt, der als Fermenter oder Nachgärer ausgebildet sein kann. Der Gärbehälter 10 weist einen unteren Bereich auf, in dem Faulschlamm 11 vorhanden ist. Oberhalb des Faulschlamms 11 befindet sich ein Gasspeicher für entstehendes Biogas bzw. Methan. Anhand Abbildung 0.15 und Abbildung 0.16 werden verschiedene Aspekte der Biogasanlage der besseren Übersichtlichkeit getrennt voneinander beschrieben. Vorteilhaft ist jedoch auch eine Kombination der beschriebenen Merkmale in der selben Biogasanlage.

Gemäß Abbildung 0.15 ist an dem Gärbehälter 10 ein Sensor zur Bestimmung des Volumens im Gasspeicher 12 vorgesehen, und zwar in Form einer Seilsonde 19. Mit der Seilsonde 19 wird der Füllstand des Gasspeichers 12 in dem Gärbehälter 10 gemessen. Ferner weist der Gärbehälter 10 im Bereich des Gasspeichers 12 ein Sensormodul 28 auf, das einen Temperatursensor und einen Drucksensor aufweist. Gegebenfalls können auch weitere Sensoren darin vorgesehen sein. Das Sensormodul 28 und die Seilsonde 19 sind über elektrische Leitungen mit einer Auswerteeinrichtung 16 verbunden. Ferner ist in einer Gasabgabeleitung 14, durch die Gas aus dem Gasspeicher 12 zu einer Gasverwertungsanlage 18, z. B. einem BHKW, transportiert wird, ein Gasdurchflussmesser 13 vorgesehen. Der Gasdurchflussmesser 13, der in Normkubikmetern den Gasdurchfluss misst, ist über eine elektrische Leitung ebenfalls mit der Auswerteeinrichtung 16 verbunden. An die Gasabgabeleitung 14 kann ferner eine Gasanalyseeinrichtung 15 angeschlossen sein, mittels der die Zusammensetzung (z. B. Methangehalt) des durch die Gasabgabeleitung 14 geführten Gases erfasst werden. Die Gasanalyseeinrichtung 15 ist ebenfalls über eine elektrische Leitung mit der Auswerteeinheit 16 verbunden.

Die Auswerteeinrichtung 16 kann ein Computer oder eine sonstige rechnergesteuerte Einrichtung sein. Die Auswerteeinrichtung 16 bestimmt anhand der zugeführten Sensorsignale die tatsächliche Biogas- und/oder Methanproduktion der Biogasanlage z.B. unter Verwendung der Größen Gasdurchflussmenge, die von dem Gasdurchflussmesser 13 ermittelt wird, und dem Volumen im Gasspeicher 12, ermittelt durch die Seilsonde 19. Es kann auch die Volumenänderung in dem Gasspeicher 12 verwendet werden. In diesem Fall wird durch die Auswerteeinrichtung 16 z. B. die zeitliche Ableitung des Signals der Seilsonde 19 bestimmt. Die Auswerteeinrichtung 16 sowie Seilsonde 19 geben Ausgangsdaten an eine Steuerungs- und Regelungseinrichtung 17 ab, die zur Steuerung und Regelung der Gasverwertungsanlage 18 vorgesehen ist. Hierdurch wird z. B. die Sollleistung der Gasverwertungsanlage 18 gesteuert.

Gemäß Abbildung 0.16 ist in dem Faulschlammbereich des Gärbehälters 10 ein Rührwerk 20 vorgesehen, das von einer Regelung 24 betätigbar ist. Ferner ist ein erster Drucksensor 26 und ein zweiter Drucksensor 27 an oder in dem Gärbehälter 10 angeordnet, und zwar im Bereich des Faulschlamms 11. Der erste Drucksensor 26 ist an einer höheren Stelle als der zweite Drucksensor 27 angeordnet. Der erste Drucksensor 26 gibt einen ersten Druckwert $p_1$ an eine Berechnungseinrichtung 23 ab. Der zweite Drucksensor 27 gibt einen zweiten Druckwert $p_2$ an die Berechnungseinrichtung 23 ab. Die Berechnungseinrichtung 23 ist dazu eingerichtet, die Dichte des Faulschlamms 11 in dem Gärbehälter 10 anhand des ersten und zweiten Druckwerts $p_1$, $p_2$ zu bestimmen. Die Berechnungseinrichtung 23 gibt den berechneten Dichtewert an die Regelung 24 ab. Die Regelung 24 ist dazu eingerichtet, das Rührwerk 29 einzuschalten, wenn die ermittelten Werte der Dichte des Faulschlamms 11 einen vorbestimmten Grenzwert unterschreiten.

Ferner ist ein Leitfähigkeitssensor 22 vorgesehen, der zur Bestimmung der elektrischen Leitfähigkeit des Faulschlamms 11 dient. Der Leitfähigkeitssensor 22 gibt ein Signal, dass die Leitfähigkeit des Faulschlamms 11 anzeigt, an die Regelung 24 ab. Die Regelung 24 ist dazu eingerichtet, bei bestimmten Leitfähigkeitswerten das Rührwerk 20 einzuschalten, z. B. wenn die Leitfähigkeitswerte über die Zeit zu stark schwanken, was dadurch ermittelt werden kann, dass die Standardabweichung der Leitfähigkeitswerte bestimmt wird und das Rührwerk 20 eingeschaltet wird, wenn die Standardabweichung größer als sein Grenzwert ist.

Gemäß Abbildung 0.16 ist der Leitfähigkeitssensor 22 in einer Faulschlammleitung 25 angeordnet. Die Faulschlammleitung 25 dient zum Abtransport von Faulschlamm 11 aus dem Gärbehälter 10. Hierzu ist eine Pumpe 21 vorgesehen. Der Leitfähigkeitssensor 22 kann auch direkt im Bereich des Faulschlamms 11 des Gärbehälters 10 angeordnet sein.

Die Erfindung betrifft ferner eine Einrichtung zur Ausführung eines Verfahrens der zuvor beschriebenen Art, z.B. eine Einrichtung zur Vorgabe einer Sollleistung einer Gasverwertungsanlage und/oder eine Einrichtung zur Regelung der Substratzufuhr einer Biogasanlage. Die Einrichtung kann insbesondere einen Computer mit einem Rechner aufweisen, auf dem ein Computerprogramm zur Durchführung des Verfahrens ausgeführt wird.

Die Erfindung betrifft ferner ein Computerprogramm mit Programmcodemitteln, eingerichtet zur Durchführung eines Verfahrens der zuvor beschriebenen Art, wenn das Computerprogramm auf einem, Rechner ausgeführt wird.

Die Erfindung betrifft ferner ein solches auf einem maschinenlesbaren Träger gespeichertes Computerprogramm.

5 Zitierte Literatur

1. Batstone, D.J., Keller, J., Angelidaki, R.I., Kalyuzhnyi, S.V., Pavlostathis, S.G., Rozzi, A., Sanders, W.T.M., Siegrist, H. and Vavilin, V.A. (2002) Anaerobic Digestion Model No.1 (ADM1), IWA Task Group for Mathematical Modelling of Anaerobic Digestion Processes, STR No. 13, IWA Publishing, London, UK. ISBN: 9781900222785

2. Bischoff, M. (2007): Was passiert im Fermenter - Parameter und deren Interpretation; Vortrag anlässlich des Biogas-Workshops "Prozessoptimierung in Biogasanlagen für nachwachsende Rohstoffe", Landwirtschaftszentrum Haus Düsse, 30.05.2007

3. Bischofsberger W., Rosenwinkel K., Dichtl N., Seyfried C.F. (2005): Anaerobtechnik Springer-Verlag Berlin Heidelberg, ISBN 3-540-06850-3

4. Cimatoribus C., (2009): Simulation and nonlinear control of anaerobic digestion, Dissertation von der Fakultät Bau- und Umweltingenieurwissenschaften der Universität Stuttgart, Institut für Siedlungswasserbau, Wassegüte- und Abfallwirtschaft der Universität Stuttgart

5. De Lemos Chernicharo C., A., (2007): Anaerobic reactors, volume four, IWA Publishing, London, New York, ISBN 1 84339 164 3, ISBN 13: 9781843391647

6. Dublein D. and Steinhauser A. (2008): Biogas from Waste and Renewable Resources. Wiley-VCH Verlag GmbH &Co. KGaA, Weinheim ISBN: 978-3-527-31847-4

7. Fachagentur Nachwachsende Rohstoffe e.V. (2006): Handreichung Biogasgewinnung und -nutzung, Institut für Energetik und Umwelt gGmbH, Bundesforschungsanstalt für Landwirtschaft, Kuratorium für Technik und Bauwesen in der Landwirtschaft e.V., Gülzow,

8. Fachagentur Nachwachsende Rohstoffe e.V. (2009): Biogas-Messprogramm II. 61 Biogasanlagen im Vergleich. Johann Heinrich von Thünen-Institut (vTI), Leibniz-Institut für Agrartechnik Potsdam-Bornim e.V., Bayerische Landesanstalt für Landwirtschaft, Universität Hohenheim, Deutsches Biomasse Forschungs Zentrum gGmbH, ISBN 978-3-9803927-8-5

9. Fachagentur Nachwachsende Rohstoffe e.V. (2010) Leitfaden Biogas. Von der Gewinnung zur Nutzung, Deutsches BiomasseForschungsZentrum gemeinnützige GmerbH, Kuratorium für Technik und Bauwesen in der Landwirtschaft e.V., Johann Heinrich von Thünen-Institut (vTI), Rechtsanwaltskanzlei Schnutenhaus & Kollegen, Gülzow,

10. Franke M., Weger A., Faulstich M. (2008): Prozessregelung von Vergärungsanlagen mit Hilfe des Parameters Wasserstoff Gülzower Fachgespräche "Messen, Steuern, Regeln bei der Biogaserzeugung" Band 27, Herausgeber: Fachagentur Nachwachsende Rohstoffe e.V. (FNR)

11. Golkowska K., Greger M. (2009) Thermophilic digestion of cellulose - Investigation of the inhibitory state. Internationale Wissenschaftstagung Biogas Science 2009, 02.12.-04.12.2009, Erding. Schriftenreihe der Bayerischen Landesanstalt für Landwirtschaft, Band 3/17

12. Heinzle et al. (1993): Modelling and control of anaerobic wastewater treatment. Advance in Biochemical Engineering/Biotechnology, 48, 79-114

13. Kapp, H (1984): Schlammfaulung mit hohem Feststoffgehalt, Stuttgarter Berichte zur Siedlungswasserwirtschaft 86, Verlag R. Oldenbourg, München

14. Kapp, H (1992): Schlammfaulung mit hohem Feststoffgehalt. Stuttgarter Berichte zur Siedlungswasserwirtschaft, Band 86, Oldenburg Verlag, Münschen, 300 pp.

15. Kessler, W., (2007): Multivariate Datenanalyse für die Pharma-, Bio- und Prozessanalytik. Wiley-VCH Verlag, Weinheim

16. Kranert, M., Cord-Landwehr, K., (2010): Einführung in die Abfallwirtschaft, 4. Ausgabe, Herausgeber: Maritn Kranert, ISBN: 978-3-8348-9681-0

17. Kranert, M., Fischer K., Hafner G., Steinbach D., Schultheis A., (2008): MODULAARE - Integrierte Module zur hocheffizienten Abwasserreinigung, Abfallbehandlung und regenerativen Energiegewinnung in Tourismus Resorts, Endbericht zum Biogasmodul, Universität Stuttgart, Institut für Siedlungswasserbau, Wassergüte- und Abfallwirtschaft, Lehrstuhl für Abfallwirtschaft und Abluft

18. Kujawski O., Steinmetz H. (2009): Development of instrumentation systems as a base for control of digestion process stability in full-scale agricultural and industrial biogas plants, Water Science & Technology—WST Vol 60 No 8 pp 2055-2063 © IWA Publishing

19. Kujawski O., Wiese J., König R. and Häck M. (2007): Instrumentation, Control and Automation for Agricultural and Co- Digestion Biogas Plants - Yesterday, Today and Tomorrow, Proceedings, 11th IWA-Specialist conference on anaerobic digestion, Brisbane, Australia

20. Kuratorium für Technik und Bauwesen in der Landwirtschaft e.V. (KTBL) (2007): Faustzahlen für Biogas, Herausgeber: KTBL und Fachagentur Nachwachsende Rohstoffe e.V. (FNR)

21. Lebuhn M., Andrade D., Bauer C., Gronauer A., (2010) Optimierung der Verfahrenstechnik und Prozessautomatisierung, Prozessmonitoring und Datenmanagement, Identifikation und Quantifizierung funktionell relevanter Mikroorganismen bei der Vergärung LCB-reicher nachwachsender Rohstoffe. Abschlussbericht des Verbundprojektes: Intensivierung des anaeroben Biomasseabbaus zur Methanproduktion aus NawaRo (IBMN), Internetseite: http://www.fnr-server.de/ftp/pdf/berichte/22011505.pdf

22. Mähnert P. (2007): Kinetik der Biogasproduktion aus nachwachsenden Rohstoffen und Gülle. Dissertation. Landwirtschaftlich-Gärtnerischen Fakultät der Humboldt-Universität zu Berlin

23. Moosburger R.E., Wenzel M.C., Ekama G.A. and Marais G., Pind P.F., Angelidaki I., Ahring B.K. (2003): A new VFA sensor technique for anaerobic reactor systems. Biotechnology and Bioengineering, 82 (1), 64-61

24. Nacke T., Barthel A., Haendly D., Beckmann D. (2008): Inline-Mikrowellen-Prozesssensorik zur Bestimmung des Trockenmasseanteils im Biogasfermenter, Gülzower Fachgespräche "Messen, Steuern, Regeln bei der Biogaserzeugung" Band 27, Herausgeber: Fachagentur Nachwachsende Rohstoffe e.V. (FNR)

25. Prause, F. (1997): Zusammenstellung mathematischer Modelle zur anaeroben Abwasserreinigung, Diplomarbeit am Lehrstuhl für Siedlungswasserwirtschaft und Abfalltechnik, Universität Hannover, unveröffentlicht

26. Prechtl S., Weger A., Faulstisch M., (2006): Wasserstoff steuert Gärprozess, Biogas Journal 1/06 S. 10

27. Punal, A., Palazzotto, L, Lardon, L., Steyer, J.P. (2004): Compared fuzzy logic approaches for automatic control for CH4 flow rate production and VFA effluent concentration in anaerobic digestion [TELEMAC Contri-bution #13]

28. Scherer P., Ergun S., Schmidt O., Löffelholz J., Henning-Jacob J., Vollmer G.-R. (2008) Entwicklung einer Fuzzy-Logik-Regelung für eine Hochdurchsatz-Biogasanlage: "Telefermentation" zwischen Hamburg und Nordhausen, Gülzower Fachgespräche "Messen, Steuern, Regeln bei der Biogaserzeugung" Band 27, Herausgeber: Fachagentur Nachwachsende Rohstoffe e.V. (FNR)

29. Torsvik, V. and Øvreås, L. (2002): Microbial diversity and function in soil: from genes to ecosystems. Curr. Opin. Microbiol. 5, 240-245. Van Der Steene et al., (2002): Automatic Buffer Capacity Model Building for Advanced Interpretation of Titration Curves, Environmental Science and Technology, 36, 715-723

30. Voß, E., Weichgrebe, D., Rosenwinkel, K.H., (2009): FOS/TAC: Herleitung, Methodik Anwendung und Aussagekraft. Internationale Wissenschaftstagung Biogas Science 2009, 02.12.-04.12.2009, Erding. Schriftenreihe der Bayerischen Landesanstalt für Landwirtschaft, Band 3/17.

31. Warmenhoven J. W., Spanjers H (2009): TOC based control of anaerobic reactor treating wastewater from a fruit juice packaging factory. Proceedings 10TH IWA Conference on Instrumentation, Control and Automation, Cairns, Australia

32. Weiland P. (2000): Stand und Perspektiven der Biogasnutzung und -Erzeugung in Deutschland; Gülzower Fachgespräche Band 15: Energetische Nutzung von Biogas: Stand der Technik und Optimierungspotenzial, 8-27; Fachagentur nachwachsende Rohstoffe.

33. Weiland, P. (2003): Biologie der Biogaserzeugung. ZNR Biogastagung, 02.04.2003, Vortrag

34. Weiland P. (2008): Wichtige Messdaten für den Prozessablauf und Stand der Technik in der Praxis, Gülzower Fachgespräche "Messen, Steuern, Regeln bei der Biogaserzeugung" Band 27, Herausgeber: Fachagentur Nachwachsende Rohstoffe e.V. (FNR)

35. Weiland P., Rieger, Ch. (2006): Prozessstörungen frühzeitig erkennen. Biogas Journal 9, 5, 18

36. Wellinger, A. (1991): Biogas-Handbuch, Grundlagen - Planung - Betrieb landwirtschaftlicher Anlagen; Verlag Wirz, Aarau

37. Wolf C., Bongards M., McLoone S. (2007): Simulation-based feed-optimization of medium-sized agricultural biogas plants Proceedings 11 th IWA World Congress on Anaerobic Digestion 23-27 September 2007 Brisbane, Australia

38. Zaher U., Bouvier J.C., Steyer J.P., Venrolleghem P.A. (2004): Titrimetric Monitoring of Anaerobic Digestion: VFA, Alkalinities and More, , Proceedings 10TH World Congress on Anaerobic Digestion, Montreal Canada, August 29 - September, 2004

**Patentansprüche**

1. Verfahren zur Vorgabe einer Sollleistung einer Gasverwertungsanlage (18), die mit einer Biogasanlage verbunden ist und mit Biogas und/oder Methan aus einem Gasspeicher (12) der Biogasanlage versorgt wird, **dadurch gekennzeichnet, dass**

   a) die Sollleistung unter Berücksichtigung der zeitlichen Änderung des in einem Gasspeicher (12) der Biogasanlage gespeicherten Gasvolumens bestimmt wird
   und/oder
   b) die Sollleistung um einen vorbestimmten ersten Wert gegenüber einer ansonsten vorgegebenen Standard-Sollleistung erhöht wird, wenn das in dem Gasspeicher (12) gespeicherte Gasvolumen einen vorbestimmten Höchstwert erreicht oder überschreitet.

2. Verfahren nach Anspruch 1, **dadurch gekennzeichnet, dass** die Sollleistung um einen vorbestimmten zweiten Wert gegenüber einer ansonsten vorgegebenen Standard-Sollleistung reduziert wird, wenn das in dem Gasspeicher (12) gespeicherte Gasvolumen einen vorbestimmtem Mindestwert erreicht oder unterschreitet.

3. Verfahren nach einem der vorhergehenden Ansprüche, **dadurch gekennzeichnet, dass** zur Regelung der Biogasanlage die Zufuhr von Substraten zu einem Fermenter (1) und einem Nachgärer (2) der Biogasanlage durch ein Regelungssystem (3) im laufenden Betrieb der Biogasanlage geregelt wird, dem als Eingangsgröße (4) eine durch Computersimulation bestimmte Substratzufuhrstrategie zugeführt wird, die nach Maßgabe der verfügbaren Substrate bestimmt ist.

4. Verfahren nach Anspruch 3, **dadurch gekennzeichnet, dass** anhand der Vorratsmengen und Arten der verfügbaren Substrate sowie deren charakteristischer Methanausbeutewerte und ihrer Abbaurate verschiedene zukünftige Substratzufuhrstrategien zunächst mittels eines Computers (5) simuliert werden, die Substratzufuhrstrategie mit einer angestrebten Methanausbeute bzw. Energieausbeute bei einer maximalen Abbaurate der organischen Trockenmasse ausgewählt wird und sodann die Regelung der Biogasanlage gemäß der ausgewählten Substratzufuhrstrategie erfolgt.

5. Verfahren nach einem der Ansprüche 3 bis 4, **dadurch gekennzeichnet, dass** aus den simulierten Substratzufuhrstrategien diejenige ausgewählt wird, bei der die $NH_4$-N-Konzentrationen im Fermenter (1) und im Nachgärer (2) der Biogasanlage jeweils kleiner als ein vorgegebener Sollwert sind.

6. Verfahren nach einem der Ansprüche 3 bis 5, **dadurch gekennzeichnet, dass** diejenige Substratzufuhrstrategie ausgewählt wird, bei der die Trockenmassekonzentration im Fermenter (1) und im Nachgärer (2) jeweils kleiner ist als ein vorgegebener Sollwert.

7. Verfahren nach einem der Ansprüche 3 bis 6, **dadurch gekennzeichnet, dass** im laufenden Betrieb der Biogasanlage eine oder mehrere der Größen: Methanausbeute, Biogasausbeute und Abbaurate der der Biogasanlage zugeführten einzelnen Substrate unter Heranziehung im laufenden Betrieb gemessener Zulauf- und Ablaufströme des Fermenters (1) und/oder des Nachgärers (2) der Biogasanlage nach Art einer Massenbilanz bestimmt werden und als Eingangsgröße (6) für die Computersimulation zur Bestimmung der Substratzufuhrstrategie verwendet werden.

8. Verfahren nach einem der Ansprüche 3 bis 7, **dadurch gekennzeichnet, dass** die tatsächliche Biogas- und/oder Methanproduktion der Biogasanlage wenigstens unter Verwendung der Größen Gasdurchflussmenge an einer Gasabgabeleitung (14) und Volumen bzw. Volumenänderung in einem Gasspeicher (12) bestimmt wird.

9. Verfahren nach Anspruch 8, **dadurch gekennzeichnet, dass** das Volumen bzw. die Volumenänderung im Gasspeicher (12) anhand eines Füllstandsensors (19, 29), eines Temperatursensors und eines Drucksensors (28) bestimmt wird.

10. Verfahren nach einem der vorhergehenden Ansprüche, **dadurch gekennzeichnet, dass** eine Messung der elektrischen Leitfähigkeit des Faulschlamms (11) im Fermenter (1) und/oder im Nachgärer (2) durchgeführt wird und anhand eines oder mehrerer gemessener Leitfähigkeitswerte das Einschalten des Rührwerks (20) im Fermenter (1) und/oder im Nachgärer (2) gesteuert wird.

11. Verfahren nach Anspruch 10, **dadurch gekennzeichnet, dass** mehrere Leitfähigkeitswerte bestimmt werden, hieraus die Standardabweichung der Leitfähigkeitswerte bestimmt wird und das Rührwerk (20) eingeschaltet wird, wenn die Standardabweichung größer als ein Grenzwert ist.

12. Verfahren nach einem der vorhergehenden Ansprüche, **dadurch gekennzeichnet, dass** zur Dichtemessung des Faulschlamms (11) in einem Gärbehälter (1, 2) der Biogasanlage wenigstens ein erster Druckwert ($p_1$) in einer ersten Höhe des Gärbehälters (1, 2) und wenigstens ein zweiter Druckwert ($p_2$) in einer zweiten, anderen Höhe des Gärbehälters (1, 2) gemessen wird und aus dem ersten und dem zweiten Druckwert ($p_1$, $p_2$) die Dichte bestimmt wird.

13. Verfahren nach Anspruch 12, **dadurch gekennzeichnet, dass** das Rührwerk (20) anhand der ermittelten Werte der Dichte des Faulschlamms (11) eingeschaltet wird.

14. Einrichtung zur Ausführung eines Verfahrens nach einem der vorhergehenden Ansprüche.

15. Computerprogramm mit Programmcodemitteln, insbesondere auf einem maschinenlesbaren Träger gespeichertes Computerprogramm, eingerichtet zur Durchführung des Verfahrens nach einem der Ansprüche 1 bis 13, wenn das Computerprogramm auf einem Rechner ausgeführt wird.

Abbildung 0.1 Verfahrensfließbild, Biogasanlage 1

Abbildung 0.2 Verfahrensfließbild, Biogasanlage 2

Abbildung 0.3 Verfahrensfließbild, Biogasanlage 3 (M&A – Misch- und Ausgleichsbehälter)

Abbildung 0.4 Spezifische Biogasrate $r_s$, Biogas-Summenkurve y und Zeitpunkt des erfüllten Abbruchkriteriums nach VDI-Richtlinie $t_{Grenz}$ ausgewählter Batch-Versuche mit Mais-, Rüben-, Roggensilage und Rindergülle [22]

**Abbildung 0.5 Gesamtabbaukinetik des anaeroben Abbaus ermittelt aus den Gassummenkurven von Batchversuchen**

Abbildung 0.6 Gemessene und mit Hilfe des Softsensors berechnete Methan- und Biogasproduktion der Biogasanlage 1

Abbildung 0.7 Gemessene und mit Hilfe des Softsensors berechnete Methan- und Biogasproduktion der Biogasanlage 3

Abbildung 0.8 Gemessene und mit Hilfe des Softsensors berechnete $NH_4$-N Konzentrationen für Fermenter und Nachgärer der Biogasanlage 1

Abbildung 0.9 Gemessene und mit Hilfe des Softsensors berechnete $NH_4$-N Konzentrationen für Fermenter der Biogasanlage 3

Abbildung 0.10 FOS Konzentrationen für Fermenter der Biogasanlage 1

EP 2 559 750 A1

Abbildung 0.11 Gemessene und berechnete mit Hilfe des Softsensors TS- und TR-Konzentrationen im Fermenter und im Nachgärer der Biogasanlage 1.

Abbildung 0.12 Gemessene und berechnete mit Hilfe des Softsensors TS- und TM-Konzentration im Fermenter und im Nachgärer der Biogasanlage 3

Abbildung 0.13 Wirkungskette für die entwickelte Regelung der Substratzufuhr für Biogasanlagen

Abbildung 0.14 Algorithmus des Regelungssystems.

10

28

13 14 18

Gasspeicher 12

Gasleitung

Gasverwertungsanlage

Gasdurch-fluss

Gasana-lyse

15

19

Faulschlamm 11

Berechnung der tatsächlichen Gas- und Methanproduktion

Steuerung und Regelung

16 17

Abbildung 0.15 Steuerung und Regelung der Gasverwertungsanlagen und der Gasspeicher

10

Gasspeicher 12

26

23

Regelung 24

Rührwerks-technik

Faulschlamm 11

Berechnung der Dichte

20

27

21 22 25

LF

Abbildung 0.16 Regelung der Rührtechnik

Europäisches
Patentamt

European
Patent Office

Office européen
des brevets

# EUROPÄISCHER RECHERCHENBERICHT

Nummer der Anmeldung

EP 12 18 0864

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | US 2009/305379 A1 (JOHNSON ET AL.) 10. Dezember 2009 (2009-12-10) | 1-4,7-9, 14,15 | INV. C12M1/00 |
| Y | * Absätze [0001], [0020] - [0025], [0030], [0035], [0036], [0042], [0045], [0054], [0055] * | 5,6 | |
| | ----- | | |
| X | WO 2009/000554 A2 (ASW ANLAGENBAU SCHLAMM- UND WASSERTECHNIK GMBH) 31. Dezember 2008 (2008-12-31) * Abbildung 1 * * Seite 3, Absatz 3 - Seite 5, Absatz 2 * * Seite 7 * | 1-4,7,8, 14 | |
| | ----- | | |
| X | DE 101 06 312 A1 (AQUA TEC GMBH) 29. August 2002 (2002-08-29) * Absätze [0009] - [0016], [0047], [0048], [0051], [0055] * * Abbildungen 1-4 * | 1-4,7,8, 14,15 | |
| | ----- | | |
| Y | DE 10 2007 018565 A1 (GEWITRA GMBH) 23. Oktober 2008 (2008-10-23) * Absätze [0001], [0008], [0009] * | 5,6 | RECHERCHIERTE SACHGEBIETE (IPC) |
| | ----- | | C12M |
| A | US 6 150 133 A (MEAD ET AL.) 21. November 2000 (2000-11-21) * Beispiele 1,4 * | 10,11 | |
| | ----- | | |
| A | DE 10 2007 047175 A1 (MARKO) 9. April 2009 (2009-04-09) * Absätze [0001], [0029], [0031], [0032] * | 10,11 | |
| | ----- | | |
| A | EP 0 192 900 A1 (UNION INDUSTRIELLE ET D'ENTREPRISE) 3. September 1986 (1986-09-03) * Seite 1, Absatz 1 * * Seite 13, Absatz 2; Abbildungen 4,5 * | 12,13 | |
| | ----- | | |

Der vorliegende Recherchenbericht wurde für alle Patentansprüche erstellt

| Recherchenort | Abschlußdatum der Recherche | Prüfer |
|---|---|---|
| Berlin | 14. Dezember 2012 | Alvarez Alvarez, C |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
........................................................................
& : Mitglied der gleichen Patentfamilie, übereinstimmendes Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 12 18 0864

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

14-12-2012

| Im Recherchenbericht angeführtes Patentdokument | Datum der Veröffentlichung | Mitglied(er) der Patentfamilie | Datum der Veröffentlichung |
|---|---|---|---|
| US 2009305379 A1 | 10-12-2009 | KEINE | |
| WO 2009000554 A2 | 31-12-2008 | KEINE | |
| DE 10106312 A1 | 29-08-2002 | KEINE | |
| DE 102007018565 A1 | 23-10-2008 | KEINE | |
| US 6150133 A | 21-11-2000 | AT 241695 T | 15-06-2003 |
| | | AU 702567 B2 | 25-02-1999 |
| | | AU 2104397 A | 01-10-1997 |
| | | CA 2242323 A1 | 18-09-1997 |
| | | DE 69722391 D1 | 03-07-2003 |
| | | DE 69722391 T2 | 22-04-2004 |
| | | DK 889949 T3 | 22-09-2003 |
| | | EP 0889949 A1 | 13-01-1999 |
| | | ES 2200153 T3 | 01-03-2004 |
| | | JP 4318751 B2 | 26-08-2009 |
| | | JP 2000506387 A | 30-05-2000 |
| | | PT 889949 E | 31-10-2003 |
| | | US 6150133 A | 21-11-2000 |
| | | WO 9733973 A1 | 18-09-1997 |
| DE 102007047175 A1 | 09-04-2009 | KEINE | |
| EP 192900 A1 | 03-09-1986 | KEINE | |

EPO FORM P0461

Für nähere Einzelheiten zu diesem Anhang : siehe Amtsblatt des Europäischen Patentamts, Nr.12/82

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Nicht-Patentliteratur**

- **BATSTONE, D.J. ; KELLER, J. ; ANGELIDAKI, R.I. ; KALYUZHNYI, S.V. ; PAVLOSTATHIS, S.G. ; ROZZI, A. ; SANDERS, W.T.M. ; SIEGRIST, H. ; VAVILIN, V.A.** Anaerobic Digestion Model No.1 (ADM1), IWA Task Group for Mathematical Modelling of Anaerobic Digestion Processes. IWA Publishing, 2002
- **BISCHOFF, M.** Was passiert im Fermenter - Parameter und deren Interpretation; Vortrag anlässlich des Biogas-Workshops. *Prozessoptimierung in Biogasanlagen für nachwachsende Rohstoffe,* 30. Mai 2007
- **BISCHOFSBERGER W. ; ROSENWINKEL K. ; DICHTL N. ; SEYFRIED C.F.** Anaerobtechnik. Springer-Verlag, 2005
- **CIMATORIBUS C.** *Simulation and nonlinear control of anaerobic digestion,* 2009
- **DE LEMOS CHERNICHARO C., A.** Anaerobic reactors, volume four. IWA Publishing, 2007
- **DUBLEIN D. ; STEINHAUSER A.** Biogas from Waste and Renewable Resources. Wiley-VCH Verlag GmbH &Co. KGaA, 2008
- *Handreichung Biogasgewinnung und -nutzung,* 2006
- *Biogas-Messprogramm II. 61 Biogasanlagen im Vergleich,* 2009, ISBN 978-3-9803927-8-5
- *Leitfaden Biogas. Von der Gewinnung zur Nutzung,* 2010
- Prozessregelung von Vergärungsanlagen mit Hilfe des Parameters Wasserstoff Gülzower Fachgespräche. **FRANKE M. ; WEGER A. ; FAULSTICH M.** Messen, Steuern, Regeln bei der Biogaserzeugung. 2008, vol. 27
- **GOLKOWSKA K. ; GREGER M.** *Thermophilic digestion of cellulose - Investigation of the inhibitory state. Internationale Wissenschaftstagung Biogas Science 2009,* 2009, vol. 3/17
- **HEINZLE et al.** Modelling and control of anaerobic wastewater treatment. *Advance in Biochemical Engineering/Biotechnology,* 1993, vol. 48, 79-114
- **KAPP, H.** Schlammfaulung mit hohem Feststoffgehalt, Stuttgarter Berichte zur Siedlungswasserwirtschaft 86. Verlag, 1984
- **KAPP, H.** Schlammfaulung mit hohem Feststoffgehalt. Stuttgarter Berichte zur Siedlungswasserwirtschaft. Oldenburg Verlag, 1992, vol. 86, 300

- **KESSLER, W.** Multivariate Datenanalyse für die Pharma-, Bio- und Prozessanalytik. Wiley-VCH Verlag, 2007
- **KRANERT, M. ; CORD-LANDWEHR, K.** Einführung in die Abfallwirtschaft. 2010
- **KRANERT, M. ; FISCHER K. ; HAFNER G. ; STEINBACH D. ; SCHULTHEIS A.** *MODULAARE - Integrierte Module zur hocheffizienten Abwasserreinigung, Abfallbehandlung und regenerativen Energiegewinnung in Tourismus Resorts, Endbericht zum Biogasmodul,* 2008
- Development of instrumentation systems as a base for control of digestion process stability in full-scale agricultural and industrial biogas plants. **KUJAWSKI O. ; STEINMETZ H.** Water Science & Technology—WST. IWA Publishing, vol. 60, 2055-2063
- **KUJAWSKI O. ; WIESE J. ; KÖNIG R. ; HÄCK M.** Instrumentation, Control and Automation for Agricultural and Co- Digestion Biogas Plants - Yesterday, Today and Tomorrow. *Proceedings, 11th IWA-Specialist conference on anaerobic digestion, Brisbane, Australia,* 2007
- Faustzahlen für Biogas. 2007
- **LEBUHN M. ; ANDRADE D. ; BAUER C. ; GRONAUER A.** Optimierung der Verfahrenstechnik und Prozessautomatisierung, Prozessmonitoring und Datenmanagement, Identifikation und Quantifizierung funktionell relevanter Mikroorganismen bei der Vergärung LCB-reicher nachwachsender Rohstoffe. *Abschlussbericht des Verbundprojektes: Intensivierung des anaeroben Biomasseabbaus zur Methanproduktion aus NawaRo (IBMN, http://www.fnr-server.de/ftp/pdf/berichte/22011505.pdf*
- **MÄHNERT P.** Kinetik der Biogasproduktion aus nachwachsenden Rohstoffen und Gülle. *Dissertation,* 2007
- **MOOSBURGER R.E. ; WENZEL M.C. ; EKAMA G.A. ; MARAIS G. ; PIND P.F. ; ANGELIDAKI I. ; AHRING B.K.** A new VFA sensor technique for anaerobic reactor systems. *Biotechnology and Bioengineering,* 2003, vol. 82 (1), 64-61
- Inline-Mikrowellen-Prozesssensorik zur Bestimmung des Trockenmasseanteils im Biogasfermenter, Gülzower Fachgespräche. **NACKE T. ; BARTHEL A. ; HAENDLY D. ; BECKMANN D.** Messen, Steuern, Regeln bei der Biogaserzeugung. 2008, vol. 27

- **PRAUSE, F.** Zusammenstellung mathematischer Modelle zur anaeroben Abwasserreinigung. *Diplomarbeit am Lehrstuhl für Siedlungswasserwirtschaft und Abfalltechnik,* 1997
- **PRECHTL S. ; WEGER A. ; FAULSTISCH M.** *Wasserstoff steuert Gärprozess, Biogas Journal,* Januar 2006, 10
- **PUNAL, A. ; PALAZZOTTO, L ; LARDON, L. ; STEYER, J.P.** Compared fuzzy logic approaches for automatic control for CH4 flow rate production and VFA effluent concentration in anaerobic digestion. *TELEMAC Contri-bution #13,* 2004
- Entwicklung einer Fuzzy-Logik-Regelung für eine Hochdurchsatz-Biogasanlage. **SCHERER P. ; ERGUN S. ; SCHMIDT O. ; LÖFFELHOLZ J. ; HENNING-JACOB J. ; VOLLMER G.-R.** Telefermentation. 2008, vol. 27
- **TORSVIK, V. ; ØVREÅS, L.** Microbial diversity and function in soil: from genes to ecosystems. *Curr. Opin. Microbiol.,* 2002, vol. 5, 240-245
- **VAN DER STEENE et al.** Automatic Buffer Capacity Model Building for Advanced Interpretation of Titration Curves. *Environmental Science and Technology,* 2002, vol. 36, 715-723
- **VOß, E.** *FOS/TAC: Herleitung, Methodik Anwendung und Aussagekraft. Internationale Wissenschaftstagung Biogas Science,* 2009, vol. 3/17
- **WARMENHOVEN J. W. ; SPANJERS H.** TOC based control of anaerobic reactor treating wastewater from a fruit juice packaging factory. *Proceedings 10TH IWA Conference on Instrumentation, Control and Automation, Cairns, Australia,* 2009
- **WEILAND P.** Stand und Perspektiven der Biogasnutzung und -Erzeugung in Deutschland. *Gülzower Fachgespräche Band 15: Energetische Nutzung von Biogas: Stand der Technik und Optimierungspotenzial,* 2000, 8-27
- **WEILAND, P.** Biologie der Biogaserzeugung. ZNR Biogastagung. Vortrag, 02. April 2003
- Wichtige Messdaten für den Prozessablauf und Stand der Technik in der Praxis, Gülzower Fachgespräche. **WEILAND P.** Messen, Steuern, Regeln bei der Biogaserzeugung. 2008, vol. 27
- **WEILAND P. ; RIEGER, CH.** Prozessstörungen frühzeitig erkennen. *Biogas Journal,* 2006, 9, 5, 18
- **WELLINGER, A.** Biogas-Handbuch, Grundlagen - Planung - Betrieb landwirtschaftlicher Anlagen. Verlag, 1991
- **WOLF C. ; BONGARDS M. ; MCLOONE S.** Simulation-based feed-optimization of medium-sized agricultural biogas plants. *Proceedings 11 th IWA World Congress on Anaerobic Digestion,* 23. September 2007
- **ZAHER U. ; BOUVIER J.C. ; STEYER J.P. ; VENROLLEGHEM P.A.** Titrimetric Monitoring of Anaerobic Digestion: VFA, Alkalinities and More. *Proceedings 10TH World Congress on Anaerobic Digestion, Montreal Canada,* 29. August 2004